# EUROPEAN PATENT SPECIFICATION

(11) **EP 3 065 744 B1**
(45) Date of publication and mention of the grant of the patent: **21.04.2021**
(21) Application number: 14795956.3
(22) Date of filing: 04.11.2014
(51) Int. Cl.: A61K 31/7088, G01N 33/50, A61P 13/12

(54) **A CCL2 ANTAGONIST FOR USE IN TREATING PROTEINURIA**
EIN CCL2-ANTAGONIST ZUR BEHANDLUNG DER PROTEINURIE
UN ANTAGONISTE DE CCL2 POUR LE TRAITEMENT DE LA PROTÉINURIE

(30) Priority: 04.11.2013 EP 13005191; 03.04.2014 EP 14001242
(43) Date of publication of application: 14.09.2016
(73) Proprietor: Noxxon Pharma AG, 10589 Berlin (DE)
(72) Inventor: EULBERG, Dirk, 10247 Berlin (DE); BAUMANN, Matthias, 14469 Potsdam (DE)
(74) Representative: Bohmann, Armin K.
(86) International application number: PCT/EP2014/002945
(87) International publication number: WO 2015/062743

(56) References cited:
- WO-A1-2012/025251
- WO-A2-2007/093409
- SAYYED SUFYAN G: "Role of pro-inflammatory and homeostatic chemokines in diabetic nephropathy", DISSERTATION ZUM ERWERB DES DOKTORGRADES DER HUMANBIOLOGY AN DER MEDIZINISCHEN FAKULTÄT DER LUDWIG-MAXIMILIANS-UNIVERSITÄT ZU MÜNCHEN, , 19 March 2010 (2010-03-19), pages 1-125, XP002662958, Retrieved from the Internet: URL:http://edoc.ub.uni-muenchen.de/11223/1 /Sayyed_Sufyan.pdf [retrieved on 2011-11-07]
- C. M. LLOYD: "RANTES and Monocyte Chemoattractant Protein-1 (MCP-1) Play an Important Role in the Inflammatory Phase of Crescentic Nephritis, but Only MCP-1 Is Involved in Crescent Formation and Interstitial Fibrosis", JOURNAL OF EXPERIMENTAL MEDICINE, vol. 185, no. 7, 7 April 1997 (1997-04-07) , pages 1371-1380, XP055160289, ISSN: 0022-1007, DOI: 10.1084/jem.185.7.1371
- Navin Jaipaul: "Diabetic Nephropathy -Genitourinary Disorders -Merck Manuals", Merck Manual Professional Version, 1 June 2019 (2019-06-01), XP055619757, Retrieved from the Internet: URL:https://www.merckmanuals.com/professio nal/genitourinary-disorders/symptoms-of-ge nitourinary-disorders/proteinuria?query=mi croalbuminuria [retrieved on 2019-09-09]
- Anonymous: "ACR | National Kidney Foundation", , 1 January 2019 (2019-01-01), XP055619333, Retrieved from the Internet: URL:https://www.kidney.org/kidneydisease/s iemens_hcp_acr [retrieved on 2019-09-06]

## Description

The present invention is related to an antagonist of chemokine (C-C motif) ligand 2 (abbr. CCL2) for use in a method for the treatment and/or prevention of proteinuria, a method for determining whether a human subject is susceptible for treatment with the antagonist of CCL2, an antagonist of CCL2 for use in a method for *in situ* improvement of glomerular filtration of kidney in a human subject, and an antagonist of CCL2 for use in a method for *in situ* repair of kidney in a human subject.

Diabetes mellitus is a chronic metabolic disorder characterized by hyperglycemia caused by defective insulin secretion, resistance to insulin action, or a combination of both (Kahn 1998). Alterations of lipid and protein metabolism are also important manifestations of these defects in insulin secretion or action. Most patients with diabetes mellitus have type 2 diabetes with a complex pathophysiology that combines progressive insulin resistance and beta-cell failure (Shoelson *et al.* 2006). Diabetes mellitus is associated with an elevated risk of cardiovascular disease, which is the leading cause of morbidity and mortality in this patient population. Other later stage complications include diabetic nephropathy (abbr. DN) which remains, by far, the leading cause of end-stage renal disease (abbr. ESRD) in the Western world (Giunti *et al.* 2010). Given the epidemic growth in type 2 diabetes and the steady increases in type 1 diabetes incidence and prevalence, this problem is likely to continue to represent an ever increasing public health problem which, because of diet and lifestyle changes, is now also rapidly spreading to the developing world. Current prevention and treatment strategies have only modestly delayed progression to ESRD and do not yet appear to have substantially impacted this global health problem. New concepts that broaden the potential treatment targets for DN are greatly needed. Substantial evidence from type 1 and type 2 diabetes patients has accumulated implicating inflammatory processes at all phases of the development and progression of DN (Mora & Navarro 2004; Mora & Navarro 2005; Saraheimo *et al.* 2003). Strategies to inhibit these processes may beneficially alter the clinical evolution of this important disease.

Most, if not all, nucleated cells express CCL2 which is also known as monocyte chemoattractant protein-1 (abbr. MCP-1) in response to activation by pro-inflammatory cytokines or stimulation of innate immune receptors by a range of microbial molecules (Brown *et al.* 1994; Rollins & Pober 1991; Struyf *et al.* 1998; Tsou *et al.* 2007; Tsuboi *et al.* 2002). CCL2 is a small secreted, heparin-binding protein that attracts and activates immune and non-immune cells and is known as a potent attractor of monocytes / macrophages, basophils, activated T cells, and NK cells. Rather unusual in the promiscuous chemokine system, CCL2 is highly specific in its receptor usage, binding only to the chemokine receptor CCR2 (Dawson *et al.* 2003) with high affinity.

There is a growing body of evidence that CCL2 plays an important role in diabetes mellitus and diabetic nephropathy in particular (Giunti *et al.* 2010; Ota 2013). Currently available data suggest that CCL2•CCR2 interactions are required for the emigration of inflammatory monocytes from the bone marrow into the peripheral circulation involved in diabetes mellitus and diabetic nephropathy in particular. In healthy human volunteers, infusion of CCL2 was associated with increased monocyte counts (Mayr *et al.* 2009). Blockage of monocyte emigration from the bone marrow is a very important aspect in the mechanism of action of a CCL2•CCR2 axis antagonist which cannot be compensated for by other physiological signals.

Given the importance of diabetes mellitus as a chronic disease and its late stage complications including end-stage renal disease (abbr. ESRD) and diabetic nephropathy (abbr. DN), there is a need for means for the treatment of diabetes mellitus and said late stage complications and ESDR and DN in particular.

WO 2007/093409 A2 is related to an MCP-1 binding nucleic acid molecule.

WO 2012/025251 A1 is related to a nucleic acid molecule capable of binding to MCP-1 for use in a method for the treatment and/or prevention of diabetes, diabetic complications, diabetic condition, inflammatory joint disease, eye disease, asthma, autoimmune disease, neuroinflammatory disease, tissue disease, cardiovascular disease, renal disease, ischemic injury, reperfusion injury, lung disease, transplantation, gynecological disease and conditions with elevated MCP-1 level.

The Ph.D. thesis of Sufyan G. Sayyed of March 19, 2010 is related to the role of pro-inflammatory and homeostatic chemokines in diabetic nephropathy.

Lloyd CM et al. (J. Exp. Med., Vol. 185, No. 7, April 7, 1997, pp. 1371-1380) report on RANTES and Monocyte Chemoattractant Protein 1 (MCP-1) playing an important role in the inflammatory phase of crescentic nephritis, butonly MCP-1 being involved in crescent formation and intersititial fibrosis.

In accordance therewith, the problem underlying the present invention is the provision of a means for the treatment and/or prevention of diabetes mellitus and type 2 diabetes mellitus in particular.

A further problem underlying the present invention is the providison of a means for the treatment and/or prevention of late stage complications of diabetes mellitus and type 2 diabetes mellitus in particular, including chronic kidney disease and diabetic nephropathy (abbr. DN) which can lead to end-stage renal disease (abbr ESRD).

A problem underlying the present invention is the provision of a method for the treatment and/or prevention of diabetes mellitus and type 2 diabetes mellitus in particular.

A further problem underlying the present invention is the provision of a method for the treatment and/or preventiovn of late stage complications of diabetes mellitus and type 2 diabetes mellitus in particular, including chronic kidney disease and diabetic nephropathy which can lead to ESRD.

Another problem underlying the present invention is the provision of a method for determining whether a subject is susceptible for treatment with an antagonist of CCL2.

These and other problems underlying the present invention are solved by the subject matter of the attached independent claims. Preferred embodiments may be taken from the attached dependent claims.

More specifically, the underlying problem is solved in a first aspect by an antagonist of CCL2 for use in a method for the treatment and/or prevention of proteinuria, wherein the method comprises administering the antagonist to a human subject, wherein the human subject is suffering from a disease, wherein proteinuria is expressed as ACR (urinary albumin/creatinine ratio), and wherein ACR of the subject is at least 30 mg/g, preferably at least 88 mg/g, and glomerular filtration rate of the subject is
a) at least 90 ml/min/1.73 m², or
b) 60 - 89 ml/min/1.73 m², or
c) 45 - 59 ml/min/1.73 m², or
d) 30 - 44 ml/min/1.73 m², or
e) 15 - 29 ml/min/1.73 m², or
f) < 15 ml/min/1.73 m²; and
wherein the antagonist is a nucleic acid molecule selected from the group comprising a type 2 MCP-1 binding nucleic acid molecule, a type 3 MCP-1 binding nucleic acid molecule, a type 4 MCP-1 binding nucleic acid molecule, a type 1A MCP-1 binding nucleic acid molecule, a type 1B MCP-1 binding nucleic acid molecule and a type 5 MCP-1 binding nucleic acid molecule,
whereby the type 2 MCP-1 binding nucleic acid molecule comprises in 5'->3' direction a first terminal stretch of nucleotides, a central stretch of nucleotides, and a second terminal stretch of nucleotides, whereby
the first terminal stretch of nucleotides comprises a nucleotide sequence selected from the group comprising ACGCA, CGCA and GCA,
the central stretch of nucleotides comprises a nucleotide sequence of CSUCCCUCACCGGUGCAAGUGAAGCCGYGGCUC, and
the second terminal stretch of nucleotides comprises a nucleotide sequence selected from the group comprising UGCGU, UGCG and UGC,
whereby the type 3 MCP-1 binding nucleic acid molecule comprises in 5'->3' direction a first terminal stretch of nucleotides, a first central stretch of nucleotides, a second central stretch of nucleotides, a third central stretch of nucleotides, a fourth central stretch of nucleotides, a fifth central stretch of nucleotides, a sixth central stretch of nucleotides, a seventh central stretch of nucleotides and a second terminal stretch of nucleotides, whereby
the first terminal stretch of nucleotides comprises a nucleotide sequence which is selected from the group comprising GURCUGC, GKSYGC, KBBSC and BNGC,
the first central stretch of nucleotides comprises a nucleotide sequence of GKMGU,
the second central stretch of nucleotides comprises a nucleotide sequence of KRRAR,
the third central stretch of nucleotides comprises a nucleotide sequence of ACKMC,
the fourth central stretch of nucleotides comprises a nucleotide sequence selected from the group comprising CURYGA, CUWAUGA, CWRMGACW and UGCCAGUG,
the fifth central stretch of nucleotides comprises a nucleotide sequence selected from the group comprising GGY and CWGC,
the sixth central stretch of nucleotides comprises a nucleotide sequence selected from the group comprising YAGA, CKAAU and CCUUUAU,
the seventh central stretch of nucleotides comprises a nucleotide sequence selected from the group comprising GCYR and GCWG, and
the second terminal stretch of nucleotides comprises a nucleotide sequence selected from the group comprising GCAGCAC, GCRSMC, GSVVM and GCNV,
whereby the type 4 MCP-1 binding nucleic acid molecule comprises in 5'->3' direction a first terminal stretch of nucleotides, a central stretch of nucleotides and a second terminal stretch of nucleotides, whereby
the first terminal stretch of nucleotides comprises a nucleotide sequence selected from the group comprising AGCGUGDU, GCGCGAG, CSKSUU, GUGUU, and UGUU;
the central stretch of nucleotides comprises a nucleotide sequence selected from the group comprising AGNDRDGBKGGURGYARGUAAAG, AGGUGGGUGGUAGUAAGUAAAG and CAGGUGGGUGGUAGAAUGUAAAGA, and
the second terminal stretch of nucleotides comprises a nucleotide sequence selected from the group comprising GNCASGCU, CUCGCGUC, GRSMSG, GRCAC, and GGCA,
whereby the type 1A MCP-1 binding nucleic acid molecule comprises in 5'->3' direction a first terminal stretch of nucleotides, a first central stretch of nucleotides, a second central stretch of nucleotides, a third central stretch of nucleotides, a fourth central stretch of nucleotides, a fifth central stretch of nucleotides and a second terminal stretch of nucleotides, whereby
the first terminal stretch of nucleotides comprises a nucleotide sequence of AGCRUG,
the first central stretch of nucleotides comprises a nucleotide sequence of CCCGGW,
the second central stretch of nucleotides comprises a nucleotide sequence of GUR,
the third central stretch of nucleotides comprises a nucleotide sequence of RYA,
the fourth central stretch of nucleotides comprises a nucleotide sequence of GGGGGRCGCGAYC,
the fifth central stretch of nucleotides comprises a nucleotide sequence of UGCAAUAAUG or URYAWUUG, and
the second terminal stretch of nucleotides comprises a nucleotide sequence of CRYGCU,
whereby the type 1B MCP-1 binding nucleic acid molecule comprises in 5'->3' direction a first terminal stretch of nucleotides, a first central stretch of nucleotides, a second central stretch of nucleotides, a third central stretch of nucleotides, a fourth central stretch of nucleotides, a fifth central stretch of nucleotides and a second terminal stretch of nucleotides, whereby
the first terminal stretch of nucleotides comprises a nucleotide sequence of AGYRUG,
the first central stretch of nucleotides comprises a nucleotide sequence of CCAGCU or CCAGY,
the second central stretch of nucleotides comprises a nucleotide sequence of GUG,
the third central stretch of nucleotides, comprises a nucleotide sequence of AUG,
the fourth central stretch of nucleotides comprises a nucleotide sequence of GGGGGGCGCGACC,
the fifth central stretch of nucleotides comprises a nucleotide sequence of CAUUUUA or CAUUUA, and
the second terminal stretch of nucleotides comprises a nucleotide sequence of CAYRCU,
whereby the type 5 MCP-1 binding nucleic acid molecule comprises a nucleotide sequence according to any one of SEQ.ID.NOs 87 to 115,
wherein preferably
a) the type 2 MCP-1 binding nucleic acid molecule comprises a nucleic acid sequence according to SEQ.ID.No 37, SEQ.ID.No 116, SEQ.ID.No 117 or SEQ.ID.No 228;
b) the type 3 MCP-1 binding nucleic acid molecule comprises a nucleic acid sequence selected from the group comprising the nucleic acid sequences according to SEQ ID NO. 56, SEQ.ID.No 57 to 61, SEQ.ID.No 67 to 71 and SEQ.ID.No 73;
c) the type 4 MCP-1 binding nucleic acid molecule comprises a nucleic acid sequence according to SEQ.ID.No 80 or SEQ.ID.No 81; and
d) the type 1 A MCP-1 binding nucleic acid molecule comprises a nucleic acid sequence according to SEQ.ID. No 21; and e) the type 1B MCP-1 binding nucleic acid molecule comprises a nucleic acid sequence according to SEQ.ID.No 28 or SEQ.ID.No 27.

In an embodiment of the first aspect, ACR of the subject is at least 100 mg/g and glomerular filtration rate of the subject is
a) at least 90 ml/min/1.73 m², or
b) 60 - 89 ml/min/1.73 m², or
c) 45 - 59 ml/min/1.73 m², or
d) 30 - 44 ml/min/1.73 m², or
e) 15 - 29 ml/min/1.73 m², or
f) < 15 ml/min/1.73 m².

In an embodiment of the first aspect,
a) ACR of the subject is at least 300 mg/g and glomerular filtration rate of the subject is at least 90 ml/min/1.73 m², or
b) ACR of the subject is at least 300 mg/g, preferably at least 303 mg/g and more preferably at least 695 mg/g, and glomerular filtration rate of the subject is 60 - 89 ml/min/1.73 m², or
c) ACR of the subject is at least 300 mg/g, preferably at least 303 mg/g and more preferably at least 695 mg/g, and glomerular filtration rate of the subject is 45 - 59 ml/min/1.73 m², or
d) ACR of the subject is at least 300 mg/g, preferably at least 303 mg/g and more preferably at least 695 mg/g, and glomerular filtration rate of the subject is 30 - 44 ml/min/1.73 m², or
e) ACR of the subject is at least 300 mg/g, preferably at least 303 mg/g and more preferably at least 695 mg/g, and glomerular filtration rate of the subject is 15 - 29 ml/min/1.73 m², or
f) ACR of the subject is at least 300 mg/g, preferably at least 303 mg/g and more preferably at least 695 mg/g, and glomerular filtration rate of the subject is < 15 ml/min/1.73 m².

In an embodiment of the first aspect, the disease is a renal disease, a cardiovascular disease, primary and secondary amyloidosis, focal-segmntal glomerulosclerosis, lupus nephritis, Fabry disease, glomerulonephritis, membranous glomerulopathy, hepatorenal syndrome, IgA nephropathy, cryoglobulinemia, multiple myeloma, Nagel-Patella syndrome, hereditary nephritis, polyarteriitis nodosa, purpura Schoenlein-Henoch, ANCA-associated vasculitides, nephrotic syndrome, rapid progressive glomerulonephritides or diabetes, wherein preferably diabetes is diabetes mellitus and more preferably diabetes is diabetes mellitus type 2.

In an embodiment of the first aspect, the HbAlc value of the subject is above 7.95 %.

In an embodiment of the first aspect, the subject has at least one of the following characteristics:
(i) the subject is diagnosed type 2 diabetes mellitus according to the American Diabetes Association (ADA) definition;
(ii) the subject is on stable treatment to control hypertension, hyperglycemia and/or dyslipidemia; and
(iii) the subject is on stable treatment with angiotensin-converting enzyme inhibitors (ACEi) and/or Angiotensin II receptor blockers (ARBs).

In an embodiment of the first aspect, the subject has at least one of following characteristics:
(i) the subject is not suffering from type 1 diabetes mellitus;
(ii) the eGFR of the subject is not ≤ 25 ml/min/1.73 m²;
(iii) the subject did not have any cardiovascular event within 3 months prior to the onset of the administration of the antagonist;
(iv) the subject is not suffering from uncontrolled hypertension, preferably the upper limit of the blood pressure of the subject is 180/110 mm Hg;
(v) the subject was not subject to dialysis within 3 months prior to the onset of the administration of the antagonist;
(vi) the subject did not experience any acute kidney injury within 3 months prior to the onset of the administration of the antagonist;
(vii) the subject does not have or undergo any significant edema, leg ulcer and infectious disease;
(viii) the subject does not use a drug selected from the group consisting of a thiazolidinedione class drug and an immune suppressant;
(ix) the subject does not undergo steroid therapy except a steroid therapy for topical use or inhalation; and
(x) the subject does not chronically use non-steroidal anti-inflammatory drug (NSAIDs), cyclooxygenase type 2 (COX-2) inhibitors, two or more diuretic drugs and/or aliskiren.

In an embodiment of the first aspect, the nucleuc acid molecule is a Spiegelmer.

In an embodiment of the first aspect, the nucleic acid molecule comprises a modification, whereby more preferably the modification is a high molecular weight moiety and/or the modification allows to modify the characteristics of the nucleic acid molecule in terms of residence time in an animal or human body.

In an embodiment of the first aspect, the level of proteinuria shown by the subject after termination of the administration of the antagonist to the subject is lower compared to the level of proteinuria shown by the subject prior to the administration of the antagonist, wherein preferably the level of proteinuria shown by the subject after termination of the administration of the antagonist to the subject is lower compared to the level of proteinuria shown by the subject prior to the administration of the antagonist for one month, for two months, for three months, for four months, for five months, for six months, for seven months, for eight months, for nine months, for ten months, for eleven months or for twelve months after termination of the administration of the antagonist to the subject.

More specifically, the underlying problem is solved in a second aspect by an *in vitro* method for determining whether a human subject is susceptible for treatment with an antagonist of CCL2 as defined in the frist aspect, including any embodiment thereof, wherein the method comprises determining whether the subject is suffering from proteinuria as defined in the first aspect, including any embodiment thereof, and wherein in case the subject is suffering from proteinuria as defined in any of the preceding claims, the subject is susceptible to treatment with an antagonist of CCL2 as defined in the first aspect, including any embodiment thereof.

More specifically, the underlying problem is solved in a third aspect by an antagonist of CCL2 as defined in any one of claims 1 to 9 for use in a method for *in situ* improvement of glomerular filtration of kidney in a human subject, wherein the method comprises administering to the subject the antagonist of CCL2, wherein the subject is suffering from proteinuria as defined in the first aspect, including any embodiment thereof, wherein the improvement of glomerular filtration is achieved if the level of proteinuria shown by the subject after termination of the administration of the antagonist of CCL2 to the subject is lower compared to the level of proteinuria shown by the subject prior to the administration of the antagonist of CCL2 for one month, for two months, for three months, for four months, for five months, for six months, for seven months, for eight months, for nine months, for ten months, for eleven months or for twelve months after termination of the administration of the antagonist of CCL2 to the subject.

More specifically, the underlying problem is solved in a third aspect by an antagonist of CCL2 as defined in the first aspect, including any embodiment thereof, for use in a method for *in situ* repair of kidney in a human subject, wherein the method comprises administering to the subject the antagonist of CCL2, wherein the subject is suffering from proteinuria as defined in the first aspect, including any embodiment thereof, wherein the kidney is deemed repaired if the level of proteinuria shown by the subject after termination of the administration of the antagonist of CCL2 to the subject is lower compared to the level of proteinuria shown by the subject prior to the administration of the antagonist of CCL2 for one month, for two months, for three months, for four months, for five months, for six months, for seven months, for eight months, for nine months, for ten months, for eleven months or for twelve months after termination of the administration of the antagonist of CCL2 to the subject.

More specifically, these and other problems underlying the present invention are also solved by the following embodiments.

In an embodiment of the first aspect, the disease is a cardiovascular disease primary and secondary amyloidosis, focal-segmental glomerulosclerosis, lupus nephritis, Fabry disease, glomerulonephritis, membranous glomerulopathy, hepatorenal syndrome, IgA nephropathy, cryoglobulinemia, multiple myeloma, Nagel-Patella syndrome, hereditary nephritis, polyarteriitis nodosa, purpura Schoenlein-Henoch, ANCA-associated vasculitides, nephrotic syndrome and rapid progressive glomerulonephritides.

In a preferred embodiment of the first aspect, the cardiovascular disease is hypertension.

In an embodiment of the first aspect, ACR of the subject is at least 100 mg/g.

In an embodiment of the first aspect, ACR of the subject is at least 300 mg/g, preferably at least 303 mg/g and more preferably at least 695 mg/g.

In an embodiment of the first aspect, ACR of the subject is at least 30 mg/g , preferably at least 88 mg/g, and glomerular filtration rate of the subject is at least 90 ml/min/1.73 m².

In an embodiment of the first aspect, ACR of the subject is at least 30 mg/g, preferably at least 88 mg/g, and glomerular filtration rate of the subject is 60 - 89 ml/min/1.73 m².

In an embodiment of the first aspect, ACR of the subject is at least 30 mg/g, preferably at least 88 mg/g, and glomerular filtration rate of the subject is 45 - 59 ml/min/1.73 m².

In an embodiment of the first aspect, ACR of the subject is at least 30 mg/g, preferably at least 88 mg/g, and glomerular filtration rate of the subject is 30 - 44 ml/min/1.73 m².

In an embodiment of the first aspect, ACR of the subject is at least 30 mg/g, preferably at least 88 mg/g, and glomerular filtration rate of the subject is 15 - 29 ml/min/1.73 m².

In an embodiment of the first aspect, ACR of the subject is at least 30 mg/g, preferably at least 88 mg/g, and glomerular filtration rate of the subject is < 15 ml/min/1.73 m².

In an embodiment of the first aspect, ACR of the subject is at least 100 mg/g and glomerular filtration rate of the subject is at least 90 ml/min/1.73 m².

In an embodiment of the first aspect, ACR of the subject is at least 100 mg/g and glomerular filtration rate of the subject is 60 - 89 ml/min/1.73 m².

In an embodiment of the first aspect, ACR of the subject is at least 100 mg/g and glomerular filtration rate of the subject is 45 - 59 ml/min/1.73 m².

In an embodiment of the first aspect, ACR of the subject is at least 100 mg/g and glomerular filtration rate of the subject is 30 - 44 ml/min/1.73 m².

In an embodiment of the first aspect, ACR of the subject is at least 100 mg/g and glomerular filtration rate of the subject is 15 - 29 ml/min/1.73 m².

In an embodiment of the first aspect, ACR of the subject is at least 100 mg/g and glomerular filtration rate of the subject is < 15 ml/min/1.73 m².

In an embodiment of the first aspect, ACR of the subject is at least 300 mg/g and glomerular filtration rate of the subject is at least 90 ml/min/1.73 m².

In an embodiment of the first aspect, ACR of the subject is at least 300 mg/g, preferably at least 303 mg/g and more preferably at least 695 mg/g, and glomerular filtration rate of the subject is 60 - 89 ml/min/1.73 m².

In an embodiment of the first aspect, ACR of the subject is at least 300 mg/g, preferably at least 303 mg/g and more preferably at least 695 mg/g, and glomerular filtration rate of the subject is 45 - 59 ml/min/1.73 m².

In an embodiment of the first aspect, ACR of the subject is at least 300 mg/g, preferably at least 303 mg/g and more preferably at least 695 mg/g, and glomerular filtration rate of the subject is 30 - 44 ml/min/1.73 m².

In an embodiment of the first aspect, ACR of the subject is at least 300 mg/g, preferably at least 303 mg/g and more preferably at least 695 mg/g, and glomerular filtration rate of the subject is 15 - 29 ml/min/1.73 m².

In an embodiment of the first aspect, ACR of the subject is at least 300 mg/g, preferably at least 303 mg/g and more preferably at least 695 mg/g, and glomerular filtration rate of the subject is < 15 ml/min/1.73 m².

In an embodiment of the first aspect, the HbAlc value of the subject is at least 6.0 %, preferably at least 6.1 % and more preferably at least 7.95 %.

In an embodiment of the first aspect, the HbAlc value of the subject is between 6.0 and 11 %, preferably 6.0 % to 10.4 %.

In an embodiment of the first aspect, the subject has at least two of characteristics (i), (ii) and (iii), preferably the subject has characteristics (i) and (ii), more preferably the subject has characteristics (i), (ii) and (iii):
(i) the subject is diagnosed type 2 diabetes mellitus according to the American Diabetes Association (ADA) definition;
(ii) the subject is on stable treatment to control hypertension, hyperglycemia and/or dyslipidemia; and
(iii) the subject is on stable treatment with angiotensin-converting enzyme inhibitors (ACEi) and/or Angiotensin II receptor blockers (ARBs).

In an embodiment of the first aspect, the subject has at least one of characteristics (i), (ii), (iii), (iv), (v) and (vi) one of following characteristics.
(i) the subject is not suffering from type 1 diabetes mellitus;
(ii) the eGFR of the subject is not ≤ 25 ml/min/1.73 m²; and
(iii) the subject did not have any cardiovascular event within 3 months prior to the onset of the administration of the antagonist;
(iv) the subject is not suffering from uncontrolled hypertension, preferably the upper limit of the blood pressure of the subject is 180/110 mm Hg;
(v) the subject was not subject to dialysis within 3 months prior to the onset of the administration of the antagonist;
(vi) the subject did not experience any acute kidney injury within 3 months prior to the onset of the administration of the antagonist;
(vii) the subject does not have or undergo any significant edema, leg ulcer and infectious disease;
(viii) the subject does not use a drug selected from the group consisting of a thiazolidinedione class drug and an immune suppressant;
(ix) the subject does not undergo steroid therapy except a steroid therapy for topical use or inhalation; and
(x) the subject does not chronically use of non-steroidal anti-inflammatory drug (NSAIDs), cyclooxygenase type 2 (COX-2) inhibitors, two or more diuretic drugs and/or aliskiren.

In an embodiment of the first aspect, including any embodiment thereof, the antagonist is an antagonist of the CCL2•CCR2 axis.

In an embodiment of the first aspect, including any embodiment thereof, the antagonist is a nucleic acid molecule is selected from the group consisting of a Spiegelmer and an aptamer, preferably the Spiegelmer is an anti-CCL2 Spiegelmer and the aptamer is an anti-CCL2 aptamer.

In an embodiment of the first aspect, including any embodiment thereof, the nucleic acid molecule comprises a modification, whereby the modification is preferably a high molecular weight moiety and/or whereby the modification preferably allows to modify the characteristics of the nucleic acid molecule according to embodiment 39 in terms of residence time in the animal or human body, preferably the human body.

In a preferred embodiment of the first aspect, including any embodiment thereof, the modification is selected from the group comprising a HES moiety and a PEG moiety.

In a more preferred embodiment of the first aspect, including any embodiment thereof, the modification is a PEG moiety consisting of a straight or branched PEG, wherein the molecular weight of the PEG moiety is preferably from about 20 to 120 kD, more preferably from about 30 to 80 kD and most preferably about 40 kD.

In a more preferred embodiment of the first aspect, including any embodiment thereof, the modification is a HES moiety, wherein preferably the molecular weight of the HES moiety is from about 10 to 200 kD, more preferably from about 30 to 170 kD and most preferably about 150 kD.

In an embodiment of the first aspect, including any embodiment thereof, the modification is coupled to the nucleic acid via a linker.

In an embodiment of the first aspect, including any embodiment thereof, the modification is coupled to the nucleic acid molecule at its 5'-terminal nucleotide and/or its 3'-terminal nucleotide and/or to a nucleotide of the nucleic acid molecule between the 5'-terminal nucleotide and the 3'-terminal nucleotide.

In an embodiment of the first aspect, including any embodiment thereof, the antagonist is the following compound: 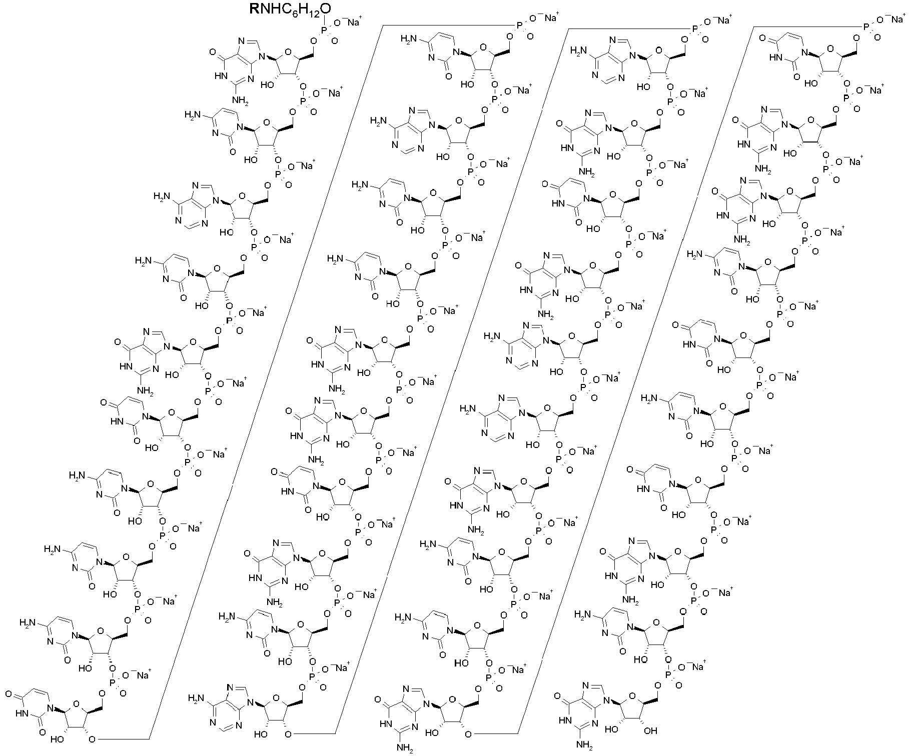
where R is 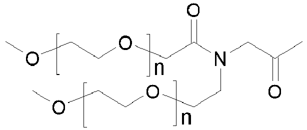
and n is 400 to 500, preferably 420 to 470, more preferably 450.

In an embodiment of the first aspect, including any embodiment thereof, the antagonist is an aptamer.

In an embodiment of the first aspect, including any embodiment thereof, the antagonist is a protein.

In a preferred embodiment of the first aspect, the protein is selected from the group consisting of an antibody, an anticaline, a DARPin, an Affilin® molecule, and a cantyrin.

In a more preferred embodiment, the antagonist is an antibody selected from the group consisting of a monoclonal antibody and a polyclonal antibody.

In a more preferred embodiment, the antibody is an anti-CCL2 antibody, preferably a monoclonal anti-CCL2 antibody.

In a more preferred embodiment, the antagonist is an anticaline.

In a more preferred embodiment, the anticaline is an anti-CCL2 anticaline.

In an embodiment of the first aspect, including any embodiment thereof, the level of proteinuria shown by the subject after termination of the administration of the antagonist to the subject is lower compared to the level of proteinuria shown by the subject prior to the administration of the antagonist.

In an embodiment of the first aspect, including any embodiment thereof, the level of proteinuria shown by the subject after termination of the administration of the antagonist to the subject is lower compared to the level of proteinuria shown by the subject prior to the administration of the antagonist for one month, for two months, for three months, for four months, for five months, for six months, for seven months, for eight months, for nine months, for ten months, for eleven months or for twelve months after termination of the administration of the antagonist to the subject.

Also disclosed is a method for the treatment of a disease, wherein the method comprises administering to a subject an antagonist as defined in the first asecpt of the present invention, including any embodiment thereof, wherein the subject is suffering from proteinuria as defined in the first aspect, including any embodiment thereof.

Also disclosed is the use of an antagonist of CCL2 as defined in the first aspect, including any embodiment thereof, for the manufacture of a medicament, wherein the medicament is for the treatment and/or prevention of a disease in a subject, wherein the subject is suffering from proteinuria as defined in the first aspect, including any embodiment thereof.

Also disclosed is a method for *in situ* improvement of glomerular filtration of kidney in a subject, wherein the method comprises administering to the subject an antagonist as defined in the first aspect, indlcuding any embodiment thereof, wherein the subject is suffering from proteinuria as defined in the first aspect, including any embodiment thereof.

In an example of such *in situ* method, improvement of glomerular filtration is achieved if the level of proteinuria shown by the subject after termination of the administration of the antagonist to the subject is lower compared to the level of proteinuria shown by the subject prior to the administration of the antagonist for one month, for two months, for three months, for four months, for five months, for six months, for seven months, for eight months, for nine months, for ten months, for eleven months or for twelve months after termination of the administration of the antagonist to the subject.

Also disclosed is a method for *in situ* repair of kidney in a subject, wherein the method comprises administering to the subject an antagonist as defined in the first aspect, including any embodiment thereof, wherein the subject is suffering from proteinuria as defined in the first aspect, including any embodiment thereof.

In an embodiment of such method for *in situ* repair of kidney, kidney is deemed repaired if the level of proteinuria shown by the subject after termination of the administration of the antagonist to the subject is lower compared to the level of proteinuria shown by the subject prior to the administration of the antagonist for one month, for two months, for three months, for four months, for five months, for six months, for seven months, for eight months, for nine months, for ten months, for eleven months or for twelve months after termination of the administration of the antagonist to the subject.

Also disclosed is the use of an antagonist of CCL2 as defined in the first aspect, including any embodiment thereof, for the manufacture of a medicament for improving glomerular filtration of kidney in a subject.

Also disclosed is the use of an antagonist of CCL2 as defined in the first aspect, including any embodiment thereof, for the manufacture of a medicament for *in situ* repair of kidney in a subject.

In an example of such uses, the subject is a subject as defined in the first aspect, including any embodiment thereof.

In an example of such uses, the subject is suffering from proteinuria as defined in the first aspect, including any embodiment thereof.

In an example of such uses, glomerular filtration rate is defined as in the first aspect, including any embodiment thereof.

In an example of such uses, glomerular filtration is improved if the level of proteinuria shown by the subject after termination of the administration of the antagonist to the subject is lower compared to the level of proteinuria shown by the subject prior to the administration of the antagonist for one month, for two months, for three months, for four months, for five months, for six months, for seven months, for eight months, for nine months, for ten months, for eleven months or for twelve months the administration of the antagonist to the subject.

In an example of such uses, kidney is repaired *in situ* in the subject if the level of proteinuria shown by the subject after termination of the administration of the antagonist to the subject is lower compared to the level of proteinuria shown by the subject prior to the administration for one month, for two months, for three months, for four months, for five months, for six months, for seven months, for eight months, for nine months, for ten months, for eleven months or for twelve months termination of the administration of the antagonist to the subject.

The present inventors have surprisingly found that an antagonist to CCL2 such as compound NOX-E36 is suitable for the treatment and/or prevention of a disease, wherein the method comprises administering the antagonist to a subject and wherein the subject is suffering from proteinuria. More specifically, the present inventors have found that upon administration of an antagonist to CCL2 such as NOX-E36 proteinuria of the subject is significantly improved, i.e. decreased. Most surprisingly, after termination of the administration of the antagonist, proteinuria further decreased and was maintained at a lower level compared to the level of proteinuria shown by the subject prior to the administration of the antagonist.

Without wishing to be bound by any theory, the present inventors assume that the antagonist activity of a compound such as NOX-E36 which preferably interferes with the CCL2•CCR2 axis as defined, for example, in Dawson et al. (2003), is responsible for this effect. More specifically, as the presence of protein such as albumin in the urine of a subject can be perceived as an indicator of inflammation in the kidney and as a marker for progression of chronic kidney disease, and as both diabetic nephropathy and type 2 diabetes are conditions with an inflammatory component, the modulation of the inflammatory state in the respective organs and of the subclinical systemic inflammation which is typical for diabetic patients by an antagonist such as NOX-E36, which thus is acticve as an anti-inflammatory molecule, facilitates the onset of repair processes which are still ongoing even after cessation of treatment. This explains the long-lasting and persistent reduction of proteinuria after the cessation of the adminstraiton of said antagonist. Persisting improvement of proteinuria can be viewed as return to normalized functionality of the cells that constitute the glomerular filtration barrier, i.e. as ongoing repair processes. As a long-term consequence of normalized proteinuria, the progressive loss of filtration capacity of the kidney, i.e. of the Glomerular Filtration Rate (abbr.) GFR, is thus attenuated or stopped. Insofar, the present invention, in another aspect, can be perceived as a method for *in situ* repair of a kidney of a subject suffering from proteinuria as disclosed herein, wherein the method comprises administration of the antagonist described herein.

In an embodiment of the invention as defined in the claims, proteinuria is albuminuria. As a preferred measure for proteinuria ACR, i.e. urinary albumin/creatinine ratio, is used and determined based on a sample from the subject. Preferably, the sample is urine from the subject. Means and methods for determining ACR of a subject are known to a person skilled and, for example, described in Labor und Diagnose (Ed. Lothar Thomas, TH-Books Verlagsgesellschaft, Frankfurt/Main 2008).

In light of the experimental evidence provided herein, it will be understood by a person skilled in the art that the observed effect is not limited to the very antagonist tested, i.e. NOX-E36. Rather, it is plausible that any other antagonist of CCL2 and in particular any antagonist of the CCL2•CCR2 axis will result in the observed effects, and will thus be equally suitable for the purposes disclosed herein.

In addition, it is also plausible to a person skilled in the art in light of the experimental evidence provided herein that any disease may be treated where a subject suffering from or being at risk of suffering from a disease which goes along with or where such patient shows proteinuria as disclosed herein. Apart from renal disease, including but not limited to end-stage renal disease, diabetic nephropathy, such disease is a disease which is selected from the group comprising cardiovascular disease, primary and secondary amyloidosis, focal-segmental glomerulosclerosis, lupus nephritis, Fabry disease, glomerulonephritis, membranous glomerulopathy, hepatorenal syndrome, IgA nephropathy, cryoglobulinemia, multiple myeloma, Nagel-Patella syndrome, hereditary nephritis, polyarteriitis nodosa, purpura Schoenlein-Henoch, ANCA-associated vasculitides, nephrotic syndrome, and rapid progressive glomerulonephritides.

Damage to the kidney can be within the parenchyma, large blood vessels or collecting systems, and is most often inferred from markers rather than direct examination of kidney tissue. The markers of kidney damage often provide a clue to the likely site of damage within the kidney and in association with other clinical findings, the cause of kidney disease. Proteinuria, expressed for example by increased ACR, is a general term for the presence of increased amounts of protein in the urine. Proteinuria may reflect abnormal loss of plasma proteins due to a) increased glomerular permeability to large molecular weight proteins (albuminuria or glomerular proteinuria), b) incomplete tubular reabsorption of normally filtered low-molecular weight proteins (tubular proteinuria), or c) increased plasma concentration of low-molecular-weight proteins (overproduction proteinuria, such as immunoglobulin light chains). Proteinuria is thus pathognomonic of kidney damage.

Glomerlar filtration rate, GFR, is widely accepted as the best overall index of kidney function because it is generally reduced after widespread structural damage and most other kidney functions decline in parallel with GFR in chronic kindney disease, CKD, as observed, among others, in diabetes mellitus and diabetic nephrophathy. Means and methods for determining GFR of a subject are known to a person skilled and, for example, described in Labor und Diagnose (Ed. Lothar Thomas, TH-Books Verlagsgesellschaft, Frankfurt/Main 2008) and (Levey *et al.* 2009).

In an embodiment of the invention as defined in the claims, the renal disease is diabetic nephropathy (abbr. DN) of patients with type 2 diabetes mellitus (abbr. T2DM). DN is common among patients with T2DM and hypertension, characterized by a persistent and usually progressive decline in renal function as measured by ACR and/or GFR. Hyperglycemia and hypertension in patients with T2DM are major determinants of the development of DN. Given the rise in the incidence of obesity, T2DM and hypertension, the associated incidence of DN has reached epidemic proportions in industrialized nations. Treatment of DN patients includes treatment of the underlying T2DM and hypertension; angiotensin-converting enzyme inhibitors (abbr. ACEis) and angiotensin II receptor blockers (abbr. ARBs) are commonly prescribed to control hypertension and slow the progression of DN. Nevertheless, about 20 % of patients eventually progress to ESRD and require renal replacement therapy. While historically considered as a non-inflammatory disease, there is now clear evidence of the role of macrophages in DN. Renal biopsies from patients with DN display elevated glomerular infiltration of macrophages that is not secondary to fibrosis, and tubular interstitial damage is strongly correlated with monocyte/macrophage cell infiltration. Experimental studies in preclinical diabetic models have clarified that monocyte/macrophage infiltration occurs at early stages of disease and this infiltration correlates with renal injury.

In another embodiment of the invention as defined in the claims, the subject is described in terms of the HbAlc value which is a generally acknowledged marker for diabetes mellitus and type 2 diabetes mellitus in particular. HbAlc is a form of hemoglobin that is measured primarily to identify the average plasma glucose concentration over prolonged periods of time. It is formed in a non-enzymatic glycation pathway by hemoglobin's exposure to plasma glucose. Normal levels of glucose produce a normal amount of glycated hemoglobin. As the average amount of plasma glucose increases, the fraction of glycated hemoglobin increases in a predictable way.

In an embodiment of the invention as defined in the claims, the subject is subject to a stable treatment for controlling hypertension, hyperglcemia and/or dyslipidemia.

Such treatment for controlling hypertension may comprise the adminstration of ACEis and ARBs. Preferred ACEis are enalapril, ramipril, lisinopril, benazpril, perindopril, iridapril, captopril, zofenopril and fosinopril. Preferred ARBs are losartan, valsartan, olmesartan, irbesartan, telmisartan and candesartan. Such treatment for controlling hypertention may comprise the use of calcium (abbr. Ca) antagonists such as, for example, nifedipin, amlodipin and verapamil, β-blockers such as, for example, metoprolol, carvedilol and bisoprolol, and/or diuretics such as, for example, furosemide, ethacrynic acid, hydrochlorothiazide, acetazolamide, spironolactone and amiloride.

Such treatment for controlling hyperglycemia may comprise the use of oral antidiabetics such as, for example biguanides inculding but not limtied to metformin; sulfonylureas including but not limited to glibenclamid and glimepirid; α-glucosidase inhibitor inducling but not limited to acarbose; glinides including but not limited to nateglinide and repaglinide; DPP4 inhibitors including but not limited to sitagliptine and vildagliptine; SGLT2 inhibitors including but not limited to dapagliflozine. Such treatment for controlling hyperglycemia may comprise the use of non-oral antidiabetics including but not lmited to incretin mimetics such as, for example, exenatide. Such treatment for controlling hyperglycemia may comprise insulin therapy.

In an embodiment of the invention as defined in the claims, type 2 diabetes mellitus is type 2 diabetes mellitus as defined by the American Diabetes Association (ADA 2012).

In an embodiment of the invention as defined in the claims, type 1 diabetes mellitus is type 1 diabetes mellitus as defined by the American Diabetes Association (ADA 2012).

It will be appreciated by a person skilled in the art that there are subjects who will benefit more than others from the use of an antagonist of CCL2 in the treatment of a disease, whereby such subject is one suffering from proteinuria. A subject who will particularly benefit from the claimed invention is one who is not suffering from type 1 diabetes mellitus. Another subject is one where eGFR is not ≤ 25 ml/min/1.73 m². In connection therewith, eGFR is estimated Glomerular Filtration Rate which is determined based on the concentration of creatinine in plasma of the subject. Means and methods for determining eGFR of a subject are known to a person skilled and, for example, described in Labor und Diagnose (Ed. Lothar Thomas, TH-Books Verlagsgesellschaft, Frankfurt/Main 2008). It will, however, be acknowledged that instead of eGFR also the directly measured GFR (abbr. mGFR) can be used. In contrast to eGFR which is obtained with the aid of endogenously produced filtration markers such as creatinine and cystatin, the mGFR is determined with the aid of an exogenous filtration marker. For mGFR, exogenous filtration markers such as inulin, iohexol, or radioactive tracers such as 51Cr-EDTA or 1251-iothalamate are intravenously administered to the patient and their clearance from the blood is followed analytically, e.g. by HPLC methodology. mGFR data are precise and accurate, but costly, time-consuming, and labor-intensive and are therefore not used in the clinical routine.

As preferably used herein, a cardiovascular event is preferably non-fatal myocardial infaction or non-fatal stroke.

As preferably used herein, acute kidney injury is rapid loss of kidney function with numerous causes including, but not limited to, intake of nephrotoxic substances, obstruction of the urinary tract, or low blood volume.

In an embodiment of the invention as defined in the claims, the antagonist of CCL2 is a compound which inibitis the binding of CCL2 to its receptor and to CCR2 in particular. Preferably, the antagonist is a compound which results in a decrease in ACR when administered to a subject suffering from proteinuria, whereby proteinuria is preferably as defined herein. More preferably the antagonist is a compound which results in a decrease in ACR when administered to a subject suffering from proteinuria, wherein proteinuria is preferably as defined herein, and wherein the decrease in ACR is maintained at a level which is below the level of ACR prior to the administration of the compound to the subject. Most preferable, the level which is below the level of ACR prior to the administration of the compound to the subject, is maintained below the level of ACR prior to the administration of the compound to the subject even upon the administration of the compound to the subject has been stopped. It is within the present invention that the level of proteinuria shown by the subject after termination of the administration of the CCL2 antagonist is lower compared to the level of proteinuria shown by the subject prior to the administration of the antagonist for one month, for two months, for three months, for four months, for five months, for six months, for seven months, for eight months, for nine months, for ten months, for eleven months and/or for twelve months. In a preferred embodiment the antagonist is a compound which shows characteristics similar or identical to NOX-E36 in the setting defined in the example part.

As disclosed herein, the antagonist may be a member of various classes of pharmaceutically active compound. Such compounds include Spiegelmers, aptamers, antibodies including monoclonal antibodies, polyclonal antibodies and Nanobodies, i.e. cameloid single-domain antibodes, DARPins, Affilin molecules, centyrins, anticalines and target molecule binding peptides. It is within the present invention that the antagonist is binding to CCL2 or CCR2. Insofar, CCL2 and CCR2, respectively, is a target molecule of such compound.

DARPins, an acronym for designed ankyrin repeat proteins, are genetically engineered antibody mimetic proteins typically exhibiting highly specific and high-affinity target protein binding. They are derived from natural ankyrin proteins, a protein class that is mediating high-affinity protein-protein interactions in nature. DARPins consist of at least three, usually four or five repeat motifs of these proteins. Their molecular mass is about 14 or 18 kDa (kilodaltons) for four- or five-repeat DARPins, respectively.

Affilin molecules are based on the human Ubiquitin scaffold.

Centyrins are generated on the baseid of the centyrin platform which is a consensus fibronectin domain.

Anticlaines are a particular form of target binding polypeptides which are, among others, described in German patent application DE 197 42 706.

Another class of compounds are CCL2 binding peptides. Such peptides may be generated by using methods according to the state of the art such as phage display. Basically, a library of peptide is generated, such as in form of phages, and this kind of libraries is contacted with the target molecule, in the present case, for example, CCL2 or CCR2. Those peptides binding to the target molecule are subsequently removed, preferably as a complex with the target molecule, from the respective reaction. It is known to the one skilled in the art that the binding characteristics, at least to a certain extent, depend on the particularly realized experimental set-up such as the salt concentration and the like. After separating those peptides binding to the target molecule with a higher affinity or a bigger force, from the non-binding members of the library, and optionally also after removal of the target molecule from the complex of target molecule and peptide, the respective peptide(s) may subsequently be characterised. Prior to the characterisation optionally an amplification step is realized such as, e. g. by propagating the peptide coding phages. The characterisation preferably comprises the sequencing of the target binding peptides. Basically, the peptides are not limited in their lengths, however, preferably peptides having a length from about 8 to 20 amino acids are preferably obtained in the respective methods. The size of the libraries may be about 10² to 10¹⁸, preferably 10⁸ to 10¹⁵ different peptides, however, is not limited thereto.

The manufacture of an antibody specific is known to the one skilled in the art and, for example, described in Harlow, E., and Lane, D., "Antibodies: A Laboratory Manual," Cold Spring Harbor Laboratory, Cold Spring Harbor, NY,(1988). Preferably, monoclonal antibodies may be used in connection with the present invention which may be manufactured according to the protocol of Cesar and Milstein and further developments based thereon. Antibodies as used herein, include, but are not limited to, complete antibodies, antibody fragments or derivatives such as Fab fragments, Fc fragments and single-stranded antibodies, as long as they are suitable and capable of binding to protein kinase N beta. Apart from monoclonal antibodies also polyclonal antibodies may be used and/or generated. The generation of polyclonal antibodies is also known to the one skilled in the art and, for example, described in Harlow, E., and Lane, D., "Antibodies: A Laboratory Manual," Cold Spring Harbor Laboratory, Cold Spring Harbor, NY, (1988). Preferably, the antibodies used for therapeutical purposes are humanized or human antibodies as defined above.

Aptamers are D-nucleic acids which are either single stranded or double stranded and which specifically interact with a target molecule such as, in the instant case, CCL2 or CCR2. The manufacture or selection of aptamers is, e. g., described in European patent EP 0 533 838. Basically, the following steps are realized. First, a mixture of nucleic acids, i. e. potential aptamers, is provided whereby each nucleic acid typically comprises a segment of several, preferably at least eight subsequent randomised nucleotides. This mixture is subsequently contacted with the target molecule whereby the nucleic acid(s) bind to the target molecule, such as based on an increased affinity towards the target or with a bigger force thereto, compared to the candidate mixture. The binding nucleic acid(s) are/is subsequently separated from the remainder of the mixture. Optionally, the thus obtained nucleic acid(s) is amplified using, e. g. polymerase chain reaction. These steps may be repeated several times giving at the end a mixture having an increased ratio of nucleic acids specifically binding to the target from which the final binding nucleic acid is then optionally selected. These specifically binding nucleic acid(s) are referred to aptamers. It is obvious that at any stage of the method for the generation or identification of the aptamers, samples of the mixture of individual nucleic acids may be taken to determine the sequence thereof using standard techniques. It is within the present invention that the aptamers may be stabilized such as, e. g., by introducing defined chemical groups which are known to the one skilled in the art of generating aptamers. Such modification may for example reside in the introduction of an amino group at the 2'-position of the sugar moiety of the nucleotides.

Spiegelmers are based on a principle similar to the one of aptamers. The manufacture of spiegelmers is described in the international patent application WO 98/08856. Spiegelmers are L-nucleic acids, which means that they are composed of L-nucleotides rather than aptamers which are composed of D-nucleotides as aptamers are. Spiegelmers are characterized by the fact that they have a very high stability in biological system and, comparable to aptamers, specifically interact with the target molecule against which they are directed.

Spiegelmers which are preferred in the practicing of the various aspects of the present invention are also referred to herein as the nucleic acid molecule of the present invention or as the nucleic acid molecule of the invention.

The nucleic acid molecules of the present invention as defined in the claims can be characterised in terms of stretches of nucleotides which are also referred to herein as boxes. The different types of nucleic acid molecules different stretches of nucleotides. In general, nucleic acid molecules of the present invention comprise at their 5'-end and the 3'-end terminal stretches of nucleotides: the first terminal stretch of nucleotides and the second terminal stretch of nucleotides (also referred to as 5'-terminal stretch of nucleotides and 3'-terminal stretch of nucleotides). The first terminal stretch of nucleotides and the second terminal stretch of nucleotides can, in principle due to their base complementarity, hybridize to each other, whereby upon hybridization a double-stranded structure is formed. However, such hybridization is not necessarily realized in the molecule under physiological and/or non-physiological conditions. The three stretches of nucleotides of nucleic acids molecules nucleic acid molecules - the first terminal stretch of nucleotides, the central stretch of nucleotides and second terminal stretch of nucleotides - are arranged to each other in 5' → 3'-direction: the first terminal stretch of nucleotides - the central stretch of nucleotides - the second terminal stretch of nucleotides. However, alternatively, the second terminal stretch of nucleotides, the central stretch of nucleotides and the terminal first stretch of nucleotides are arranged to each other in 5' → 3'-direction.

The terms 'stretch' and 'stretch of nucleotide' are used herein in a synonymous manner if not indicated to the contrary.

It is within the present invention as defined in the claims that the nucleic acid according to the present invention is a nucleic acid molecule. Insofar the terms nucleic acid and nucleic acid molecule are used herein in a synonymous manner if not indicated to the contrary.

It is within the present invention as defined in the claims that the nucleic acids according to the present invention comprise two or more stretches or part(s) thereof can, in principle, hybridise with each other. Upon such hybridisation a double-stranded structure is formed. It will be acknowledged by the ones skilled in the art that such hybridisation may or may not occur, particularly under in vitro and/or in vivo conditions. Also, in case of such hybridisation, it is not necessarily the case that the hybridisation occurs over the entire length of the two stretches where, at least based on the rules for base pairing, such hybridisation and thus formation of a double-stranded structure may, in principle, occur. As preferably used herein, a double-stranded structure is a part of a nucleic acid molecule or a structure formed by two or more separate strands or two spatially separated stretches of a single strand of a nucleic acid molecule, whereby at least one, preferably two or more base pairs exist which are base pairing preferably in accordance with the Watson-Crick base pairing rules. It will also be acknowledged by the one skilled in the art that other base pairing such as Hoogsten base pairing may exist in or form such double-stranded structure. It is also to be acknowledged that the feature that two stretches hybridize preferably indicates that such hybridization is assumed to happen due to base complementarity of the two stretches.

In a preferred embodiment of the invention as defined in the claims, the term arrangement as used herein, means the order or sequence of structural or functional features or elements described herein in connection with the nucleic acids disclosed herein.

It will be acknowledged by the person skilled in the art that the nucleic acids according to the present invention as defined in the claims, are capable of binding to CCL2. Without wishing to be bound by any theory, the present inventors assume that the CCL2 binding results from a combination of three-dimensional structural traits or elements of the claimed nucleic acid molecule, which are caused by orientation and folding patterns of the primary sequence of nucleotides forming such traits or elements, whereby preferably such traits or elements are the first terminal stretch of nucleotides, the central stretch of nucleotides and the second terminal stretch of nucleotides of the CCL2 binding nucleic acid molecules. It is evident that the individual trait or element may be formed by various different individual sequences the degree of variation of which may vary depending on the three-dimensional structure such element or trait has to form. The overall binding characteristic of the claimed nucleic acid results from the interplay of the various elements and traits, respectively, which ultimately results in the interaction of the claimed nucleic acid with its target, i. e. CCL2. Again without being wished to be bound by any theory, the central stretch of nucleotides that is characteristic for CCL2 binding nucleic acids seems to be important for mediating the binding of the claimed nucleic acid molecules with CCL2. Accordingly, the nucleic acids according to the present invention are suitable for the interaction with CCL2, respectively. Also, it will be acknowledged by the person skilled in the art that the nucleic acids according to the present invention are antagonists to CCL2. Because of this the nucleic acids according to the present invention are suitable for the treatment and prevention, respecticely, of any disease or condition which is associated with or caused by CCL2. Such diseases and conditions may be taken from the prior art which establishes that CCL2 is involved or associated with said diseases and conditions, respectively, and which provides the scientific rationale for the therapeutic use of the nucleic acids according to the invention.

The nucleic acids according to the present invention as defined in the claims, shall also comprise nucleic acids which are essentially homologous to the particular sequences disclosed herein. The term substantially homologous shall preferably be understood such that the homology is at least 75%, preferably 85%, more preferably 90% and most preferably more than 95 %, 96 %, 97 %, 98 % or 99%.

The actual percentage of homologous nucleotides present in the nucleic acid molecule according to the present invention as defined in the claims, relative to a reference nucleotide sequence or reference nucleic acid molecule according to the present invention as defined in the claims, will depend on the total number of nucleotides present in the nucleic acid molecule. The percent modification can be based upon the total number of nucleotides present in the nucleic acid molecule. Preferably, the homologous nucleotides of the nucleic acid molecule of the present invention are selected from the group comprising ribonucleotides and 2'-deoxyribonucleotides.

The homology between two nucleic acid molecules can be determined as known to the person skilled in the art. More specifically, a sequence comparison algorithm may be used for calculating the percent sequence homology for the test sequence(s) relative to the reference sequence, based on the designated program parameters. The test sequence is preferably the sequence or nucleic acid molecule which is said to be homologous or to be tested whether it is homologous, and if so, to what extent, to a different nucleic acid molecule, whereby such different nucleic acid molecule is also referred to as the reference sequence. In an embodiment of the invention as defined in the claims, the reference sequence is a nucleic acid molecule as described herein, preferably a nucleic acid molecule having a sequence according to any one of SEQ ID NO: 37, SEQ ID NO: 67 to SEQ ID NO: 71, SEQ ID NO: 57 to SEQ ID NO: 61, SEQ ID NO: 73, SEQ ID NO: 80, SEQ ID NO: 81, SEQ ID NO: 27 and SEQ ID NO: 28. Optimal alignment of sequences for comparison can be conducted, e.g., by the local homology algorithm of Smith & Waterman (Smith & Waterman, 1981) by the homology alignment algorithm of Needleman & Wunsch (Needleman & Wunsch, 1970) by the search for similarity method of Pearson & Lipman (Pearson & Lipman, 1988), by computerized implementations of these algorithms (GAP, BESTFIT, FASTA, and TFASTA in the Wisconsin Genetics Software Package, Genetics Computer Group, 575 Science Dr., Madison, Wis.), or by visual inspection.

One example of an algorithm that is suitable for determining percent sequence identity is the algorithm used in the basic local alignment search tool (hereinafter "BLAST "), see, e.g. Altschul et al (Altschul et al., 1990; and Altschul et al, 1997). Software for performing BLAST analyses is publicly available through the National Center for Biotechnology Information (hereinafter "NCBI"). The default parameters used in determining sequence identity using the software available from NCBI, e.g., BLASTN (for nucleotide sequences) and BLASTP (for amino acid sequences) are described in McGinnis et al (McGinnis et al., 2004).

The term inventive nucleic acid or nucleic acid according to the present invention as defined in the claims, shall also comprise those nucleic acids comprising the nucleic acid sequences disclosed herein or part thereof, preferably to the extent that the nucleic acids or said parts are involved in the binding to CCL2.

The nucleic acids according to the present invention as defined in the claims, shall also comprise nucleic acids which have a certain degree of identity relative to the nucleic acids disclosed herein and defined by their nucleotide sequence. More preferably, the instant invention also comprises those nucleic acid molecules which have an identity of at least 75%, preferably 85%, more preferably 90% and most preferably more than 95 %, 96 %, 97 %, 98 % or 99% relative to the nucleic acids disclosed herein and defined by their nucleotide sequence or a part thereof.

The term inventive nucleic acid as preferably used herein, shall also comprise in an embodiment a nucleic acid which is suitable to bind CCL2 and to any molecule selected from the group comprising CCL8, CCL13 and CCL11. It will be acknowledged by the ones skilled in the art that the individual nucleic acids according to the present invention will bind to one or several of such molecules. The binding behaviour of nucleic acids according to the present inventions was previously determinded as shown in the international patent application WO 2007/093409.

In an embodiment of the invention as defined in the claims, one of the nucleic acid molecules described herein, or a derivative and/ or a metabolite thereof is truncated, whereby such derivative and/ or metabolite are preferably a truncated nucleic acid compared to the nucleic acid molecules described herein. Truncation may be related to either or both of the ends of the nucleic acids as disclosed herein. Also, truncation may be related to the inner sequence of nucleotides of the nucleic acid, i.e. it may be related to the nucleotide(s) between the 5' and the 3' terminal nucleotide, respectively. Moreover, truncation shall comprise the deletion of as little as a single nucleotide from the sequence of the nucleic acids disclosed herein. Truncation may also be related to more than one stretch of the inventive nucleic acid(s), whereby the stretch can be as little as one nucleotide long. The binding of a nucleic acid according to the present invention, preferably to a molecule selected from the group comprising CCL2, CCL8, CCL13 and CCL11 can be determined by the ones skilled in the art using routine experiments or by using or adopting a method as described herein, preferably as described herein in the example part. It is within an embodiment of the present invention as defined in the claims, unless explicitly indicated to the contrary, that whenever it is referred herein to the binding of the nucleic acids according to the present invention as defined in the claims,to or with CCL2, this applies also to the binding of the nucleic acids according to the present invention as defined in the claims, to or with any molecule selected from the group comprising MCP-2, MCP-4 and eotaxin.

It is also within an embodiment of the present invention as defined in the claims, that each and any of the nucleic acid molecules described herein in their entirety in terms of their nucleic acid sequence(s) are limited to the particular nucleotide sequence(s). In other words, the terms "comprising" or "comprise(s)" shall be interpreted in such embodiment in the meaning of containing or consisting of.

L-nucleic acids or L-nucleic acid molecules as used herein are nucleic acids or nucleic acid molecules consisting of L-nucleotides, preferably consisting completely of L-nucleotides.

D-nucleic acids or D-nucleic acid molecules as used herein are nucleic acids or nucleic acid molecules consisting of D-nucleotides, preferably consisting completely of D-nucleotides.

Also, if not indicated to the contrary, any nucleotide sequence is set forth herein in 5' → 3' direction.

As preferably used, herein any position of a nucleotide is determined or referred to relative to the 5' end of a sequence, a stretch or a substretch. Accordingly, a second nucleotide is the second nucleotide counted from the 5' end of the sequence, stretch and substretch, respectively. Also, in accordance therewith, a penultimate nucleotide is the seond nucleotide counted from the 3' end of a sequence, stretch and substretch, respectively.

Irrespective of whether the inventive nucleic acid consists of D-nucleotides, L-nucleotides or a combination of both with the combination being e.g. a random combination or a defined sequence of stretches consisting of at least one L-nucleotide and at least one D-nucleic acid, the nucleic acid may consist of desoxyribonucleotide(s), ribonucleotide(s) or combinations thereof.

Designing the inventive nucleic acids as L-nucleic acids is advantageous for several reasons. L-nucleic acids are enantiomers of naturally occurring nucleic acids. D-nucleic acids, however, are not very stable in aqueous solutions and particularly in biological systems or biological samples due to the widespread presence of nucleases. Naturally occurring nucleases, particularly nucleases from animal cells are not capable of degrading L-nucleic acids. Because of this the biological half-life of the L-nucleic acid is significantly increased in such a system, including the animal and human body. Due to the lacking degradability of L-nucleic acids no nuclease degradation products are generated and thus no side effects arising therefrom observed. This aspect delimits the L-nucleic acids of factually all other compounds which are used in the therapy of diseases and/or disorders involving the presence of MCP-1. L-nucleic acids which specifically bind to a target molecule through a mechanism different from Watson Crick base pairing, or aptamers which consists partially or completely of L-nucleotides, particularly with those parts of the aptamer being involved in the binding of the aptamer to the target molecule, are also called Spiegelmers. Aptamers as such are known to a person skilled in the art and are, among others, described in 'The Aptamer Handbook' (eds. Klussmann, 2006).

It is also within the present invention as defined in the claims, that the nucleic acids according to the invention, regardless whether they are present as D-nucleic acids, L-nucleic acids or D, L-nucleic acids or whether they are DNA or RNA, may be present as single-stranded or double-stranded nucleic acids. Typically, the inventive nucleic acids are single-stranded nucleic acids which exhibit defined secondary structures due to the primary sequence and may thus also form tertiary structures. The inventive nucleic acids, however, may also be double-stranded in the meaning that two strands regardless whether they are two separate strands or whether they are bound, preferably covalently, to each other, which are complementary or partially complementary to each other are hybridised to each other.

The inventive nucleic acids may be modified. Such modifications may be related to a single nucleotide of the nucleic acid and are well known in the art. Examples for such modification are described in, among others, Venkatesan (2003), Kusser (2000) and Klussmann (2006). Such modification can be a H atom, a F atom or O-CH3 group or NH2-group at the 2' position of an individual nucleotide which is part of the nucleic acid of the present invention. Also, the nucleic acid according to the present invention can comprises at least one LNA nucleotide. In an embodiment the nucleic acid according to the present invention consists of LNA nucleotides.

A possibility to determine the binding constants of the nucleic acid molecules according to the present invention as defined in the claims is the use of the methods as described in example 3 and 5 which confirms the above finding that the nucleic acids according to the present invention exhibit a favourable KD value range. An appropriate measure in order to express the intensity of the binding between the individual nucleic acid molecule and the target which is in the present case CCL2 is the so-called KD value which as such as well the method for its determination are known to the one skilled in the art.

Preferably, the KD value shown by the nucleic acids according to the present invention as defined in the claims is below 1 µM. A KD value of about 1 µM is said to be characteristic for a non-specific binding of a nucleic acid to a target. As will be acknowledged by the ones skilled in the art, the KD value of a group of compounds such as the nucleic acids according to the present invention is within a certain range. The above-mentioned KD of about 1 µM is a preferred upper limit for the KD value. The lower limit for the KD of target binding nucleic acids can be as little as about 10 picomolar or can be higher. It is within the present invention that the KD values of individual nucleic acids binding to CCL2 is preferably within this range. Preferred ranges can be defined by choosing any first number within this range and any second number within this range. Preferred upper KD values are 250 nM and 100 nM, preferred lower KD values are 50 nM, 10 nM, 1 nM, 100 pM and 10 pM. The more preferred upper KD value is 2.5 nM, the more preferred lower KD value is 100 pM.

In addition to the binding properties of the nucleic acid molecules according to the present invention as defined in the claims, the nucleic acid molecules according to the present invention as defined in the claims inhibit the function of the respective target molecule which is in the present case CCL2. The inhibition of the function of CCL2 - for instance the stimulation of the respective receptor CCR2 - is achieved by binding of nucleic acid molecules according to the present invention to CCL2 and forming a complex of a nucleic acid molecule according to the present invention and CCL2. Such complex of a nucleic acid molecule and CCL2 cannot stimulate the receptor CCR2 that normally are stimulated by CCL2. Accordingly, the inhibition of receptor function by nucleic acid molecules according to the present invention as defined in the claims is independent from the respective receptor that can be stimulated by CCL2 but results from preventing the stimulation of the receptor by CCL2 by the nucleic acid molecules according to the present invention as defined in the claims.

A possibility to determine the inhibitory constant of the nucleic acid molecules according to the present invention as defined in the claims is the use of the methods as described in example 4 which confirms the above finding that the nucleic acids according to the present invention as defined in the claims exhibit a favourable inhibitory constant which allows the use of said nucleic acids in a therapeutic treatment scheme. An appropriate measure in order to express the intensity of the inhibitory effect of the individual nucleic acid molecule on interaction of the target which is in the present case CCL2 and the receptor CCR2, is the so-called half maximal inhibitory concentration (abbr. IC50) which as such as well the method for its determination are known to the one skilled in the art.

Preferably, the IC50 value shown by the nucleic acid molecules according to the present invention as defined in the claims is below 1 µM. An IC50 value of about 1 µM is said to be characteristic for a non-specific inhibition of target functions by a nucleic acid molecule. As will be acknowledged by the ones skilled in the art, the IC50 value of a group of compounds such as the nucleic acid molecules according to the present invention as defined in the claims is within a certain range. The above-mentioned IC50 of about 1 µM is a preferred upper limit for the IC50 value. The lower limit for the IC50 of target binding nucleic acid molecules can be as little as about 10 picomolar or can be higher. It is within the present invention as defined in the claims that the IC50 values of individual nucleic acids binding to CCL2 is preferably within this range. Preferred ranges can be defined by choosing any first number within this range and any second number within this range. Preferred upper IC50 values are 250 nM and 100 nM, preferred lower IC50 values are 50 nM, 10 nM, 1 nM, 100 pM and 10 pM. The more preferred upper IC50 value is 2.5 nM, the more preferred lower IC50 value is 100 pM.

The nucleic acid molecules according to the present invention as defined in the claims may have any length provided that they are still able to bind to the target molecule. It will be acknowledged by a person skilled in the art that there are preferred lengths for the nucleic acids according to the present inventions as defined in the claims. Typically, the length is between 15 and 120 nucleotides. It will be acknowledged by the ones skilled in the art that any integer between 15 and 120 is a possible length for the nucleic acids according to the present invention as defined in the claims. More preferred ranges for the length of the nucleic acids according to the present invention are lengths of about 20 to 100 nucleotides, about 20 to 80 nucleotides, about 20 to 60 nucleotides, about 20 to 50 nucleotides and about 30 to 50 nucleotides.

It is within the present invention as defined in the claims that the nucleic acids disclosed herein comprise a moiety which preferably is a high molecular weight moiety and/or which preferably allows to modify the characteristics of the nucleic acid in terms of, among others, residence time in an animal body, preferably a human body. A particularly preferred embodiment of such modification is PEGylation and HESylation of the nucleic acids according to the present invention as defined in the claims. As used herein PEG stands for poly(ethylene glycole) and HES for hydroxyethly starch. PEGylation as preferably used herein is the modification of a nucleic acid according to the present invention as defined in the claims, whereby such modification consists of a PEG moiety which is attached to a nucleic acid according to the present invention as defined in the claims. HESylation as preferably used herein is the modification of a nucleic acid according to the present invention whereby such modification consists of a HES moiety which is attached to a nucleic acid according to the present invention. The modifications such as linear poly (ethylene) glycol, branched poly (ethylene) glycol, hydroxyethyl starch, a peptide, a protein, a polysaccharide, a sterol, polyoxypropylene, polyoxyamidate, poly (2-hydroxyethyl)-L-glutamine and polyethylene glycol as well as the process of modifying a nucleic acid using such modifications, are described in the European patent application EP 1 306 382.

In the case of PEG being such high molecular weight moiety, the molecular weight is preferably about 20,000 to about 120,000 Da, more preferably from about 30,000 to about 80,000 Da and most preferably about 40,000 Da. In the case of HES being such high molecular weight moiety the molecular weight is is preferably from about 50 to about 1000 kDa, more preferably from about 100 to about 700 kDa and most preferably from 200 to 500 kDa. HES exhibits a molar substitution of 0.1 to 1.5, more preferably of 1 to 1.5 and exhibits a substitution sample expressed as the C2/C6 ratio of approximately 0.1 to 15, preferably of approximately 3 to 10. The process of HES modification is, e.g., described in German patent application DE 1 2004 006 249.8.

The modification can, in principle, be made to the nucleic acid molecules of the present invention at any position thereof. Preferably such modification is made either to the 5' - terminal nucleotide, the 3'-terminal nucleotide and/or any nucleotide between the 5' nucleotide and the 3' nucleotide of the nucleic acid molecule.

The modification and preferably the PEG and/or HES moiety can be attached to the nucleic acid molecule of the present invention as defined in the claims either directly or indirectly, preferably through a linker. It is also within the present invention that the nucleic acid molecule according to the present invention as defined in the claims comprises one or more modifications, preferably one or more PEG and/or HES moiety. In an embodiment the individual linker molecule attaches more than one PEG moiety or HES moiety to a nucleic acid molecule according to the present invention. The linker used in connection with the present invention can itself be either linear or branched. This kind of linkers are known to the ones skilled in the art and are further described in patent applications WO2005/074993 and WO2003/03 5665.

In a preferred embodiment of the invention as defined in the claims the linker is a biodegradable linker. The biodegradable linker allows to modify the characteristics of the nucleic acid according to the present invention in terms of, among other, residence time in an animal body, preferably in a human body, due to release of the modification from the nucleic acid according to the present invention. Usage of a biodegradable linker may allow a better control of the residence time of the nucleic acid according to the present invention. A preferred embodiment of such biodegradable linker is a biodegradable linker as described in, but not limited to, international patent applications WO2006/052790, WO2008/034122, WO2004/092191 and WO2005/099768.

It is within the present invention as defined in the claims that the modification or modification group is a biodegradable modification, whereby the biodegradable modification can be attached to the nucleic acid molecule of the present invention either directly or indirectly, preferably through a linker. The biodegradable modification allows to modify the characteristics of the nucleic acid according to the present invention in terms of, among other, residence time in an animal body, preferably in a human body, due to release or degradation of the modification from the nucleic acid according to the present invention. Usage of biodegradable modification may allow a better control of the residence time of the nucleic acid according to the present invention. A preferred embodiment of such biodegradable modification is biodegradable as described in, but not restricted to, international patent applications WO2002/065963, WO2003/070823, WO2004/113394 and WO2000/41647, preferably in WO2000/41647, page 18, line 4 to 24.

Beside the modifications as described above, other modifications can be used to modify the characteristics of the nucleic acids according to the present invention as defined in the claims, whereby such other modifications may be selected from the group of proteins, lipids such as cholesterol and sugar chains such as amylase, dextran etc..

Without wishing to be bound by any theory, it seems that by modifying the nucleic acids according to the present invention as defined in the claims with high molecular weight moiety such as a polymer and more particularly one or several of the polymers disclosed herein, which are preferably physiologically acceptable, the excretion kinetic is changed. More particularly, it seems that due to the increased molecular weight of such modified inventive nucleic acids and due to the nucleic acids of the invention not being subject to metabolism particularly when in the L form, excretion from an animal body, preferably from a mammalian body and more preferably from a human body is decreased. As excretion typically occurs via the kidneys, the present inventors assume that the glomerular filtration rate of the thus modified nucleic acids is significantly reduced compared to the nucleic acids not having this kind of high molecular weight modification which results in an increase in the residence time in the animal body. In connection therewith it is particularly noteworthy that, despite such high molecular weight modification the specificity of the nucleic acids according to the present invention is not affected in a detrimental manner. Insofar, the nucleic acids according to the present invention have among others, the surprising characteristic - which normally cannot be expected from pharmaceutically active compounds - such that a pharmaceutical formulation providing for a sustained release is not necessarily required to provide for a sustained release of the nucleic acids according to the present invention. Rather the nucleic acids according to the present invention in their modified form comprising a high molecular weight moiety, can as such already be used as a sustained release-formulation as they act, due to their modification, already as if they were released from a sustained-release formulation. Insofar, the modification(s) of the nucleic acid molecules according to the present invention as disclosed herein and the thus modified nucleic acid molecules according to the present invention and any composition comprising the same may provide for a distinct, preferably controlled pharmacokinetics and biodistribution thereof. This also includes residence time in circulation and distribution to tissues. Such modifications are further described in the patent application WO2003/035665.

However, it is also within the present invention that the nucleic acids according to the present invention as defined in the claims do not comprise any modification and particularly no high molecular weight modification such as PEGylation or HESylation. Such embodiment is particularly preferred when the nucleic acid according to the present invention shows preferential distribution to any target organ or tissue in the body or when a fast clearance of the nucleic acid according to the present invention from the body after administration is desired. Nucleic acids according to the present invention as disclosed herein with a preferential distribution profile to any target organ or tissue in the body would allow establishment of effective local concentrations in the target tissue while keeping systemic concentration of the nucleic acids low. This would allow the use of low doses which is not only beneficial from an economic point of view, but also reduces unnecessary exposure of other tissues to the nucleic acid agent, thus reducing the potential risk of side effects. Fast clearance of the nucleic acids according to the present invention from the body after administration might be desired, among others, in case of in vivo imaging or specific therapeutic dosing requirements using the nucleic acids according to the present invention or medicaments comprising the same.

The nucleic acids according to the present invention as defined in the claims, and/or the antagonists according to the present invention may be used for the generation or manufacture of a medicament. Such medicament or a pharmaceutical composition according to the present invention contains at least one of the inventive nucleic acids, optionally together with further pharmaceutically active compounds, whereby the inventive nucleic acid preferably acts as pharmaceutically active compound itself. Such medicaments comprise in preferred embodiments at least a pharmaceutically acceptable carrier. Such carrier may be, e.g., water, buffer, PBS, glucose solution, preferably a 5% glucose salt balanced solution, starch, sugar, gelatine or any other acceptable carrier substance. Such carriers are generally known to the one skilled in the art. It will be acknowledged by the person skilled in the art that any embodiments, use and aspects of or related to the medicament of the present invention is also applicable to the pharmaceutical composition of the present invention and vice versa.

"Combination therapy" or "co-therapy" as preferably used herein, includes the administration of a medicament of the invention and at least a second agent as part of a treatment regimen intended to provide a beneficial effect from the co-action of these therapeutic agents, i. e. the medicament of the present invention and said second agent. Administration of these therapeutic agents as or in combination typically is carried out over a defined time period (usually minutes, hours, days or weeks depending upon the combination selected).

"Combination therapy" may, but generally is not, intended to encompass the administration of two or more of therapeutic agents as part of separate monotherapy regimens that incidentally and arbitrarily result in the combinations of the present invention. "Combination therapy" is intended to embrace administration of these therapeutic agents in a sequential manner, that is, wherein each therapeutic agent is administered at a different time, as well as administration of these therapeutic agents, or at least two of the therapeutic agents, in a substantially simultaneous manner. Substantially simultaneous administration can be accomplished, for example, by administering to a subject a single capsule having a fixed ratio of each therapeutic agent or in multiple, single capsules for each of the therapeutic agents.

Sequential or substantially simultaneous administration of a therapeutic agent can be effected by any appropriate route including, but not limited to, topical routes, oral routes, intravenous routes, intramuscular routes, and direct absorption through mucous membrane tissues. The therapeutic agents can be administered by the same route or by different routes. For example, a first therapeutic agent of a specific combination of therapeutically effective agents may be administered by injection while the or an other therapeutic agent of the combination may be administered topically.

Alternatively, for example, all therapeutic agents may be administered topically or all therapeutic agents may be administered by injection. The sequence in which the therapeutic agents are administered is not critical unless noted otherwise. Where the combination therapy further comprises a non-drug treatment, the non-drug treatment may be conducted at any suitable time as long as a beneficial effect from the combination of the therapeutic agents and the non-drug treatment is achieved. For example, in appropriate cases, the beneficial effect may still be achieved when the non-drug treatment is temporally stayed, perhaps by days or even weeks whereas the therapeutic agents are still administered.

As outlined in general terms above, the medicament according can be administered, in principle, in any form known to the ones skilled in the art. A preferred route of administration is systemic administration, more preferably by parenteral administration, preferably by injection. Alternatively, the medicament may be administered locally. Other routes of administration comprise intramuscular, intraperitoneal, subcutaneous, per orum, intranasal, intratracheal and pulmonary with preference given to the route of administration that is the least invasive while ensuring efficiancy.

Parenteral administration is generally used for subcutaneous, intramuscular or intravenous injections and infusions. Additionally, one approach for parenteral administration employs the implantation of a slow-release or sustained-released systems, which assures that a constant level of dosage is maintained and which are well known to the ordinary skill in the art.

Furthermore, preferred medicaments can be administered by the intranasal route via topical use of suitable intranasal vehicles, inhalants, or via transdermal routes, using those forms of transdermal skin patches well known to those of ordinary skill in that art. To be administered in the form of a transdermal delivery system, the dosage administration will typically be continuous rather than intermittent throughout the dosage regimen. Other preferred topical preparations include creams, ointments, lotions, aerosol sprays and gels.

The medicament will generally comprise an amount of the active component(s) effective for the therapy, including, but not limited to, a nucleic acid molecule of the present invention, preferably dissolved or dispersed in a pharmaceutically acceptable medium. Pharmaceutically acceptable media or carriers include any and all solvents, dispersion media, coatings, antibacterial and antifungal agents, isotonic and absorption delaying agents and the like. The use of such media and agents for pharmaceutical active substances is well known in the art. Supplementary active ingredients can also be incorporated into the medicament of the present invention.

In a further aspect the instant disclosure is related to a pharmaceutical composition. Such pharmaceutical composition comprises at least one of the nucleic acids according to the present invention and preferably a pharmaceutically acceptable vehicle. Such vehicle can be any vehicle or any binder used and/or known in the art. More particularly such binder or vehicle is any binder or vehicle as discussed in connection with the manufacture of the medicament disclosed herein. In a further embodiment, the pharmaceutical composition comprises a further pharmaceutically active agent.

The preparation of a medicament and a pharmaceutical composition, respectively, is known to those of skill in the art in light of the present disclosure. Typically, such compositions may be prepared as injectables, either as liquid solutions or suspensions; solid forms suitable for solution in, or suspension in, liquid prior to injection; as tablets or other solids for oral administration; as time release capsules; or in any other form currently used, including eye drops, creams, lotions, salves, inhalants and the like. The use of sterile formulations, such as saline-based washes, by surgeons, physicians or health care workers to treat a particular area in the operating field may also be particularly useful. Compositions may also be delivered via a microdevice, microparticles or a sponge.

Upon formulation, a medicament will be administered in a manner compatible with the dosage formulation, and in such amount as is pharmacologically effective. The formulations are easily administered in a variety of dosage forms, such as the type of injectable solutions described above, but drug release capsules and the like can also be employed.

The medicament can also be administered in such oral dosage forms as timed release and sustained release tablets or capsules, pills, powders, granules, elixirs, tinctures, suspensions, syrups and emulsions. Suppositories are advantageously prepared from fatty emulsions or suspensions.

The pharmaceutical composition or medicament may be sterilized and/or contain adjuvants, such as preserving, stabilizing, wetting or emulsifying agents, solution promoters, salts for regulating the osmotic pressure and/or buffers. In addition, they may also contain other therapeutically valuable substances. The compositions are prepared according to conventional mixing, granulating, or coating methods, and typically contain about 0.1% to 75%, preferably about 1% to 50%, of the active ingredient.

Liquid, particularly injectable compositions can, for example, be prepared by dissolving, dispersing, etc. The active compound is dissolved in or mixed with a pharmaceutically pure solvent such as, for example, water, saline, aqueous dextrose, glycerol, ethanol, and the like, to thereby form an injectable solution or suspension. Additionally, solid forms suitable for dissolving in liquid prior to injection can be formulated.

The medicaments and nucleic acid molecules, respectively, can also be administered in the form of liposome delivery systems, such as small unilamellar vesicles, large unilamellar vesicles and multilamellar vesicles. Liposomes can be formed from a variety of phospholipids, containing cholesterol, stearylamine or phosphatidylcholines. In some examples, a film of lipid components is hydrated with an aqueous solution of drug to form a lipid layer encapsulating the drug, which is well known to the ordinary person skilled in the art. For example, the nucleic acid molecules according to the invention can be provided as a complex with a lipophilic compound or non-immunogenic, high molecular weight compound constructed using methods known in the art. Additionally, liposomes may bear such nucleic acid molecules on their surface for targeting and carrying cytotoxic agents internally to mediate cell killing. An example of nucleic-acid associated complexes is provided in U.S. Patent No. 6,011,020.

The medicaments and nucleic acid molecules, respectively, may also be coupled with soluble polymers as targetable drug carriers. Such polymers can include polyvinylpyrrolidone, pyran copolymer, polyhydroxypropyl-methacrylamide-phenol, polyhydroxyethylaspanamidephenol, or polyethyleneoxidepolylysine substituted with palmitoyl residues Furthermore, the medicaments and nucleic acid molecules, respectively, may be coupled to a class of biodegradable polymers useful in achieving controlled release of a drug, for example, polylactic acid, polyepsilon capro lactone, polyhydroxy butyric acid, polyorthoesters, polyacetals, polydihydropyrans, polycyanoacrylates and cross- linked or amphipathic block copolymers of hydrogels.

Effective plasma levels of the nucleic acid according to the present invention as defined in the claims preferably range from 500 fM to 200 µM, preferably from 1 nM to 20 µM, more preferably from 5 nM to 20 µM, most preferably 50 nM to 20 µM in the treatment of any of the diseases disclosed herein.

The nucleic acid molecules of the present invention as defined in the claims may preferably be administered in a single daily dose, every second or third day, weekly, every second week, in a single monthly dose or every third month.

The medicament as described herein constitutes the pharmaceutical composition disclosed herein.

It will be understood by a person skilled in the art that what has been said above for a nucleic acid molecule acting as antagonist of CCL2, preferably equally applies to any antagonist of CCL2 and particularly to those classes of pharmaceutically active compounds disclosed herein.

As preferably used herein, the term treatment comprises in a preferred embodiment additionally or alternatively prevention and/or follow-up.

As preferably used herein, the terms disease and disorder shall be used in an interchangeable manner, if not indicated to the contrary.

As preferably used herein, the terms CCL2 and MCP-1 shall be used in an interchangeable manner, if not indicated to the contrary.

As preferably used herein, the terms CCL8 and MCP-2 shall be used in an interchangeable manner, if not indicated to the contrary.

As preferably used herein, the terms CCL11 and eotaxin shall be used in an interchangeable manner, if not indicated to the contrary.

As preferably used herein, the terms CCL13 and MCP-4 shall be used in an interchangeable manner, if not indicated to the contrary.

As preferably used herein, the terms NOX-E36 and emapticap pegol shall be used in an interchangeable manner, if not indicated to the contrary. The nucleotide sequence of NOX-E36, the PEG moiety of NOX-E36 and the linker that links the nucleotide sequence of NOX-E36 and the PEG moiety of NOX-E36 are specified in Table 1, SEQ. ID. No. 228.

The various SEQ.ID. Nos., the chemical nature of the nucleic acid molecules according to the present invention as defined in the claims and the target molecules CCL2 as used herein, the actual sequence thereof and the internal reference number is summarized in the following table.

**TABLE 1**

| **Seq.-ID** | **RNA/Peptide** | **Sequence** | **Internal Reference** |
|---|---|---|---|
| 1 | L-protein | | human MCP-1, huMCP-1, CCL2 |
| 2 | L-RNA | CSUCCCUCACCGGUGCAAGUGAAGCCGYGGCUC | |
| 3 | L-RNA | AGNDRDGBKGGURGYARGUAAAG | |
| 4 | L-RNA | AGGUGGGUGGUAGUAAGUAAAG | |
| 5 | L-RNA | CAGGUGGGUGGUAGAAUGUAAAGA | |
| 6 | L-RNA | GGGGGRCGCGAYC | |
| 7 | L-RNA | UGCAAUAAUG | |
| 8 | L-protein | | human eotaxin/CCL11 |
| 9 | L-protein | | human MCP-2, CCL8, huMCP-2 |
| 10 | L-RNA | AGCGUGCCCGGAGUGGCAGGGGGACGCGACCUGCAAUAAUGCACGCU | 169-B1trc |
| 11 | L-RNA | AGCGUGCCCGGAGUGGCAGGGGGACGCGACCUGCAAUUGCACGCU | 169-F3trc |
| 12 | L-RNA | AGCGUGCCCGGAGUGGCAGGGGGACGCGACCUGUAAUAAUGCACGCU | 169-C1trc |
| 13 | L-RNA | AGCGUGCCCGGUGUGGCAGGGGGACGCGACCUGCAAUAAUGCGCGCU | 169-A3trc |
| 14 | L-RNA | AGCGUGCCCGGAGUAGCAGGGGGGCGCGACCUGCAAUAAUGCACGCU | 169-B2trc |
| 15 | L-RNA | AGCGUGCCCGGUGUGGUAGGGGGGCGCGAUCUACAAUUGCACGCU | 176-B 12trc |
| 16 | L-RNA | AGCGUGCCCGGUGUGACAGGGGGGCGCGACCUGCAUUUGCACGCU | 176-D9trc |
| 17 | L-RNA | AGCGUGCCCGGUGUGGCAGGGGGGCGCGACCUGUAUUUGCACGCU | 176-B 10trc |
| 18 | L-RNA | AGCGUGCCCGGAGUGGCAGGGGGGCGCGACCUGCAAUAAUGCACGCU | 169-F2trc |
| 19 | L-RNA | AGCGUGCCCGGUGUGGCAGGGGGGCGCGACCUGCAAUUGCACGCU | 176-B9trc |
| 20 | L-RNA | AGCAUGCCCGGUGUGGCAGGGGGGCGCGACCUGCAUUUGCAUGCU | 176-H9trc |
| 21 | L-RNA | AGCGUGCCCGGUGUGGUAGGGGGGCGCGACCUACAUUUGCACGCU | 176-E10trc |
| 22 | L-RNA | AGUGUGCCAGCUGUGAUGGGGGGGCGCGACCCAUUUUACACACU | 176-G9trc |
| 23 | L-RNA | AGUGUGCCAGCGUGAUGGGGGGGCGCGACCCAUUUUACACACU | 176-F9trc |
| 24 | L-RNA | AGUGUGCGAGCGUGAUGGGGGGGCGCGACCCAUUUUACAUACU | 176-C 11trc |
| 25 | L-RNA | AGUGUGCCAGCGUGAUGGGGGGGCGCGACCCAUUUUACAUACU | 176-E11trc |
| 26 | L-RNA | AGUAUGCCAGCGUGAUGGGGGGGCGCGACCCAUUUACAUACU | 176-D10trc |
| 27 | L-RNA | AGUGUGCCAGUGUGAUGGGGGGGCGCGACCCAUUUUACACACU | 176-H10trc |
| 28 | L-RNA | AGCGUGCCAGUGUGAUGGGGGGGCGCGACCCAUUUUACACGCU | 176-C9trc |
| 29 | L-RNA | ACGCACGUCCCUCACCGGUGCAAGUGAAGCCGCGGCUCUGCGU | 180-B1-001 |
| 30 | L-RNA | ACGCACCUCCCUCACCGGUGCAAGUGAAGCCGUGGCUCUGCGC | 180-A4-002 |
| 31 | L-RNA | ACGCACGUCCCUCACCGGUGCAAGUGAAGCCGUGGCUCUGCGU | 180-D1-002 |
| 32 | L-RNA | GCACGUCCCUCACCGGUGCAAGUGAAGCCGUGGCUCUGCGU | 180-D1-011 |
| 33 | L-RNA | ACGCACGUCCCUCACCGGUGCAAGUGAAGCCGUGGCUCUGC | 180-D1-012 |
| 34 | L-RNA | GCACGUCCCUCACCGGUGCAAGUGAAGCCGUGGCUCUGC | 180-D1-018 |
| 35 | L-RNA | CGCACGUCCCUCACCGGUGCAAGUGAAGCCGUGGCUCUGCGU | 180-D1-034 |
| 36 | L-RNA | CGCACGUCCCUCACCGGUGCAAGUGAAGCCGUGGCUCUGCG | 180-D1-035 |
| 37 | L-RNA | GCACGUCCCUCACCGGUGCAAGUGAAGCCGUGGCUCUGCG | 180-D1-036 |
| 38 | L-RNA | GUGCUGCGUAGUGGAAGACUACCUAAUGACAGCCGAAUGCUGGCAGCAC | 178-A8 |
| 39 | L-RNA | GUGCUGCGUAGUGGAAGACUACCUAAUGACAGCCUAAUGCUGGCAGCAC | 178-F7 |
| 40 | L-RNA | GUGCUGCGUAGUGGAAGACUACCUUAUGACAGCCGAAUGCUGGCAGCAC | 178-G7 |
| 41 | L-RNA | GUGCUGCGUAGUGAAAAACUACUGCCAGUGGGUCAGAGCUAGCAGCAC | 178-C6 |
| 42 | L-RNA | GUGCUGCGGAGUUAAAAACUCCCUAAGACAGGCCAGAGCCGGCAGCAC | 178-E7 |
| 43 | L-RNA | GUGCUGCGGAGUUGAAAACUCCCUAAGACAGGCCAGAGCCGGCAGCAC | 178-G6 |
| 44 | L-RNA | GUGCUGCGUAGUGGAAGACUACCUAUGACAGCCUAAUGCUGGCAGCAC | 178-A7 |
| 45 | L-RNA | GUGCUGCGGAGUUAAAAACUCCCUAAGACAGGCUAGAGCCGGCAGCAC | 178-C7 |
| 46 | L-RNA | | 178-E5 |
| 47 | L-RNA | GUGCUGCGUAGUGAAAAACUACCAACGACUGGCUAGAGCCGGCAGCAC | 181-F1 |
| 48 | L-RNA | GUGCUGCGUAGUGAAAGACUACCUGUGACAGCCGAAUGCUGGCAGCAC | 181-B2 |
| 49 | L-RNA | GUACUGCGUAGUUAAAAACUACCAACGACUGGCUAGAGCCGGCAGCAC | 181-C2 |
| 50 | L-RNA | GUGCUGCGUAGUUAAAAACUACCAACGACUGGCUAGAGCCGGCAGCAC | 178-A6 |
| 51 | L-RNA | GUGCUGCGUAGUUAAAAACUACCAGCGACAGGCUAGAGCCGGCAGCAC | 178-D6 |
| 52 | L-RNA | GUGCUGCGUAGUUAAAAACUACCAGCGACUGGCUAGAGCCGGCAGCAC | 178-D5 |
| 53 | L-RNA | GUGCUGCGUAGUGAGAAACUACCAACGACUGGCUAGAGCCGGCAGCAC | 181-A2 |
| 54 | L-RNA | GGCUGCGUAGUUAAAAACUACCAGCGACUGGCUAGAGCCGGCAGCC | 178-D5-020 |
| 55 | L-RNA | GGCGCGUAGUUAAAAACUACCAGCGACUGGCUAGAGCCGGCGCC | 178-D5-027 |
| 56 | L-RNA | GUGCGCGUAGUUAAAAACUACCAGCGACUGGCUAGAGCCGGCGCAC | 178-D5-030 |
| 57 | L-RNA | GUGCGCGUAGUGAGAAACUACCAACGACUGGCUAGAGCCGGCGCAC | 181-A2-002 |
| 58 | L-RNA | GUGCCGUAGUGAGAAACUACCAACGACUGGCUAGAGCCGGGCAC | 181-A2-004 |
| 59 | L-RNA | GUGGCGUAGUGAGAAACUACCAACGACUGGCUAGAGCCGGCCAC | 181-A2-005 |
| 60 | L-RNA | GUCGCGUAGUGAGAAACUACCAACGACUGGCUAGAGCCGGCGAC | 181-A2-006 |
| 61 | L-RNA | UGCGCGUAGUGAGAAACUACCAACGACUGGCUAGAGCCGGCGCA | 181-A2-007 |
| 62 | L-RNA | GCUGCGUAGUGAGAAACUACCAACGACUGGCUAGAGCCGGCAGC | 181-A2-008 |
| 63 | L-RNA | GCUGCGUAGUGAGAAACUACCAACGACUGGCUAGAGCCGGCAGC | 181-A2-011 |
| 64 | L-RNA | GGUGCGUAGUGAGAAACUACCAACGACUGGCUAGAGCCGGCACC | 181-A2-012 |
| 65 | L-RNA | UGGCGUAGUGAGAAACUACCAACGACUGGCUAGAGCCGGCCA | 181-A2-015 |
| 66 | L-RNA | GCGCGUAGUGAGAAACUACCAACGACUGGCUAGAGCCGGCGC | 181-A2-016 |
| 67 | L-RNA | GUGCGUAGUGAGAAACUACCAACGACUGGCUAGAGCCGGCAC | 181-A2-017 |
| 68 | L-RNA | GG-GCGUAGUGAGAAACUACCAACGACUGGCUAGAGCCGGCCC | 181-A2-018 |
| 69 | L-RNA | GAGCGUAGUGAGAAACUACCAACGACUGGCUAGAGCCGGCUC | 181-A2-019 |
| 70 | L-RNA | CGGCGUAGUGAGAAACUACCAACGACUGGCUAGAGCCGGCCG | 181-A2-020 |
| 71 | L-RNA | CCGCGUAGUGAGAAACUACCAACGACUGGCUAGAGCCGGCGG | 181-A2-021 |
| 72 | L-RNA | CAGCGUAGUGAGAAACUACCAACGACUGGCUAGAGCCGGCUG | 181-A2-022 |
| 73 | L-RNA | CUGCGUAGUGAGAAACUACCAACGACUGGCUAGAGCCGGCAG | 181-A2-023 |
| 74 | L-RNA | AGCGUGUUAGUGAAGUGGGUGGCAGGUAAAGGACACGCU | 184-B8trc |
| 75 | L-RNA | AGCGUGGUAGCGGUGUGGGUGGUAGGUAAAGGCCACGCU | 184-C6trc |
| 76 | L-RNA | AGCGUGAUAGAAGAGCGGGUGGUAGGUAAAGGUCAGGCU | 184-H5trc |
| 77 | L-RNA | AGCGUGUUAGGUAGGGUGGUAGUAAGUAAAGGACACGCU | 184-A7trc |
| 78 | L-RNA | AGCGUGUUAGGUGGGUGGUAGUAAGUAAAGGACACGCU | 187-A5trc |
| 79 | L-RNA | AGCGUGUUAGGUGGGUGGUAGUAAGUAAAGGGCACGCU | 187-H5trc |
| 80 | L-RNA | CCGCUUAGGUGGGUGGUAGUAAGUAAAGGGGCGG | 174-D4-004 |
| 81 | L-RNA | GCGCGAGCAGGUGGGUGGUAGAAUGUAAAGACUCGCGUC | 166-A4-002 |
| 82 | L-RNA | CGUGUUAGGUGGGUGGUAGUAAGUAAAGGACACG | 187-A5trc-001 |
| 83 | L-RNA | GUGUUAGGUGGGUGGUAGUAAGUAAAGGACAC | 187-A5trc-002 |
| 84 | L-RNA | CGUGUUAGGUGGGUGGUAGUAAGUAAAGGGCACG | 187-H5trc-002 |
| 85 | L-RNA | GUGUUAGGUGGGUGGUAGUAAGUAAAGGGCAC | 187-H5trc-003 |
| 86 | L-RNA | UGUUAGGUGGGUGGUAGUAAGUAAAGGGCA | 187-H5trc-004 |
| 87 | L-RNA | GGACGAGAGUGACAAAUGAUAUAACCUCCUGACUAACGCUGCGGGCGACAGG | 177-B3 |
| 88 | L-RNA | GGACCUAUCGCUAAGACAACGCGCAGUCUACGGGACAUUCUCCGCGGACAGG | 177-C1 |
| 89 | L-RNA | GGACAAUUGUUACCCCCGAGAGAGACAAAUGAGACAACCUCCUGAAGACAGG | 177-C2 |
| 90 | L-RNA | GGACGAAAGUGAGAAAUGAUACAACCUCCUGUUGCUGCGAAUCCGGACAGG | 177-E3 |
| 91 | L-RNA | GGACGUAAAAGACGCUACCCGAAAGAAUGUCAGGAGGGUAGACCGACAGG | 177-D1 |
| 92 | L-RNA | GGACUAGAAACUACAAUAGCGGCCAGUUGCACCGCGUUAUCAACGACAGG | 177-E1 |
| 93 | L-RNA | GGACUAGUCAGCCAGUGUGUAUAUCGGACGCGGGUUUAUUUACUGACAGG | 177-A1 |
| 94 | L-RNA | | 177-G3 |
| 95 | L-RNA | | 177-C3 |
| 96 | L-RNA | | 177-A2 |
| 97 | L-RNA | CCUGUGCUACACGCAGUAAGAAGUGAACGUUCAGUAUGUGUGCACAGG | 170-E4trc |
| 98 | L-RNA | CGUGAGCCAGGCACCGAGGGCGUUAACUGGCUGAUUGGACACGACACG | 166-D2trc |
| 99 | L-RNA | CGUGAACAUGCAAGCUAAGCGGGGCUGUUGGUUGCUUGGCCCGCCACG | 174-A2trc |
| 100 | L-RNA | CGUGCAGAGAGAGACCAACCACGUAAAAUCAACCUAAUGGGCCGCACG | 174-E2trc |
| 101 | L-RNA | CGUGCAGAGAGAGACCAACCACGUAAAAUCAACCUAAUGGGCCGCACG | 183-G3trc |
| 102 | L-RNA | CGUGAACAUUCAAGCUAAGCGGGGCUGUUGGUUGCUUGGCCCGCCACG | 183-B2trc |
| 103 | L-RNA | CGUGCCGAGGCGGCGACCAGCGUUACUUAGAGAGGCUUUGGCACCACG | 166-B2trc |
| 104 | L-RNA | CGUGAUAACAGCCGUCGGUCAAGAAAACAAAGUUCGGGCGGCGCACG | 166-G3trc |
| 105 | L-RNA | CGUGGGUGGCGCACCGAGGGCGAAAAGCCACCAGUAAAGAUAGACCG | 166-D1trc |
| 106 | L-RNA | CGUGUGAUCUCCUUUGGGGUGAUUAGCUUAGAGACUUCCCACACG | 183-H2trc |
| 107 | L-RNA | GCACCUUCGCCUAAUACACGUGCCGGCUAGCUAAUACUCGUCCGC | 167-A7trc |
| 108 | L-RNA | GCACGACUUGGGCGACCAGUGAUACUUAGAGAGCAAGUCGUCGGC | 167-C7trc |
| 109 | L-RNA | GCGCGCGCUCAGUAAGAAAUUGAAAGUUCAGAAUGUCGUCGCGC | 167-B5trc |
| 110 | L-RNA | AGUGUGUGGCAGGCUAAGGAGAUAUUCCGAGACCACGCU | 184-D7trc |
| 111 | L-RNA | AGUGUGUGGCAGACUAUGGAUAGACUCCGAGACCACGCU | 184-D6trc |
| 112 | L-RNA | AGCGUGAGGCGACCAGCGGAUUACUUAGAGAGUCACGCU | 184-E5trc |
| 113 | L-RNA | AGCGUGAAGGGGACCAGCGUUACUUACAGAGUUCACGCU | 184-G6trc |
| 114 | L-RNA | AGCGUGUGAUGUAUGUAGCACCGUAUCAGAGGACACGCU | 184-B7trc |
| 115 | L-RNA | AGCGUGAGGCGACCCGUGUUUCGUAGAGAGUCACGCU | 184-B6trc |
| 116 | L-RNA | 5'PEG-GCACGUCCCUCACCGGUGCAAGUGAAGCCGUGGCUCUGCG | NOX-E36-5'PEG |
| 117 | L-RNA | GCACGUCCCUCACCGGUGCAAGUGAAGCCGUGGCUCUGCG-3 'PEG | NOX-E36-3 'PEG |
| 118 | L-RNA | GGGGGGCGCGACC | |
| 119 | D-PROTEIN | | biotinylated human D-MCP-1 |
| 120 | L-RNA | CGUCCCUCACCGGUGCAAGUGAAGCCGUGGCUC | |
| 121 | D-RNA | AGCGUGCCCGGAGUGGCAGGGGGACGCGACCUGCAAUAAUGCACGCU | 169-B1trc |
| 122 | D-RNA | AGCGUGCCCGGAGUGGCAGGGGGACGCGACCUGCAAUUGCACGCU | 169-F3trc |
| 123 | D-RNA | AGCGUGCCCGGAGUGGCAGGGGGACGCGACCUGUAAUAAUGCACGCU | 169-C1trc |
| 124 | D-RNA | AGCGUGCCCGGUGUGGCAGGGGGACGCGACCUGCAAUAAUGCGCGCU | 169-A3trc |
| 125 | D-RNA | AGCGUGCCCGGAGUAGCAGGGGGGCGCGACCUGCAAUAAUGCACGCU | 169-B2trc |
| 126 | D-RNA | AGCGUGCCCGGUGUGGUAGGGGGGCGCGAUCUACAAUUGCACGCU | 176-B 12trc |
| 127 | D-RNA | AGCGUGCCCGGUGUGACAGGGGGGCGCGACCUGCAUUUGCACGCU | 176-D9trc |
| 128 | D-RNA | AGCGUGCCCGGUGUGGCAGGGGGGCGCGACCUGUAUUUGCACGCU | 176-B10trc |
| 129 | D-RNA | AGCGUGCCCGGAGUGGCAGGGGGGCGCGACCUGCAAUAAUGCACGCU | 169-F2trc |
| 130 | D-RNA | AGCGUGCCCGGUGUGGCAGGGGGGCGCGACCUGCAAUUGCACGCU | 176-B9trc |
| 131 | D-RNA | AGCAUGCCCGGUGUGGCAGGGGGGCGCGACCUGCAUUUGCAUGCU | 176-H9trc |
| 132 | D-RNA | AGCGUGCCCGGUGUGGUAGGGGGGCGCGACCUACAUUUGCACGCU | 176-E10trc |
| 133 | D-RNA | AGUGUGCCAGCUGUGAUGGGGGGGCGCGACCCAUUUUACACACU | 176-G9trc |
| 134 | D-RNA | AGUGUGCCAGCGUGAUGGGGGGGCGCGACCCAUUUUACACACU | 176-F9trc |
| 135 | D-RNA | AGUGUGCGAGCGUGAUGGGGGGGCGCGACCCAUUUUACAUACU | 176-C 11trc |
| 136 | D-RNA | AGUGUGCCAGCGUGAUGGGGGGGCGCGACCCAUUUUACAUACU | 176-E11trc |
| 137 | D-RNA | AGUAUGCCAGCGUGAUGGGGGGGCGCGACCCAUUUACAUACU | 176-D10trc |
| 138 | D-RNA | AGUGUGCCAGUGUGAUGGGGGGGCGCGACCCAUUUUACACACU | 176-H10trc |
| 139 | D-RNA | AGCGUGCCAGUGUGAUGGGGGGGCGCGACCCAUUUUACACGCU | 176-C9trc |
| 140 | D-RNA | ACGCACGUCCCUCACCGGUGCAAGUGAAGCCGCGGCUCUGCGU | 180-B1-001 |
| 141 | D-RNA | ACGCACCUCCCUCACCGGUGCAAGUGAAGCCGUGGCUCUGCGC | 180-A4-002 |
| 142 | D-RNA | ACGCACGUCCCUCACCGGUGCAAGUGAAGCCGUGGCUCUGCGU | 180-D1-002 |
| 143 | D-RNA | GCACGUCCCUCACCGGUGCAAGUGAAGCCGUGGCUCUGCGU | 180-D1-011 |
| 144 | D-RNA | ACGCACGUCCCUCACCGGUGCAAGUGAAGCCGUGGCUCUGC | 180-D1-012 |
| 145 | D-RNA | GCACGUCCCUCACCGGUGCAAGUGAAGCCGUGGCUCUGC | 180-D1-018 |
| 146 | D-RNA | CGCACGUCCCUCACCGGUGCAAGUGAAGCCGUGGCUCUGCGU | 180-D1-034 |
| 147 | D-RNA | CGCACGUCCCUCACCGGUGCAAGUGAAGCCGUGGCUCUGCG | 180-D1-035 |
| 148 | D-RNA | GCACGUCCCUCACCGGUGCAAGUGAAGCCGUGGCUCUGCG | 180-D1-036 |
| 149 | D-RNA | | 178-A8 |
| 150 | D-RNA | | 178-F7 |
| 151 | D-RNA | | 178-G7 |
| 152 | D-RNA | GUGCUGCGUAGUGAAAAACUACUGCCAGUGGGUCAGAGCUAGCAGCAC | 178-C6 |
| 153 | D-RNA | GUGCUGCGGAGUUAAAAACUCCCUAAGACAGGCCAGAGCCGGCAGCAC | 178-E7 |
| 154 | D-RNA | GUGCUGCGGAGUUGAAAACUCCCUAAGACAGGCCAGAGCCGGCAGCAC | 178-G6 |
| 155 | D-RNA | GUGCUGCGUAGUGGAAGACUACCUAUGACAGCCUAAUGCUGGCAGCAC | 178-A7 |
| 156 | D-RNA | GUGCUGCGGAGUUAAAAACUCCCUAAGACAGGCUAGAGCCGGCAGCAC | 178-C7 |
| 157 | D-RNA | | 178-E5 |
| 158 | D-RNA | GUGCUGCGUAGUGAAAAACUACCAACGACUGGCUAGAGCCGGCAGCAC | 181-F1 |
| 159 | D-RNA | GUGCUGCGUAGUGAAAGACUACCUGUGACAGCCGAAUGCUGGCAGCAC | 181-B2 |
| 160 | D-RNA | GUACUGCGUAGUUAAAAACUACCAACGACUGGCUAGAGCCGGCAGCAC | 181-C2 |
| 161 | D-RNA | GUGCUGCGUAGUUAAAAACUACCAACGACUGGCUAGAGCCGGCAGCAC | 178-A6 |
| 162 | D-RNA | GUGCUGCGUAGUUAAAAACUACCAGCGACAGGCUAGAGCCGGCAGCAC | 178-D6 |
| 163 | D-RNA | GUGCUGCGUAGUUAAAAACUACCAGCGACUGGCUAGAGCCGGCAGCAC | 178-D5 |
| 164 | D-RNA | GUGCUGCGUAGUGAGAAACUACCAACGACUGGCUAGAGCCGGCAGCAC | 181-A2 |
| 165 | D-RNA | GGCUGCGUAGUUAAAAACUACCAGCGACUGGCUAGAGCCGGCAGCC | 178-D5-020 |
| 166 | D-RNA | GGCGCGUAGUUAAAAACUACCAGCGACUGGCUAGAGCCGGCGCC | 178-D5-027 |
| 167 | D-RNA | GUGCGCGUAGUUAAAAACUACCAGCGACUGGCUAGAGCCGGCGCAC | 178-D5-030 |
| 168 | D-RNA | GUGCGCGUAGUGAGAAACUACCAACGACUGGCUAGAGCCGGCGCAC | 181-A2-002 |
| 169 | D-RNA | GUGCCGUAGUGAGAAACUACCAACGACUGGCUAGAGCCGGGCAC | 181-A2-004 |
| 170 | D-RNA | GUGGCGUAGUGAGAAACUACCAACGACUGGCUAGAGCCGGCCAC | 181-A2-005 |
| 171 | D-RNA | GUCGCGUAGUGAGAAACUACCAACGACUGGCUAGAGCCGGCGAC | 181-A2-006 |
| 172 | D-RNA | UGCGCGUAGUGAGAAACUACCAACGACUGGCUAGAGCCGGCGCA | 181-A2-007 |
| 173 | D-RNA | GCUGCGUAGUGAGAAACUACCAACGACUGGCUAGAGCCGGCAGC | 181-A2-008 |
| 174 | D-RNA | GCUGCGUAGUGAGAAACUACCAACGACUGGCUAGAGCCGGCAGC | 181-A2-011 |
| 175 | D-RNA | GGUGCGUAGUGAGAAACUACCAACGACUGGCUAGAGCCGGCACC | 181-A2-012 |
| 176 | D-RNA | UGGCGUAGUGAGAAACUACCAACGACUGGCUAGAGCCGGC-CA | 181-A2-015 |
| 177 | D-RNA | GCGCGUAGUGAGAAACUACCAACGACUGGCUAGAGCCGGCGC | 181-A2-016 |
| 178 | D-RNA | GUGCGUAGUGAGAAACUACCAACGACUGGCUAGAGCCGGCAC | 181-A2-017 |
| 179 | D-RNA | GG-GCGUAGUGAGAAACUACCAACGACUGGCUAGAGCCGGCCC | 181-A2-018 |
| 180 | D-RNA | GAGCGUAGUGAGAAACUACCAACGACUGGCUAGAGCCGGCUC | 181-A2-019 |
| 181 | D-RNA | CGGCGUAGUGAGAAACUACCAACGACUGGCUAGAGCCGGCCG | 181-A2-020 |
| 182 | D-RNA | CCGCGUAGUGAGAAACUACCAACGACUGGCUAGAGCCGGCGG | 181-A2-021 |
| 183 | D-RNA | CAGCGUAGUGAGAAACUACCAACGACUGGCUAGAGCCGGCUG | 181-A2-022 |
| 184 | D-RNA | CUGCGUAGUGAGAAACUACCAACGACUGGCUAGAGCCGGCAG | 181-A2-023 |
| 185 | D-RNA | AGCGUGUUAGUGAAGUGGGUGGCAGGUAAAGGACACGCU | 184-B8trc |
| 186 | D-RNA | AGCGUGGUAGCGGUGUGGGUGGUAGGUAAAGGCCACGCU | 184-C6trc |
| 187 | D-RNA | AGCGUGAUAGAAGAGCGGGUGGUAGGUAAAGGUCAGGCU | 184-H5trc |
| 188 | D-RNA | AGCGUGUUAGGUAGGGUGGUAGUAAGUAAAGGACACGCU | 184-A7trc |
| 189 | D-RNA | AGCGUGUUAGGUGGGUGGUAGUAAGUAAAGGACACGCU | 187-A5trc |
| 190 | D-RNA | AGCGUGUUAGGUGGGUGGUAGUAAGUAAAGGGCACGCU | 187-H5trc |
| 191 | D-RNA | CC GCUUAGGUGGGUGGUAGUAAGUAAAGGGGC GG | 174-D4-004 |
| 192 | D-RNA | GCGCGAGCAGGUGGGUGGUAGAAUGUAAAGACUCGCGUC | 166-A4-002 |
| 193 | D-RNA | CGUGUUAGGUGGGUGGUAGUAAGUAAAGGACACG | 187-A5trc-001 |
| 194 | D-RNA | GUGUUAGGUGGGUGGUAGUAAGUAAAGGACAC | 187-A5trc-002 |
| 195 | D-RNA | CGUGUUAGGUGGGUGGUAGUAAGUAAAGGGCACG | 187-H5trc-002 |
| 196 | D-RNA | GUGUUAGGUGGGUGGUAGUAAGUAAAGGGCAC | 187-H5trc-003 |
| 197 | D-RNA | UGUUAGGUGGGUGGUAGUAAGUAAAGGGCA | 187-H5trc-004 |
| 198 | D-RNA | | 177-B3 |
| 199 | D-RNA | | 177-C1 |
| 200 | D-RNA | GGACAAUUGUUACCCCCGAGAGAGACAAAUGAGACAACCUCCUGAAGACAGG | 177-C2 |
| 201 | D-RNA | GGACGAAAGUGAGAAAUGAUACAACCUCCUGUUGCUGCGAAUCCGGACAGG | 177-E3 |
| 202 | D-RNA | GGACGUAAAAGACGCUACCCGAAAGAAUGUCAGGAGGGUAGACCGACAGG | 177-D1 |
| 203 | D-RNA | GGACUAGAAACUACAAUAGCGGCCAGUUGCACCGCGUUAUCAACGACAGG | 177-E1 |
| 204 | D-RNA | GGACUAGUCAGCCAGUGUGUAUAUCGGACGCGGGUUUAUUUACUGACAGG | 177-A1 |
| 205 | D-RNA | GGACUGUCCGGAGUGUGAAACUCCCCGAGACCGCCAGAAGCGGGGACAGG | 177-G3 |
| 206 | D-RNA | GGACUUCUAUCCAGGUGGGUGGUAGUAUGUAAAGAGAUAGAAGUGACAGG | 177-C3 |
| 207 | D-RNA | GGACGAGAGCGAACAAUGAUAUAACCUCCUGACGGAAAGAGAUCGACAGG | 177-A2 |
| 208 | D-RNA | CCUGUGCUACACGCAGUAAGAAGUGAACGUUCAGUAUGUGUGCACAGG | 170-E4trc |
| 209 | D-RNA | CGUGAGCCAGGCACCGAGGGCGUUAACUGGCUGAUUGGACACGACACG | 166-D2trc |
| 210 | D-RNA | CGUGAACAUGCAAGCUAAGCGGGGCUGUUGGUUGCUUGGCCCGCCACG | 174-A2trc |
| 211 | D-RNA | CGUGCAGAGAGAGACCAACCACGUAAAAUCAACCUAAUGGGCCGCACG | 174-E2trc |
| 212 | D-RNA | CGUGCAGAGAGAGACCAACCACGUAAAAUCAACCUAAUGGGCCGCACG | 183-G3trc |
| 213 | D-RNA | CGUGAACAUUCAAGCUAAGCGGGGCUGUUGGUUGCUUGGCCCGCCACG | 183-B2trc |
| 214 | D-RNA | CGUGCCGAGGCGGCGACCAGCGUUACUUAGAGAGGCUUUGGCACCACG | 166-B2trc |
| 215 | D-RNA | CGUGAUAACAGCCGUCGGUCAAGAAAACAAAGUUCGGGCGGCGCACG | 166-G3trc |
| 216 | D-RNA | CGUGGGUGGCGCACCGAGGGCGAAAAGCCACCAGUAAAGAUAGACCG | 166-D1trc |
| 217 | D-RNA | CGUGUGAUCUCCUUUGGGGUGAUUAGCUUAGAGACUUCCCACACG | 183-H2trc |
| 218 | D-RNA | GCACCUUCGCCUAAUACACGUGCCGGCUAGCUAAUACUCGUCCGC | 167-A7trc |
| 219 | D-RNA | GCACGACUUGGGCGACCAGUGAUACUUAGAGAGCAAGUCGUCGGC | 167-C7trc |
| 220 | D-RNA | GCGCGCGCUCAGUAAGAAAUUGAAAGUUCAGAAUGUCGUCGCGC | 167-B5trc |
| 221 | D-RNA | AGUGUGUGGCAGGCUAAGGAGAUAUUCCGAGACCACGCU | 184-D7trc |
| 222 | D-RNA | AGUGUGUGGCAGACUAUGGAUAGACUCCGAGACCACGCU | 184-D6trc |
| 223 | D-RNA | AGCGUGAGGCGACCAGCGGAUUACUUAGAGAGUCACGCU | 184-E5trc |
| 224 | D-RNA | AGCGUGAAGGGGACCAGCGUUACUUACAGAGUUCACGCU | 184-G6trc |
| 225 | D-RNA | AGCGUGUGAUGUAUGUAGCACCGUAUCAGAGGACACGCU | 184-B7trc |
| 226 | D-RNA | AGCGUGAGGCGACCCGUGUUUCGUAGAGAGUCACGCU | 184-B6trc |
| 227 | L-Protein | | CCL13/MCP-4 |
| 228 | L-RNA | RNHC6H12O-GCACGUCCCUCACCGGUGCAAGUGAAGCCGUGGCUCUGCG where R is | NOX-E36/emamticap pegol |
| | | 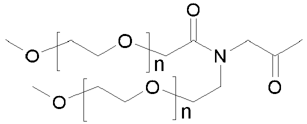 | |
| | | and n is 400 to 500, preferably 420 to 470, more preferably 450. | |

In a further aspect, the instant disclosure is realted to a method for the treatment of a disease, wherein the method comprises administering to a subject an antagonist as defined and disclosed herein in connection with each and any aspect and embodiment of the invention wherein the subject is suffering from proteinuria as defined in connection with each and any aspect and embodiment of the invention.

In a further aspect the instant disclosure is related to the use of an antagonist of CCL2 as defined in connection with each and any aspect and embodiment of the invention as defined in the claims and of the instant disclosure for the manufacture of a medicament, wherein the medicament is for the treatment and/or prevention of a disease in a subject, wherein the subject is suffering from proteinuria as defined in connection with each and any aspect and embodiment of the invention and the instant disclosure.

In a still further aspect, the instant disclosure is related to a pharmaceutical composition comprising a pharmaceutically acceptale excipient and an antagonist of CCL2 as defined in connection with each and any aspect and embodiment of the invention, wherein the pharmaceutical composition for use in the treatment and/or prevention of a disease in a subject, wherein the subject is suffering from proteinuria as defined in connection with each and any aspect and embodiment of the invention.

The present invention is further illustrated by the figures, examples and the sequence listing from which further features, embodiments and advantages may be taken, wherein
- Fig. 1: shows an alignment of sequences of MCP-1 binding nucleic acid molecules of "Type 1A;
- Fig. 2: shows an alignment of sequences of MCP-1 binding nucleic acid molecules of "Type 1B";
- Fig. 3: shows an alignment of sequences of MCP-1 binding nucleic acid molecules of "Type 2" and derivatives of MCP-1 binding nucleic acid molecule 180-D1-002;
- Fig. 4: shows an alignment of sequences of MCP-1 binding nucleic acid molecules of "Type 3",
- Fig. 5: shows derivatives of the MCP-1 binding nucleic acid molecules 178-D5 and 181-A2 (MCP-1 binding nucleic acid molecules of "Type 3");
- Fig. 6: shows an alignment of sequences of MCP-1 binding nucleic acid molecules of "Type 4";
- Fig. 7: shows further MCP-1 binding nucleic acid molecules which are, in addition to other MCP-1 binding nucleic acid molecules, also referred to as type 5 MCP-1 binding nucleic acid molecules;
- Fig. 8: shows ACR over time during treatment of a group of 51 patients suffering from type 2 diabetes mellitus with compound NOX-E36, and for a period of 84 days after administration of compound NOX-E36 had been terminated (follow-up);
- Fig. 9: shows ACR over time during treatment of a group of 51 patients suffering from type 2 diabetes mellitus with compound NOX-E36 or a placebo, and for a period of 84 days after administration of compound NOX-E36 and of the placebo had been terminated (follow-up);
- Fig. 10: shows HbAlc titer in the blood over time during treatment of a group of 51 patients suffering from type 2 diabetes mellitus with compound NOX-E36, and for a period of 28 days after administration of compound NOX-E36 had been terminated (follow-up);
- Fig. 11: shows HbAlc titer in the blood over time during treatment of a group of 51 patients suffering from type 2 diabetes mellitus with compound NOX-E36 or a placebo, and for a period of 28 days after administration of compound NOX-E36 and of the placebo had been terminated (follow-up);
- Fig. 12: shows plasma concentration of compound emapticap pegol (NOX-E36) over time during treatment of a group of 75 patients suffering from type 2 diabetes mellitus with compound NOX-E36 and 28 days after administration of compound NOX-E36 had been terminated;
- Fig. 13: shows ACR over time during treatment of a group of 75 patients suffering from type 2 diabetes mellitus with compound emapticap pegol (NOX-E36) or a placebo, and for a period of 84 days after administration of compound NOX-E36 and of the placebo had been terminated (follow-up);
- Fig. 14: shows the percent change in ACR at day 85 compared to baseline ACR of a group of 75 patients suffering from type 2 diabetes mellitus either treated with placebo (left bar) or with emapticap pegol (NOX-E36) (middle bar); and the relative change in ACR at day 85 of a group of 75 patients suffering from type 2 diabetes mellitus when the ACR of said patients treated with NOX-E36 is compared to the ACR of said patients treated with placebo;
- Fig. 15: shows HbAlc titer in the blood over time during treatment of a group of 75 patients suffering from type 2 diabetes mellitus with emapticap pegol (NOX-E36) or a placebo, and for a period of 28 days after administration of compound NOX-E36 and of the placebo had been terminated (follow-up); and
- Fig. 16: shows the precent change in HbAlc at day 85 compared to baseline HbAlc of a group of 75 patients suffering from type 2 diabetes mellitus either treated with placebo (left bar) or with emapticap pegol (NOX-E36) (middle bar); and the relative change in HbAlc at day 85 of a group of 75 patients suffering from type 2 diabetes mellitus when the HbAlc of said patients treated with NOX-E36 is compared to the HbAlc of said patients treated with placebo.

### Examples

In the following the terms 'nucleic acid' and 'nucleic acid molecule' are used herein in a synonymous manner if not indicated to the contrary. Moreover, the terms 'stretch' and 'stretch of nucleotide' are used herein in a synonymous manner if not indicated to the contrary. In the following the terms 'MCP-1' and 'CCL2' are used herein in a synonymous manner if not indicated to the contrary.

### Example 1: Nucleic acid molecules that bind human MCP-1/CCL2

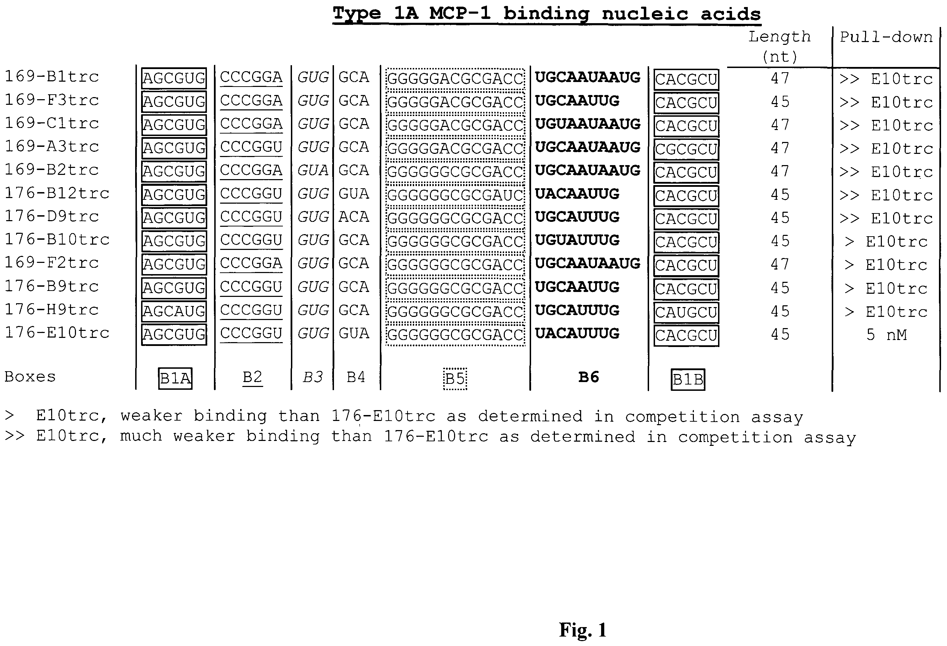

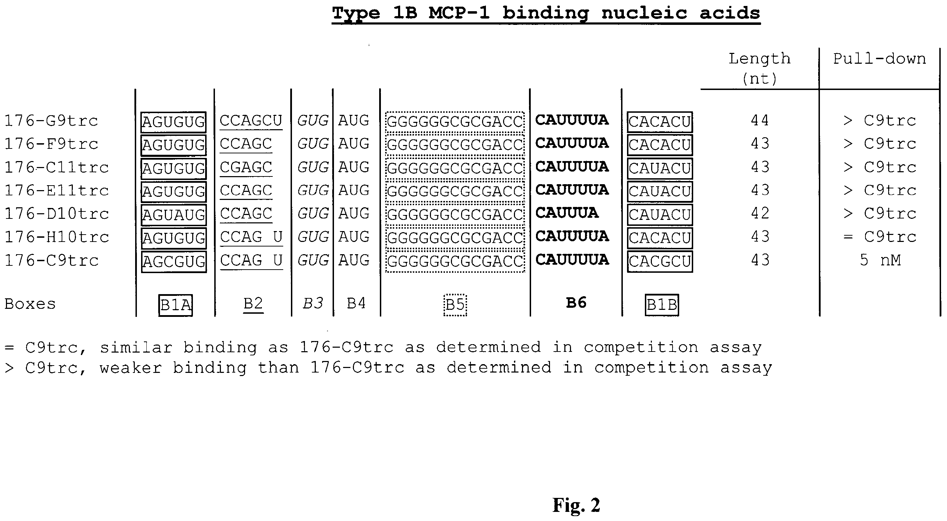

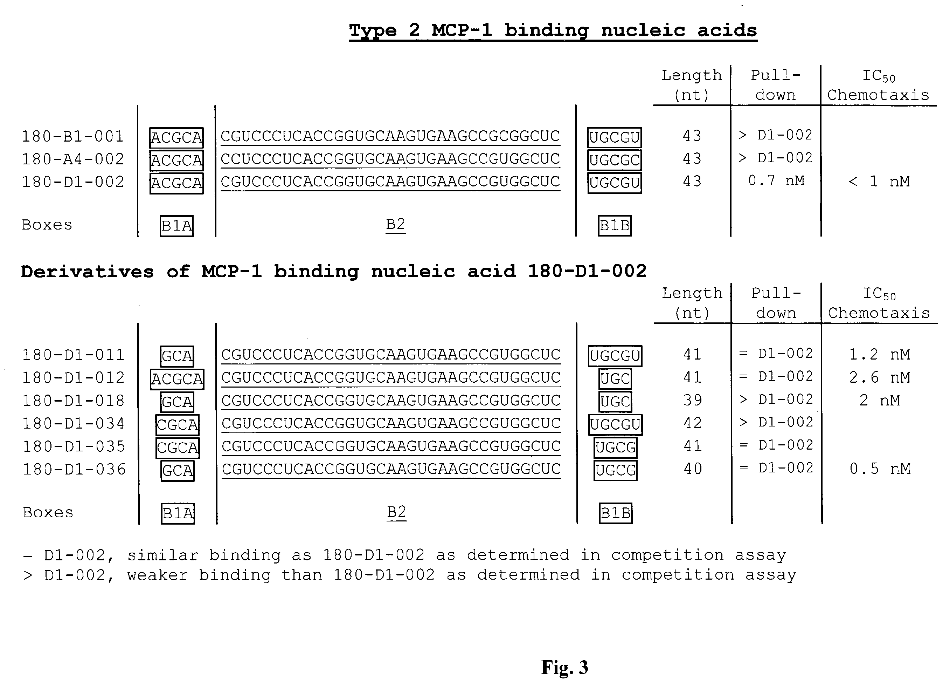

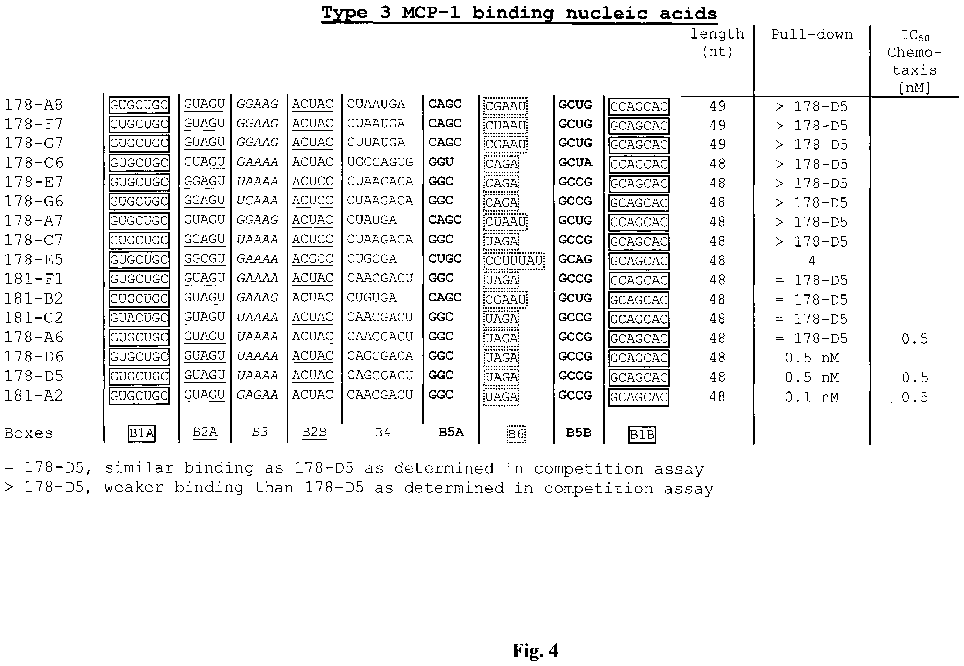

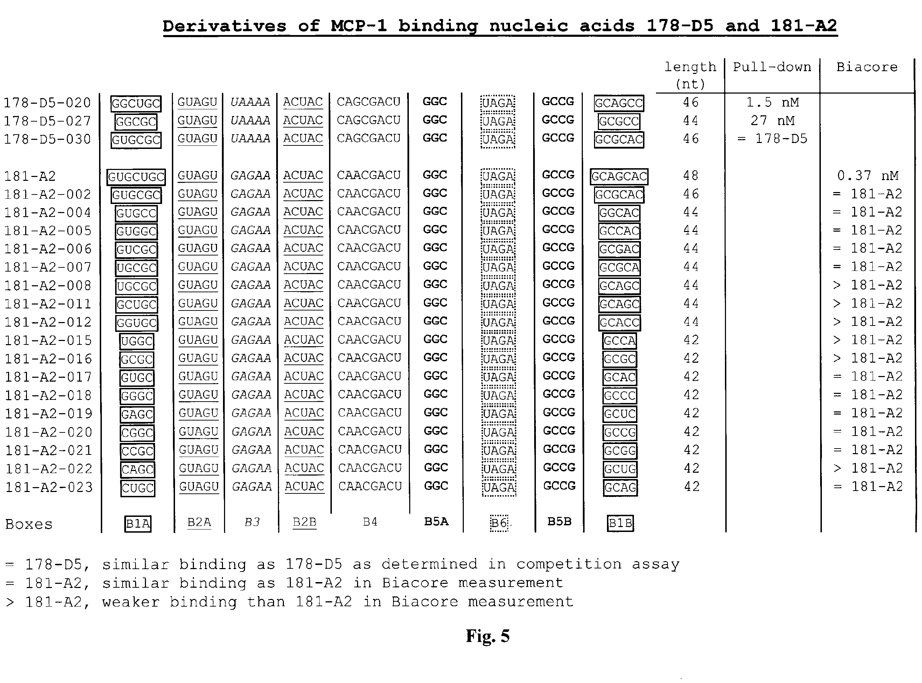

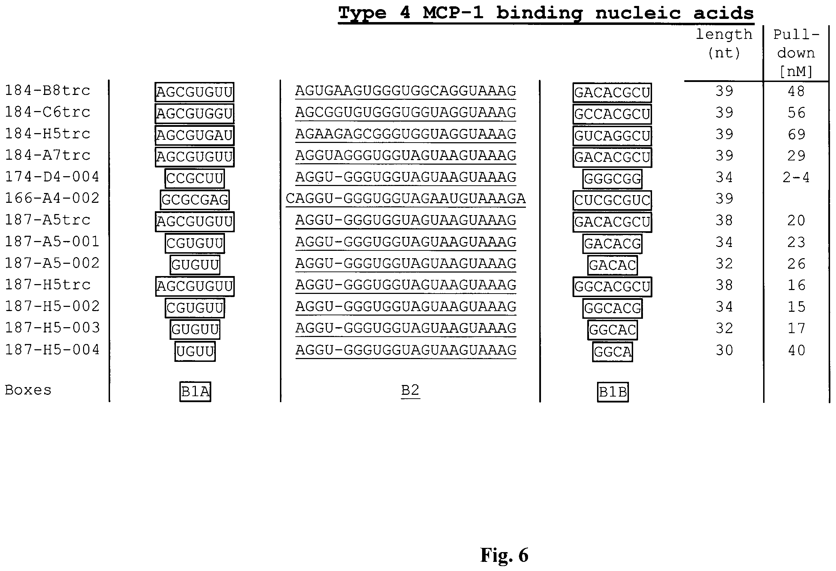

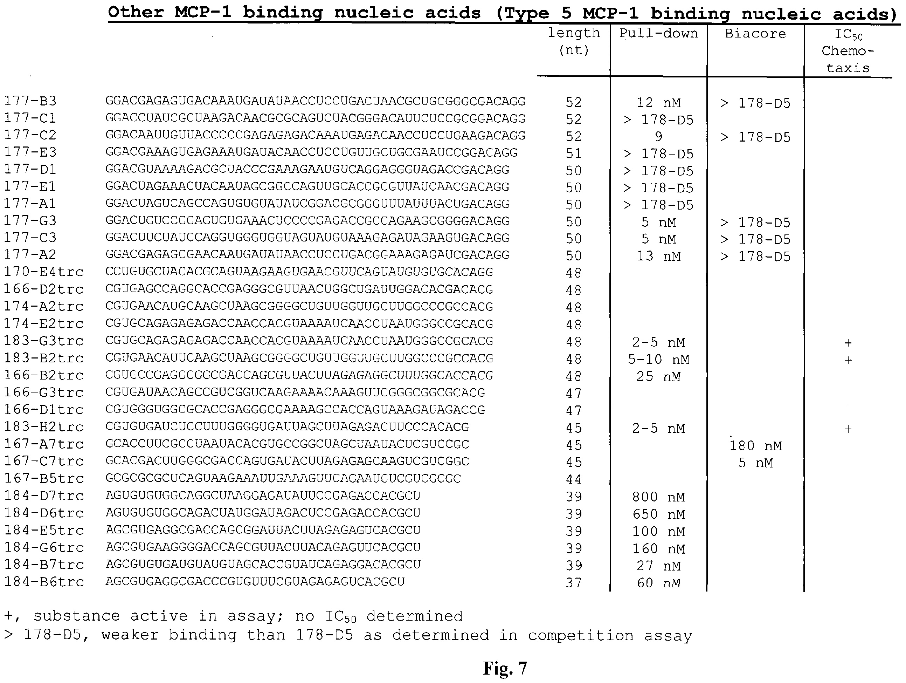

L-nucleic acid molculess that bind to human MCP-1 and their respective nucleotide sequences are depicted in Figures 1 to 7. The nucleic acid molecules exhibit different sequence motifs, four main types are defined in Figs. 1 and 2 (Type 1A / 1B), Fig. 3 (Type 2), Figs. 4 and 5 (Type 3), and Fig. 6 (Type 4), additional MCP-1 binding nucleic acid molecules which can not be related to each other and to the differerent sequence motifs decribed herein, are listed in Fig. 7 and are also referred to as type 5.

For definition of nucleotide sequence motifs, the IUPAC abbreviations for ambiguous nucleotides is used:

| | | |
|---|---|---|
| S | strong | G or C; |
| W | weak | A or U; |
| R | purine | G or A; |
| Y | pyrimidine | C or U; |
| K | keto | G or U; |
| M | imino | A or C; |
| B | not A | C or U or G; |
| D | not C | A or G or U; |
| H | not G | A or C or U; |
| V | not U | A or C or G; |
| N | all | A or G or C or U |

If not indicated to the contrary, any nucleic acid sequence or sequence of stretches and boxes, respectively, is indicated in the 5' → 3' direction.

The nucleic acid molecules were characterized on the aptamer level, i.e. as D-nucleic acid molecules, using direct and competitive pull-down assays with biotinylated human D-MCP-1 in order to rank them with respect to their binding behaviour (for protocol, see Example 3). Selected sequences were synthesized as Spiegelmer (for protocol, see Example 2) and were tested using the natural configuration of MCP-1 (L-MCP) in an *in vitro* chemotaxis assay (for protocol, see Example 4) or by surface plasmon resonance measurement using a Biacore 2000 instrument (for protocol, see Example 5).

### Type 1A MCP-1 binding nucleic acid molecules

As depicted in Fig. 1 all sequences of MCP-1 binding nucleic acid molcules of Type 1A comprise several sequences stretches of nucleotides or boxes whereby boxes 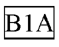 and 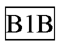 are the 5'- and 3' terminal stretches of nucleotides (also referred to as first terminal stretch of nucleotides and second stretch of nucleotides) that can hybridize with each other. However, such hybridization is not necessarily given in the molecule as actually present under physiological conditions. Boxes B2, *B3,* B4, B5 and box **B6** are flanked by box 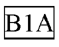 and box 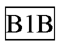 .

The sequences of the defined boxes may be different between the MCP-1 binding nucleic acids of Type 1A which influences the binding affinity to MCP-1. Based on binding analysis of the different MCP-1 binding nucleic acids summarized as ***Type 1A MCP-1 binding nucleic acids,*** the boxes 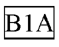 , B2, *B3*, B4, B5, **B6** and 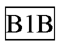 and their nucleotide sequences as described in the following are individually and more preferably in their entirety essential for binding to MCP-1:
- boxes 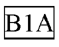 and 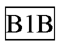 are the first and the second terminal stretch of nucleotides (also referred to as 5' and 3' terminal stretch of nucleotides), whereby both stretches of nucleotides can hybridize with each other; where 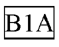 is 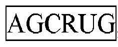 , preferably 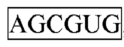 ; and whereby 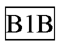 is 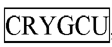 , preferably 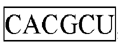 ;
- box B2 is the first central stretch of nucleotides, which is CCCGGW, preferably CCCGGU;
- box *B3* is the second central stretch of nucleotides, which is *GUR,* preferably *GUG;*
- box B4 is the third central stretch of nucleotides, which is RYA, preferably GUA;
- box B5 is the fourth central stretch of nucleotides, which is 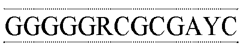 ; preferably 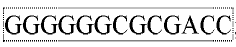 ;
- box **B6** is the fifth central stretch of nucleotides, which is **UGCAAUAAUG** or **URYAWUUG,** preferably **UACAUUUG.**

As depicted in Fig. 1, the nucleic acid molecule referred to as 176-E10trc has the best binding affinity to MCP-1 with a K_{D} of 5 nM (protocol, see Example 3) and therefore may constitute the optimal sequence and the optimal combination of sequence elements 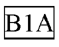 , B2, *B3*, B4, 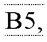 **B6** and 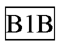 .

### Type 1B MCP-1 binding nucleic acid molecules

As depicted in Fig. 2, all sequences of Type 1B comprise several sequences stretches of nucleotides or boxes whereby boxes 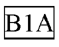 and 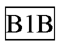 are the 5'- and 3' terminal stretches of nucleotides (also referred to as first terminal stretch of nucleotides and second stretch of nucleotides) that can hybridize with each other and boxes B2, *B3*, B4, 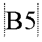 and box **B6** are flanked by box 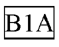 and box 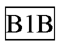 . However, such hybridization is not necessarily given in the molecule as actually present under physiological conditions.

The sequences of the defined boxes may be different between the MCP-1 binding nucleic acids of Type 1B which influences the binding affinity to MCP-1. Based on binding analysis of the different MCP-1 binding nucleic acids summarized as ***Type 1B MCP-1 binding nucleic acids,*** the boxes 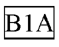 , B2, *B3*, B4, 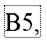 **B6** and 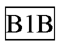 and their nucleotide sequences as described in the following are individually and more preferably in their entirety essential for binding to MCP-1:
- boxes 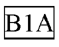 and 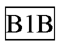 are the first and the second terminal stretch of nucleotides (also referred to as 5' and 3' terminal stretch of nucleotides), whereby both stretches of nucleotides can hybridize with each other, where 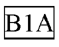 is 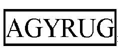 , preferably 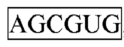 ; and where 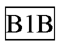 is 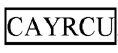 , preferably 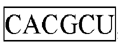 ;
- box B2 is the first central stretch of nucleotides, which is CCAGCU or CCAGY, preferably CCAGU;
- box *B3* is the second central stretch of nucleotides, which is *GUG;*
- box B4 is the third central stretch of nucleotides, which is AUG;
- box 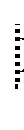 B5 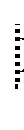 is the fourth central stretch of nucleotides, which is 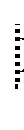 GGGGGGCGCGACC 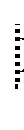 ;
- box **B6** is the fifth central stretch of nucleotides, which is **CAUUUUA** or **CAUUUA,** preferably **CAUUUUA.**

As depicted in Fig. 2, the nucleic acid referred to as 176-C9trc has the best binding affinity to MCP-1 with a K_{D} of 5 nM (protocol, see Example 3) and therefore may constitute the optimal sequence and the optimal combination of sequence elements 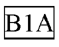 , B2, *B3*, B4, B5, **B6** and 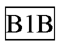 .

### Type 2 MCP-1 binding nucleic acid molecules

As depicted in Fig. 3, all sequences of Type 2 comprise several sequences stretches of nucleotides or boxes whereby boxes 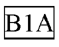 and 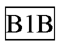 are the 5'- and 3' terminal stretches of nucleotides (also referred to as first terminal stretch of nucleotides and second stretch of nucleotides) that can hybridize with each other and box B2 is the central sequence element. However, such hybridization is not necessarily given in the molecule as actually present under physiological conditions.

The sequences of the defined boxes may be different between the MCP-1 binding nucleic acids of Type 3 which influences the binding affinity to MCP-1. Based on binding analysis of the different MCP-1 binding nucleic acids summarized as ***Type 2 MCP-1 binding nucleic acids,*** the boxes 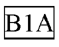 , B2, and 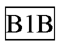 and their nucleotide sequences as described in the following are individually and more preferably in their entirety essential for binding to MCP-1:
- boxes 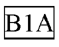 and 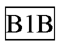 are the first and the second terminal stretch of nucleotides (also referred to as 5' and 3' terminal stretch of nucleotides), whereby both stretches of nucleotides can hybridize with each other, whereby 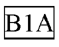 is 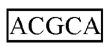 and 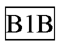 is 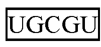 , or 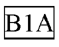 is 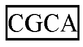 and 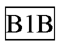 is 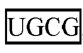 , or 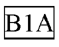 is 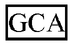 and 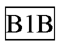 is 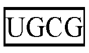 or 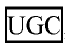 ; preferably 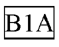 is 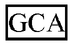 and 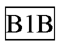 is 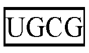 ;
- box B2 is the central stretch of nucleotides, CSUCCCUCACCGGUGCAAGUGAAGCCGYGGCUC, preferably CGUCCCUCACCGGUGCAAGUGAAGCCGUGGCUC.

As depicted in Fig. 3, the nucleic acid referred to as 180-D1-002 as well as the derivatives of 180-D1-002 like 180-D1-011, 180-D1-012, 180-D1-035, and 180-D1-036 have the best binding affinity to MCP-1 as aptamer in the pull-down or competitive pull-down assay with an K_{D} of < 1 nM (protocol, see Example 3) and therefore may constitute the optimal sequence and the optimal combination of sequence elements 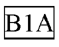 , B2, and 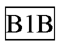 .

For nucleic acid molecule 180-D1-036, a dissociation constant (K_{D}) of 890 ± 65 pM at room temperature and of 146 ± 13 pM at 37°C was determined (protocol, see Example 3). The respective Spiegelmer 180-D1-036 exhibited an inhibitory concentration (IC₅₀) of ca. 0.5 nM in an *in vitro* chemotaxis assay (protocol, see Example 4). For the PEGylated derivatives of Spiegelmer 180-D1-036, 180-D1-036-3'PEG and 180-D1-036-5'PEG, an IC₅₀s of < 1 nM in the chemotaxis assay was determined (protocol, see Example 4), whereas in the cell culture experiments as Spiegelmer 180-D1-036-5'PEG Spiegelmer NOX-E36 was used. Spiegelmer NOX-E36 (also referred to as emapticap pegol) is a specific variant of Spiegelmer 180-D1-036-5'PEG comprising a 40kDa-PEG that is linked by specific linker to its nucleotide sequence (see Table 1; SEQ ID NO. 228).

### Type 3 MCP-1 binding nucleic acid molecules

As depicted in Figs. 4 and 5, all sequences of Type 3 comprise several sequence stretches of nucleotides or boxes whereby three pairs of boxes are characteristic for ***Type 3 MCP-1 binding nucleic acids.*** Both boxes 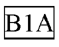 and 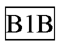 as well as boxes B2A and B2B as well as boxes **B5A** and **B5B** bear the ability to hybridize with each other. However, such hybridization is not necessarily given in the molecule as actually present under physiological conditions. Between these potentially hybridized sequence elements, non-hybridizing nucleotides are located, defined as box *B3*, box B4 and box 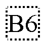 .

The sequences of the defined boxes may be different between the MCP-1 binding nucleic acids of Type 3 which influences the binding affinity to MCP-1. Based on binding analysis of the different MCP-1 binding nucleic acids summarized as ***Type 3 MCP-1 binding nucleic acids,*** the boxes 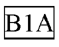 , B2A, *B3*, B2B, B4, **B5A,** 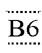 , **B5B,** 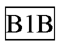 and their nucleotide sequences as described in the following are individually and more preferably in their entirety essential for binding to MCP-1:
- boxes 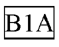 and 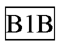 are the first and the second terminal stretch of nucleotides (also referred to as 5' and 3' terminal stretch of nucleotides), whereby both stretches of nucleotides can hybridize with each other, whereby 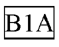 is 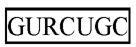 and 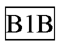 is 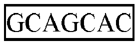 ; preferably 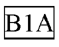 is 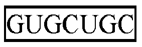 and 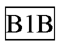 is 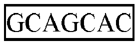 ;
   or 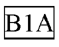 is 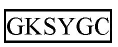 and 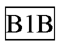 is 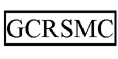 ; preferably 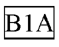 is 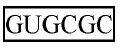 and 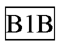 is GCGCAC;
   or 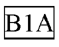 is 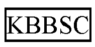 and 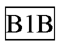 is 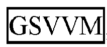 ; preferably 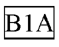 is 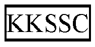 and 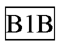 is 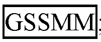 ; or 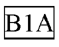 is 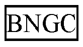 and 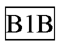 is 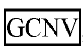 ; preferably 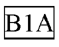 is 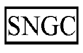 and 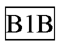 is 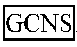 ; most preferably 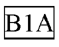 is 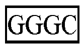 and 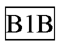 is 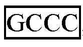 ;
- boxes B2A and B2B are the first and the third central stretch of nucleotides, whereby both stretches of nucleotides can hybridize with each other, whereby B2A is GKMGU and B2B is ACKMC; preferably B2A is GUAGU and B2B is ACUAC;
- box *B3* is the second central stretch of nucleotides, which is *KRRAR,* preferably *UAAAA* or *GAGAA;*
- box B4 is the fourth central stretch of nucleotides, which is CURYGA or CUWAUGA or CWRMGACW or UGCCAGUG, preferably CAGCGACU or CAACGACU;
- **B5A** and **B5B** are the fifth and the seventh central stretch of nucleotides, whereby both stretches can hybridize with each other, **B5A** is **GGY** and **B5B** is **GCYR** whereas **GCY** can hybridize with the nucleotides of **B5A**; or **B5A** is **CWGC** and **B5B** is **GCWG;** preferably **B5A** is **GGC** and **B5B** is **GCCG;**
- box B6 is the sixth central stretch of nucleotides, which is: 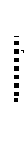 YAGA 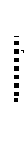 or 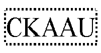 or CCUUUAU, preferably UAGA.

As depicted in Figs. 4 and 5, the nucleic acid referred to as 178-D5 and its derivative 178-D5-030 as well as 181-A2 with its derivatives 181-A2-002, 181-A2-004, 181-A2-005, 181-A2-006, 181-A2-007, 181-A2-017, 181-A2-018, 181-A2-019, 181-A2-020, 181-A2-021, and 181-A2-023 have the best binding affinity to MCP-1. 178-D5 and 178-D5-030 were evaluated as aptamers in direct or competitive pull-down assays (protocol, see Example 3) with an K_{D} of approx. 500 pM. In the same experimental set-up, 181-A2 was determined with an K_{D} of approx. 100 pM. By Biacore analysis (protocol, see Example 5), the K_{D} of 181-A2 and its derivatives towards MCP-1 was determined to be 200 - 300 pM. In chemotaxis assays with cultured cells (protocol, see Example 4), for both 178-D5 and 181-A2, an IC₅₀ of approx. 500 pM was measured. Therefore, 178-D5 as well as 181-A2 and their derivatives may constitute the optimal sequence and the optimal combination of sequence elements 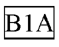 , B2A, *B3*, B2B, B4, **B5A,** 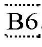 B6, **B5B** and 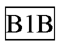 .

### Type 4 MCP-1 binding nucleic acids

As depicted in Fig. 6, all sequences of Type 4 comprise several sequences, stretches of nucleotides or boxes whereby boxes 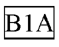 and 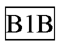 are the 5'- and 3' terminal stretches (also referred to as first terminal stretch of nucleotides and second stretch of nucleotides) that can hybridize with each other and box B2 is the central sequence element.

The sequences of the defined boxes may differ among the MCP-1 binding nucleic acids of Type 4 which influences the binding affinity to MCP-1. Based on binding analysis of the different MCP-1 binding nucleic acids summarized as ***Type 4 MCP-1 binding nucleic acids,*** the boxes 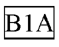 , B2, and 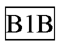 and their nucleotide sequences as described in the following are individually and more preferably in their entirety essential for binding to MCP-1:
- boxes 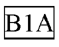 and 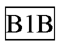 the first and the second terminal stretch of nucleotides (also referred to as 5' and 3' terminal stretch of nucleotides), whereby both stretches can hybridize with each other, whereby 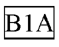 is 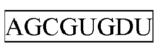 and 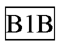 is 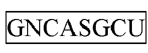 ; or 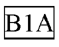 is 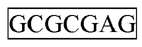 and 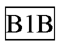 is 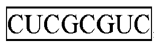 ; or 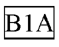 is 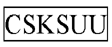 and 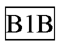 is 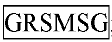 ; or 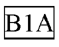 is 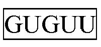 and 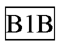 is 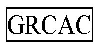 ; or 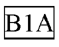 is 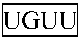 and 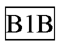 is 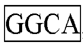 GGCA; preferably 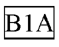 is 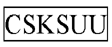 and 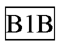 is 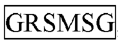 ; mostly preferred 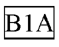 is 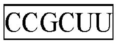 CCGCUU and 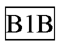 is GGGCGG; and
- box B2 is the central stretch of nucleotides, which is AGNDRDGBKGGURGYARGUAAAG or AGGUGGGUGGUAGUAAGUAAAG or CAGGUGGGUGGUAGAAUGUAAAGA, preferably AGGUGGGUGGUAGUAAGUAAAG.

As depicted in Fig. 6, the nucleic acid referred to as 174-D4-004 and 166-A4-002 have the best binding affinity to MCP-1 and may, therefore, constitute the optimal sequence and the optimal combination of sequence elements 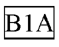 , B2, and 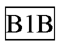 .

### Other MCP-1 binding nucleic acid molecules

Additionally, the 29 other MCP-1 binding nucleic acids shown in Fig. 7 cannot be described by a combination of nucleotide sequence elements as has been shown for Types 1 - 4 of MCP-1 binding nucleic acids.

It is to be understood that any of the sequences shown in Figs. 1 through 7 are nucleic acid molecules according to the present invention, including those truncated forms thereof but also including those extended forms thereof under the proviso, however, that the thus truncated and extended, respectively, nucleic acid molecules are still capable of binding to the target.

### Example 2: Synthesis and derivatization of Aptamers and Spiegelmers

### Small scale synthesis

Aptamers (D-RNA nucleic acids) and Spiegelmers (L-RNA nucleic acids) were produced by solid-phase synthesis with an ABI 394 synthesizer (Applied Biosystems, Foster City, CA, USA) using 2'TBDMS RNA phosphoramidite chemistry (Damha and Ogilvie, 1993). rA(N-Bz)-, rC(Ac)-, rG(N-ibu)-, and rU- phosphoramidites in the D- and L-configuration were purchased from ChemGenes, Wilmington, MA. Aptamers and Spiegelmers were purified by gel electrophoresis.

### Large scale synthesis plus modification

Spiegelmers were produced by solid-phase synthesis with an ÄktaPilot100 synthesizer (Amersham Biosciences; General Electric Healthcare, Freiburg) using 2'TBDMS RNA phosphoramidite chemistry (Damha and Ogilvie, 1993). L-rA(N-Bz)-, L-rC(Ac)-, L-rG(N-ibu)-, and L-rU- phosphoramidites were purchased from ChemGenes, Wilmington, MA. The 5'-aminomodifier was purchased from American International Chemicals Inc. (Framingham, MA, USA). Synthesis of the unmodified or 5'-Amino-modified Spiegelmer was started on L-riboG, L-riboC, L-riboA or L-riboU modified CPG pore size 1000 Å (Link Technology, Glasgow, UK, for the 3'-NH₂-modified Spiegelmer, 3'-Aminomodifier-CPG, 1000 Å (ChemGenes, Wilmington, MA) was used. For coupling (15 min per cycle), 0.3 M benzylthiotetrazole (CMS-Chemicals, Abingdon, UK) in acetonitrile, and 3.5 equivalents of the respective 0.1 M phosphoramidite solution in acetonitrile was used. An oxidation-capping cycle was used. Further standard solvents and reagents for oligonucleotide synthesis were purchased from Biosolve (Valkenswaard, NL). The Spiegelmer was synthesized DMT-ON; after deprotection, it was purified via preparative RP-HPLC (Wincott et al., 1995) using Source15RPC medium (Amersham). The 5'DMT-group was removed with 80% acetic acid (30 min at RT). Subsequently, aqueous 2 M NaOAc solution was added and the Spiegelmer was desalted by tangential-flow filtration using a 5 K regenerated cellulose membrane (Millipore, Bedford, MA).

### PEGylation of Spiegelmers

In order to prolong the Spiegelmer's plasma residence time *in vivo,* the Spiegelmers were covalently coupled to a 40 kDa polyethylene glycol (PEG) moiety at the 3'-end or 5'-end.

For PEGylation (for technical details of the method for PEGylation see European patent application EP 1 306 382), the purified 5'-amino or 3'-amino modified Spiegelmer was dissolved in a mixture of H₂O (2.5 ml), DMF (5 ml), and buffer A (5 ml; prepared by mixing citric acid · H₂O [7 g], boric acid [3.54 g], phosphoric acid [2.26 ml], and 1 M NaOH [343 ml] and adding water to a final volume of 11; pH = 8.4 was adjusted with 1 M HCl).

The pH of the Spiegelmer solution was brought to 8.4 with 1 M NaOH. Then, 40 kDa PEG-NHS ester (Jenkem Technology, Allen, TX, USA) was added at 37°C every 30 min in six portions of 0.25 equivalents until a maximal yield of 75 to 85% was reached. The pH of the reaction mixture was kept at 8 - 8.5 with 1 M NaOH during addition of the PEG-NHS ester.

The reaction mixture was blended with 4 ml urea solution (8 M), and 4 ml buffer B (0.1 M triethylammonium acetate in H₂O) and heated to 95°C for 15 min. The PEGylated Spiegelmer was then purified by RP-HPLC with Source 15RPC medium (Amersham), using an acetonitrile gradient (buffer B; buffer C: 0.1 M triethylammonium acetate in acetonitrile). Excess PEG eluted at 5% buffer C, PEGylated Spiegelmer at 10 - 15% buffer C. Product fractions with a purity of >95% (as assessed by HPLC) were combined and mixed with 40 ml 3 M NaOAc. The PEGylated Spiegelmer was desalted by tangential-flow filtration (5 K regenerated cellulose membrane, Millipore, Bedford MA).

### Example 3: Determination of Binding Constants (Pull-Down Assay)

### Direct pull-down assay

The affinity of aptamers to D-MCP-1 was measured in a pull down assay format at 20 or 37°C, respectively. Aptamers were 5'-phosphate labeled by T4 polynucleotide kinase (Invitrogen, Karlsruhe, Germany) using [γ-³²P]-labeled ATP (Hartmann Analytic, Braunschweig, Germany). The specific radioactivity of labeled aptamers was 200,000 - 800,000 cpm/pmol. Aptamers were incubated after de- and renaturation at 20 pM concentration at 37°C in selection buffer (20 mM Tris-HCl pH 7.4; 137 mM NaCl; 5 mM KCl; 1 mM MgCl₂; 1 mM CaCl₂; 0.1% [w/vol] Tween-20) together with varying amounts of biotinylated D-MCP-1 for 4 - 12 hours in order to reach equilibrium at low concentrations. Selection buffer was supplemented with 10 µg/ml human serum albumin (Sigma-Aldrich, Steinheim, Germany), and 10 µg/ml yeast RNA (Ambion, Austin, USA) in order to prevent adsorption of binding partners with surfaces of used plasticware or the immobilization matrix. The concentration range of biotinylated D-MCP-1 was set from 8 pM to 100 nM; total reaction volume was 1 ml. Peptide and peptide-aptamer complexes were immobilized on 1.5 µl Streptavidin Ultralink Plus particles (Pierce Biotechnology, Rockford, USA) which had been preequilibrated with selection buffer and resuspended in a total volume of 6 µl. Particles were kept in suspension for 30 min at the respective temperature in a thermomixer. Immobilized radioactivity was quantitated in a scintillation counter after detaching the supernatant and appropriate washing. The percentage of binding was plotted against the concentration of biotinylated D-MCP-1 and dissociation constants were obtained by using software algorithms (GRAFIT; Erithacus Software; Surrey U.K.) assuming a 1:1 stoichiometry.

### Competitive pull-down assay

In order to compare different D-MCP-1 binding aptamers, a competitive ranking assay was performed. For this purpose the most affine aptamer available was radioactively labeled (see above) and served as reference. After de- and renaturation it was incubated at 37°C with biotinylated D-MCP-1 in 1 ml selection buffer at conditions that resulted in around 5 - 10 % binding to the peptide after immobilization and washing on NeutrAvidin agarose or Streptavidin Ultralink Plus (both from Pierce) without competition. An excess of de- and renatured non-labeled D-RNA aptamer variants was added to different concentrations (e.g. 2, 10, and 50 nM) with the labeled reference aptamer to parallel binding reactions. The aptamers to be tested competed with the reference aptamer for target binding, thus decreasing the binding signal in dependence of their binding characteristics. The aptamer that was found most active in this assay could then serve as a new reference for comparative analysis of further aptamer variants.

### Example 4: Analysis of the inhibition of MCP-1 induced chemotaxis by MCP-1-binding Spiegelmers

THP-1 cells grown as described above were centrifuged, washed once in HBH (HBSS, containing 1 mg/ml bovine serum albumin and 20 mM HEPES) and resuspended at 3 x 10⁶ cells/ml. 100 µl of this suspension were added to Transwell inserts with 5 µm pores (Corning, #3421). In the lower compartments MCP-1 was preincubated together with Spiegelmers in various concentrations in 600 µl HBH at 37°C for 20 to 30 min prior to addition of cells. Cells were allowed to migrate at 37°C for 3 hours. Thereafter the inserts were removed and 60 µl of 440 µM resazurin (Sigma) in phosphate buffered saline was added to the lower compartments. After incubation at 37°C for 2.5 hours, fluorescence was measured at an excitation wavelength of 544 nm and an emission wavelength of 590 nm in a Fluostar Optima multidetection plate reader (BMG).

### Determination of half-maximal effective concentration (EC₅₀) for human MCP-1

After 3 hours migration of THP-1 cells towards various human MCP-1 concentrations, a dose-response curve for human MCP-1 was obtained, indicating a maximal effective concentration of about 1 nM and reduced activation at higher concentrations. For the further experiments on inhibition of chemotaxis by Spiegelmers a MCP-1 concentration of 0.5 nM was used.

### Determination of half-maximal effective concentration (EC₅₀) for murine MCP-1

After 3 hours migration of THP-1 cells towards various murine MCP-1 concentrations, a dose-response curve for murine MCP-1 was obtained, indicating a maximal effective concentration of about 1 - 3 nM and reduced activation at higher concentrations. For the further experiments on inhibition of chemotaxis by Spiegelmers a murine MCP-1 concentration of 0.5 nM was used.

### Example 5: Binding Analysis by Surface Plasmon Resonance Measurement

The Biacore 2000 instrument (Biacore AB, Uppsala, Sweden) was used to analyze binding of Spiegelmers to the protein human MCP-1. When coupling of human MCP-1 was to be achieved via amine groups, human MCP-1 was dialyzed against water for 1 - 2 h (Millipore VSWP mixed cellulose esters; pore size, 0.025 µM) to remove interfering amines. CM4 sensor chips (Biacore AB, Uppsala, Sweden) were activated before protein coupling by a 35-µl injection of a 1:1 dilution of 0.4 M NHS and 0.1 M EDC at a flow of 5 µl/min. Human MCP-1 was then injected in concentrations of 0.1 - 1.5 µg/ml at a flow of 2 µl/min until the instrument's response was in the range of 1000 - 2000 RU (relative units). Unreacted NHS esters were deactivated by injection of 35 µl ethanolamine hydrochloride solution (pH 8.5) at a flow of 5 µl/min. The sensor chip was primed twice with binding buffer and equilibrated at 10 µl/min for 1 - 2 hours until the baseline appeared stable. For all proteins, kinetic parameters and dissociation constants were evaluated by a series of Spiegelmer injections at concentrations of 1000, 500, 250, 125, 62.5, 31.25, and 0 nM in selection buffer (Tris-HCl, 20 mM; NaCl, 137 mM; KCl, 5 mM; CaCl₂, 1 mM; MgCl₂, 1 mM; Tween20, 0.1% [w/v]; pH 7.4). In all experiments, the analysis was performed at 37°C using the Kinject command defining an association time of 180 and a dissociation time of 360 seconds at a flow of 10 µl/min. Data analysis and calculation of dissociation constants (K_{D}) was done with the BIAevaluation 3.0 software (BIACORE AB, Uppsala, Sweden) using the Langmuir 1:1 stochiometric fitting algorithm.

### Example 6: First results of a Phase IIa study for characterizing the effects of CCL2 inhibition with NOX-E36 in patients with type 2 diabetes mellitus and albuminuria

This study was a prospective, multi-center, randomized, double-blind, placebo-controlled, parallel group phase IIa study with multiple subcutaneous administrations in patients with type 2 diabetes mellitus and albuminuria who were receiving standard of care to control hypertension (ACEis and ARBs), hyperglycemia (oral antidiabetics and/or insulin) and dyslipidemia. The study consisted of (i) a screening period of up to 30 days duration, to ensure that the subject is stable on his/her concomitant therapy and life style, (ii) a treatment period of 12 weeks duration with twice a week injections of the study drug NOX-E36 (0.5 mg/kg) and regular examinations and blood samplings, and (iii) a treatment-free follow-up period of 12 weeks with a final visit and a full examination of the subject status.

Study drug NOX-E36 is an L-nucleic acid comprising a nucleotide sequence of SEQ ID NO: 228 and a 40 kDa PEG moiety attached to the 5' end of the nucleotide sequence.

### Main inclusion criteria

The Main inclusion criteria were as follows:
- Type 2 diabetes mellitus according to American Diabetes Association (ADA) definition
- HbAlc between 6.0% and 10.5%, inclusive
- ACR > 100 mg/g calculated 3 times in first morning void urine, at least 2 of the measurements > 100 mg/g
- Patients on stable (unchanged medication for at least 3 months) treatment to control hypertension, hyperglycemia and (if applicable) dyslipidemia
- Stable treatment with angiotensin-converting enzyme inhibitors (ACEis) or Angiotensin II receptor blockers (ARBs), i.e. renin-angiotensin system (RAS) blockade

### Main exclusion criteria

The main exclusion criteria were as follows:
- Type 1 diabetes mellitus
- eGFR ≤25 mL/min/1.73m²
- Recent cardiovascular events (3 months)
- Uncontrolled hypertension (upper limits 180/110 mmHg)
- Dialysis and/or acute kidney injury within 3 months before screening
- Significant edema, infectious diseases, leg ulcers
- Severe concurrent disease which, in the judgment of the investigator, would interfere significantly with the assessments of safety and efficacy during this study
- In the judgment of the clinical investigator, clinically significant abnormal laboratory values at the screening visit
- Use of thiazolidinedione class drugs, immune suppressants, steroid therapy (except for topical use or inhalation), chronic use of non-steroidal anti-inflammatory drug (NSAIDs), cyclooxygenase type 2 (COX-2) inhibitors, two or more diuretic drugs and/or aliskiren

### Efficacy evaluation

Efficacy evaluation was as follows:
The clinical response of study drug to CCL2 inhibition in patients with type 2 diabetes and albuminuria was assessed by means of the following parameters:
- ACR (albumine/creatinin ratio) calculated in first morning void urine
- HbAlc
- hsCRP in serum
- Further urine and serum/plasma markers of glycemic disorders, systemic inflammation, renal and liver disease and cardiovascular function
- Homeostasis Model of Insulin Resistance (HOMA-IR)
- Flow cytometric determination of CCR2 positive leukocyte subsets in peripheral blood
- Changes in blood pressure as marker of cardiovascular function

### Safety evaluation:

Safety evaluation was as follows
- Adverse events(AE)
- Physical examination
- Vital signs
- 12-lead electrocardiogram (ECG)
- Safety laboratory
- Immunogenicity assessment
- eGFR calculated on basis of serum creatinine level by the CKD-EPI formula and calculated by serum cystatin C

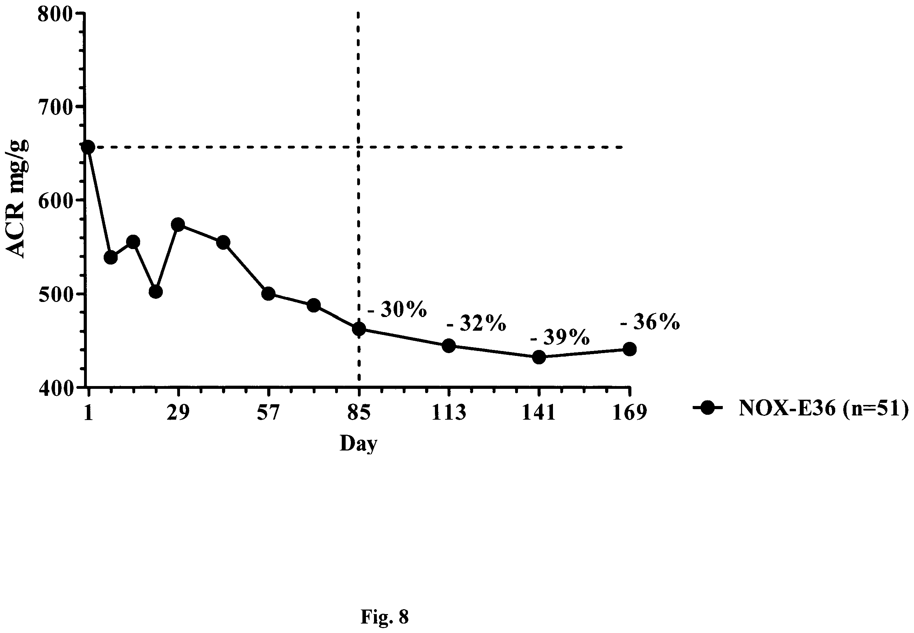

The results of the study are summarized in Fig. 8 to 11.

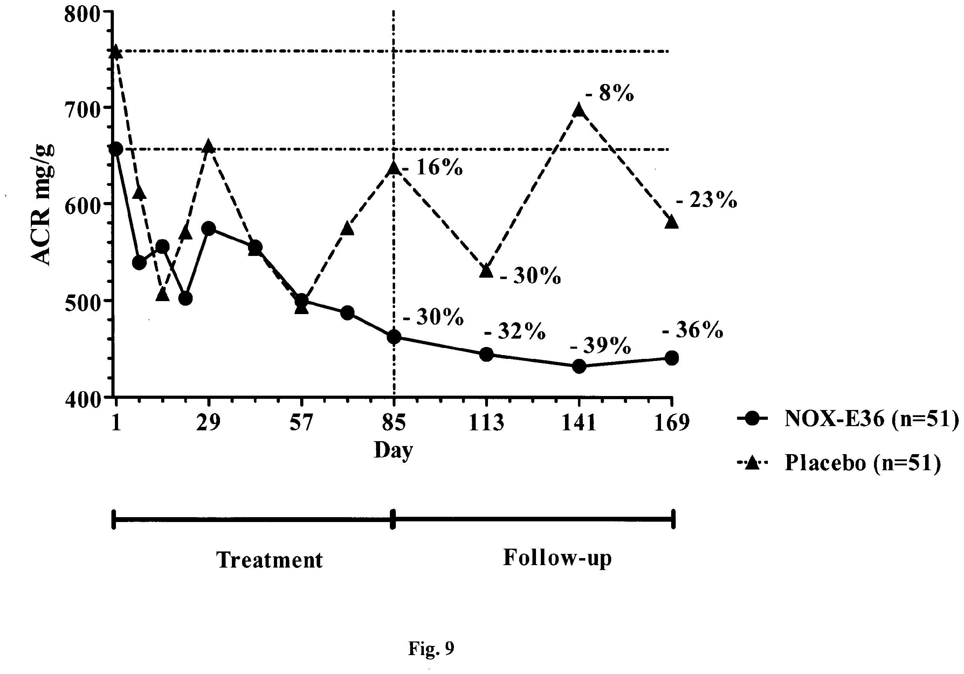

### Description of ACR results as shown in Figs. 8 and 9:

The ACR response during treatment and the follow up period of 3 months for 51 patients showed a low volatility and a steady decrease from day 29 until the end of treatment which is ongoing until day 141 (-39%) after which a slight re-increase at day 169 is suggestive of a fading treatment effect (-36%). In contrast to that, the high volatility of the placebo group was maintained throughout the follow-up period until day 169.

The observed persistence of the ACR response following cessation of treatment is very encouraging and has the potential to be a strong differentiating factor of compounds such as NOX-E36 from other compounds. This prolonged effect cannot be due to the persistence of NOX-E36 in the body for such long time as elimination of the compound had been shown on day 113 to pharmacologically irrelevant levels. As the effect was clearly maintained beyond the time of pharmacological exposure, it is suggestive of induction of a structural effect in the kidney.

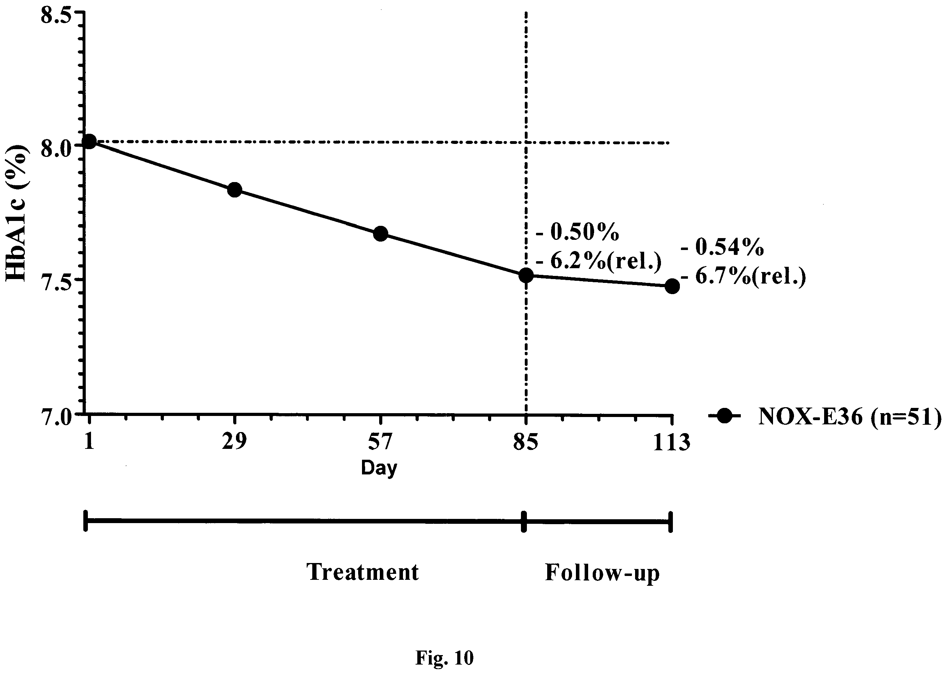

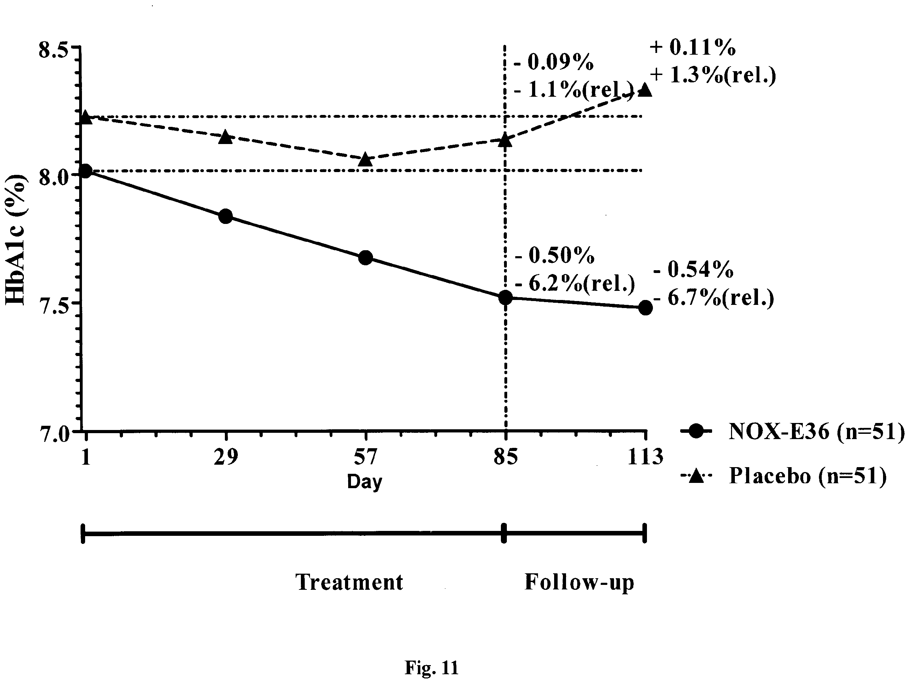

### Description of HbAlc results as shown in Figs. 10 and 11:

The HbAlc response during treatment and a follow-up period of 1 month for 51 patients showed a constant decrease followed by a slight further improvement resulting in an effect of -0.54% (abs.) on day 113. In contrast to that, the placebo group showed a time course with transient decrease during treatment and a rebound starting at day 57 resulting in an effect of +1.1% (abs.) at day 113.

### Example 7: Final results of a Phase IIa study for characterizing the effects of CCL2 inhibition with NOX-E36 in patients with type 2 diabetes mellitus and albuminuria

A total of 75 patients were enrolled in the phase IIa study described in Example 6. This example is related to the final results obtained from said phase IIa study, whereby for the primary efficacy analysis, patients with major protocol violations, treatment with dual RAS blockade and concomitant haematuria and leukocyturia were excluded.

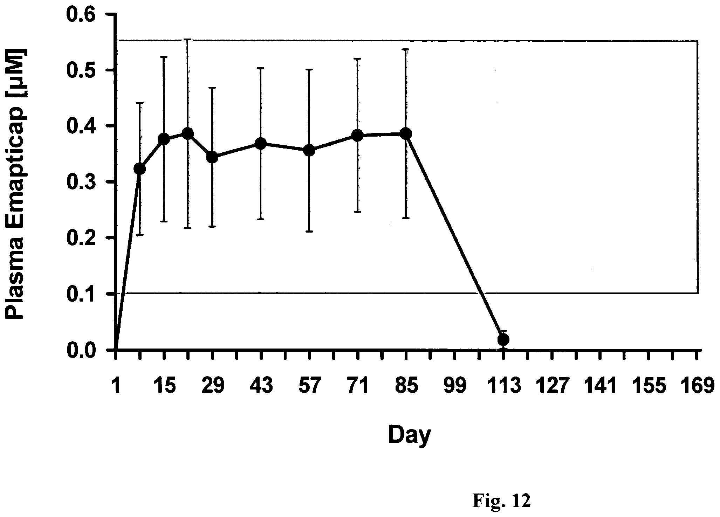

NOX-E36 was safe and well tolerated with a few mild local injection site reactions as the only relevant treatment-related adverse events. Plasma concentrations reached pharmacologically relevant levels of 358 ± 106 nM (Fig. 12) and the expected pharmacodynamic effect was observed, i.e. a change in the number of monocytes in peripheral blood which express CCR2.

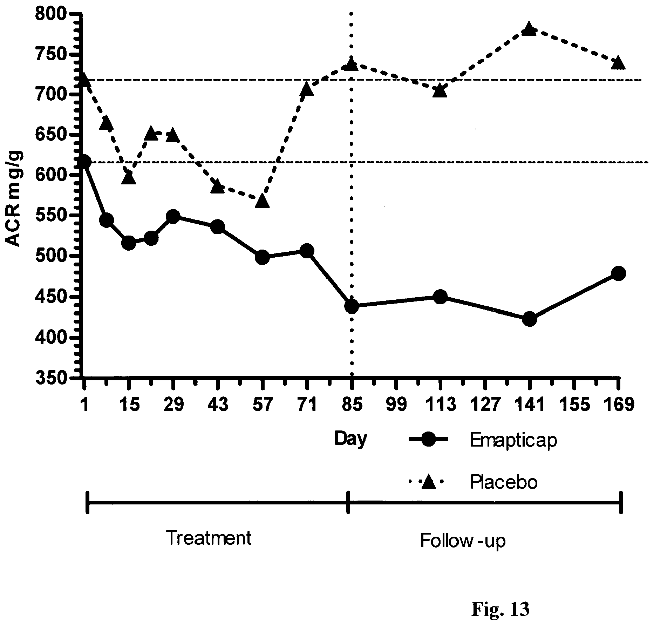

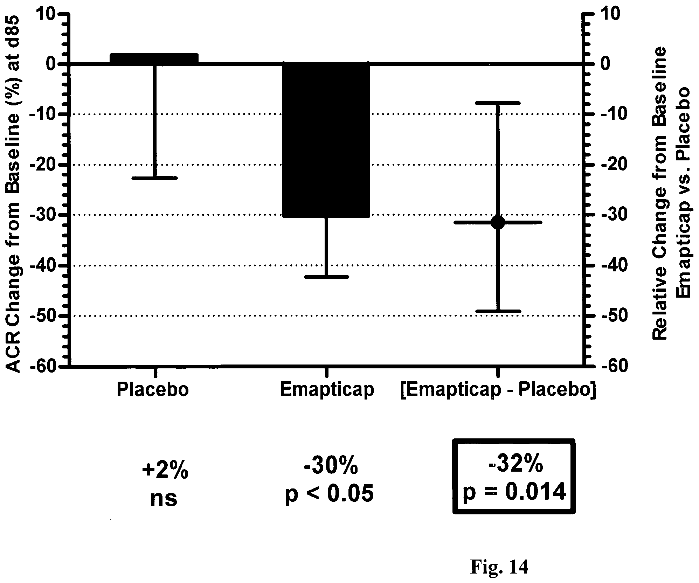

The time course of ACR during and after dosing is illustrated in Fig. 13. At the end of treatment on day 85, NOX-E36 reduced the mean ACR as compared with placebo by 32% (P=0.014; see Fig. 14) with a reduction of 50% or more in 31% of the patients who received NOX-E36 as compared with only 6% of those who received placebo. The therapeutic effect of NOX-E36 was maintained after the cessation of dosing until the end of the observation period (see Fig.13). The maximum effect on mean ACR (-39 % vs. placebo, P=0.010) was observed eight weeks after the last dose.

No relevant difference in blood pressure or eGFR was seen between the treatment groups throughout the study.

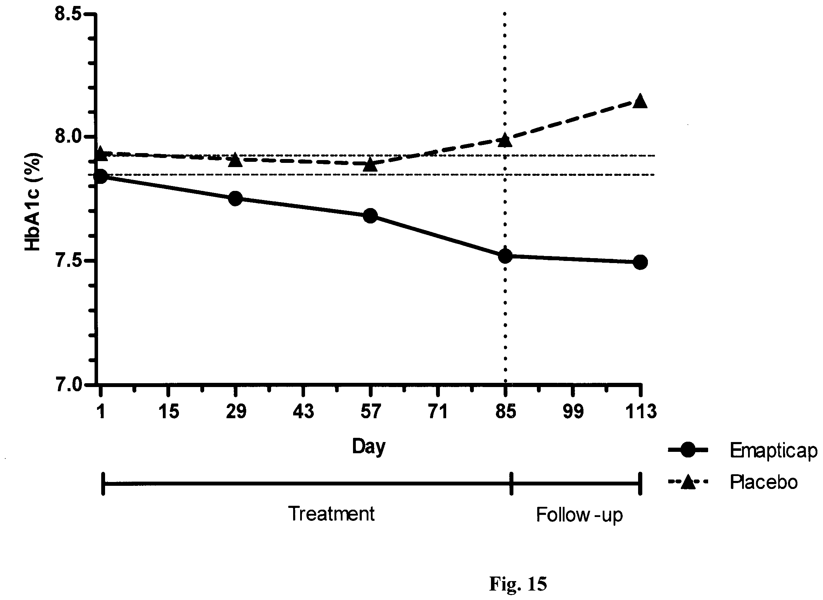

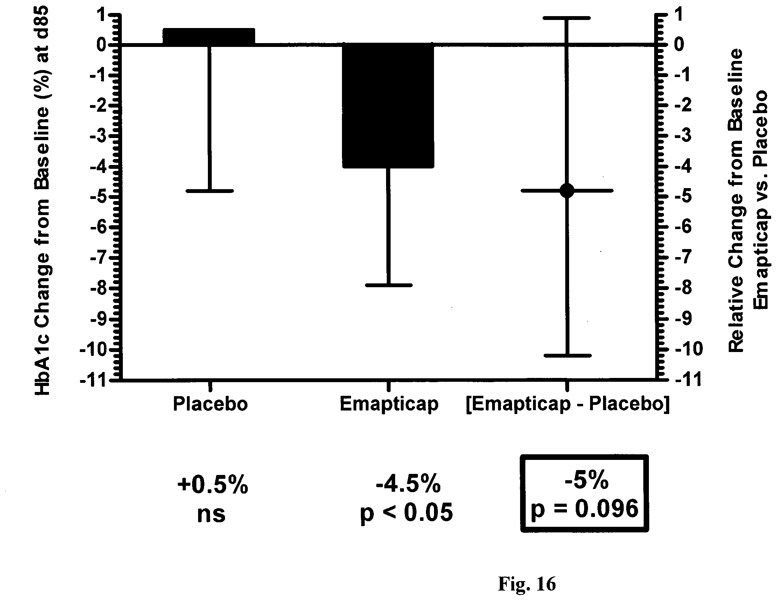

A relevant decrease of HbAlc was observed at day 85 (-0.32% and +0.06% absolute change from baseline for NOX-E36 and placebo; P=0.096), which was maintained after cessation of treatment and became statistically significant (P=0.036) four weeks after the last dose (Figs. 15 and 16).

"ns" as indicated in Figs. 14 and 16 means not significant.

From the results presented in both Examples 6 and 7 it is evident that prolonged treatment with NOX-E36 is safe and well tolerated and reduces urinary albumin excretion as well as HbAlc in type 2 diabetics with albuminuria. Said results also provide experimental evidence of a sustained effect on albuminuria even after cessation of treatment which indicates that important pathophysiological mechanisms of diabetic nephropathy are influenced. This distinguishes NOX-E36 from existing therapeutic strategies and indicates the disease-modifying potential of the drug. Furthermore and in contrast to approved drugs and other novel approaches in this indication, the effect of NOX-E36 on urinary albumin excretion is not associated with changes of blood pressure or eGFR.

### References

The complete bibliographic data of the documents recited herein is, if not indicated to the contrary, as follows.
ADA (2012) Standards of medical care in diabetes--2012. Diabetes Care 35 Suppl 1: S11-63
Altschul SF, Gish W, Miller W, Myers EW, Lipman DJ (1990), Basic local alignment search tool. J Mol Biol. 215(3):403-10.
Altschul SF, Madden TL, Schaffer AA, Zhang J, Zhang Z, Miller W, Lipman DJ (1997). Gapped BLAST and PSI-BLAST: a new generation of protein database search programs. Nucleic Acids Res. Sep 1;25(17):3389-402.
Brown Z, Gerritsen ME, Carley WW, Strieter RM, Kunkel SL, Westwick J (1994) Chemokine gene expression and secretion by cytokine-activated human microvascular endothelial cells. Differential regulation of monocyte chemoattractant protein-1 and interleukin-8 in response to interferon-gamma. Am J Pathol 145(4): 913-921
Damha MJ and Ogilvie KK, Methods in Molecular Biology, Vol. 20 Protocols for oligonucleotides and analogs, ed. S. Agrawal, p. 81-114, Humana Press Inc. (1993)
Dawson J, Miltz W, Mir AK, Wiessner C (2003) Targeting monocyte chemoattractant protein-1 signalling in disease. Expert Opin Ther Targets 7(1): 35-48
Giunti S, Barutta F, Perin PC, Gruden G (2010) Targeting the MCP-1/CCR2 System in diabetic kidney disease. Curr Vasc Pharmacol 8(6): 849-860
Kahn BB (1998) Type 2 diabetes: when insulin secretion fails to compensate for insulin resistance. Cell 92(5): 593-596
Klussmann S. (2006). "The Aptamer Handbook - Functional Oligonucleotides and their Applications." Edited by S. Klussmann. WILEY-VCH, Weinheim, Germany, ISBN 3-527-31059-2
Kusser W (2000). J Biotechnol 74:27-38
Levey AS, Stevens LA, Schmid CH, Zhang YL, Castro AF, 3rd, Feldman HI, Kusek JW, Eggers P, Van Lente F, Greene T, Coresh J (2009) A new equation to estimate glomerular filtration rate. Ann Intern Med 150(9): 604-612
Mayr FB, Spiel AO, Leitner JM, Firbas C, Schnee J, Hilbert J, Derendorf H, Jilma B (2009) Influence of the duffy antigen on pharmacokinetics and pharmacodynamics of recombinant monocyte chemoattractant protein (MCP-1, CCL-2) in vivo. Int J Immunopathol Pharmacol 22(3): 615-625
McGinnis S, Madden TL (2004). BLAST: at the core of a powerful and diverse set of sequence analysis tools.Nucleic Acids Res. 32(Web Server issue):W20-5.
Mora C, Navarro JF (2004) Inflammation and pathogenesis of diabetic nephropathy. Metabolism 53(2): 265-266; author reply 266-267
Mora C, Navarro JF (2005) The role of inflammation as a pathogenic factor in the development of renal disease in diabetes. Curr Diab Rep 5(6): 399-401
Needleman & Wunsch (1970), A general method applicable to the search for similarities in the amino acid sequence of two proteins. J Mol Biol. 48(3):443-53.
Ota T (2013) Chemokine systems link obesity to insulin resistance. Diabetes Metab J 37(3): 165-172
Pearson & Lipman (1988), Improved tools for biological sequence comparison. Proc. Nat'l. Acad. Sci. USA 85: 2444
Rollins BJ, Pober JS (1991) Interleukin-4 induces the synthesis and secretion of MCP-1/JE by human endothelial cells. Am J Pathol 138(6): 1315-1319
Saraheimo M, Teppo AM, Forsblom C, Fagerudd J, Groop PH (2003) Diabetic nephropathy is associated with low-grade inflammation in Type 1 diabetic patients. Diabetologia 46(10): 1402-1407
Shoelson SE, Lee J, Goldfine AB (2006) Inflammation and insulin resistance. J Clin Invest 116(7): 1793-1801
Smith & Waterman (1981), Adv. Appl. Math. 2: 482
Struyf S, Van Collie E, Paemen L, Put W, Lenaerts JP, Proost P, Opdenakker G, Van Damme J (1998) Synergistic induction of MCP-1 and -2 by IL-1beta and interferons in fibroblasts and epithelial cells. J Leukoc Biol 63(3): 364-372
Tsou CL, Peters W, Si Y, Slaymaker S, Aslanian AM, Weisberg SP, Mack M, Charo IF (2007) Critical roles for CCR2 and MCP-3 in monocyte mobilization from bone marrow and recruitment to inflammatory sites. J Clin Invest 117(4): 902-909
Tsuboi N, Yoshikai Y, Matsuo S, Kikuchi T, Iwami K, Nagai Y, Takeuchi O, Akira S, Matsuguchi T (2002) Roles of toll-like receptors in C-C chemokine production by renal tubular epithelial cells. J Immunol 169(4): 2026-2033
Venkatesan N, Kim SJ, Kim BH (2003) Novel phosphoramidite building blocks in synthesis and applications toward modified oligonucleotides. Curr Med Chem 10(19): 1973-1991
Wincott F, DiRenzo A, Shaffer C, Grimm S, Tracz D, Workman C, Sweedler D, Gonzalez C, Scaringe S, and Usman N (1995). Synthesis, deprotection, analysis and purification of RNA and ribosomes. Nucleic Acids Res. 23:2677-2684.

## Claims

1. An antagonist of CCL2 for use in a method for the treatment and/or prevention of proteinuria, wherein the method comprises administering the antagonist to a human subject, wherein the human subject is suffering from a disease, wherein proteinuria is expressed as ACR (urinary albumin/creatinine ratio), and wherein ACR of the subject is at least 30 mg/g, preferably at least 88 mg/g, and glomerular filtration rate of the subject is
a) at least 90 ml/min/1.73 m², or
b) 60 - 89 ml/min/1.73 m², or
c) 45 - 59 ml/min/1.73 m², or
d) 30 - 44 ml/min/1.73 m², or
e) 15 - 29 ml/min/1.73 m², or
f) < 15 ml/min/1.73 m²; and
wherein the antagonist is a nucleic acid molecule selected from the group comprising a type 2 MCP-1 binding nucleic acid molecule, a type 3 MCP-1 binding nucleic acid molecule, a type 4 MCP-1 binding nucleic acid molecule, a type 1A MCP-1 binding nucleic acid molecule, a type 1B MCP-1 binding nucleic acid molecule and a type 5 MCP-1 binding nucleic acid molecule,
whereby the type 2 MCP-1 binding nucleic acid molecule comprises in 5'->3' direction a first terminal stretch of nucleotides, a central stretch of nucleotides, and a second terminal stretch of nucleotides, whereby
the first terminal stretch of nucleotides comprises a nucleotide sequence selected from the group comprising ACGCA, CGCA and GCA,
the central stretch of nucleotides comprises a nucleotide sequence of CSUCCCUCACCGGUGCAAGUGAAGCCGYGGCUC, and
the second terminal stretch of nucleotides comprises a nucleotide sequence selected from the group comprising UGCGU, UGCG and UGC,
whereby the type 3 MCP-1 binding nucleic acid molecule comprises in 5'->3' direction a first terminal stretch of nucleotides, a first central stretch of nucleotides, a second central stretch of nucleotides, a third central stretch of nucleotides, a fourth central stretch of nucleotides, a fifth central stretch of nucleotides, a sixth central stretch of nucleotides, a seventh central stretch of nucleotides and a second terminal stretch of nucleotides, whereby
the first terminal stretch of nucleotides comprises a nucleotide sequence which is selected from the group comprising GURCUGC, GKSYGC, KBBSC and BNGC,
the first central stretch of nucleotides comprises a nucleotide sequence of GKMGU,
the second central stretch of nucleotides comprises a nucleotide sequence of KRRAR,
the third central stretch of nucleotides comprises a nucleotide sequence of ACKMC,
the fourth central stretch of nucleotides comprises a nucleotide sequence selected from the group comprising CURYGA, CUWAUGA, CWRMGACW and UGCCAGUG,
the fifth central stretch of nucleotides comprises a nucleotide sequence selected from the group comprising GGY and CWGC,
the sixth central stretch of nucleotides comprises a nucleotide sequence selected from the group comprising YAGA, CKAAU and CCUUUAU,
the seventh central stretch of nucleotides comprises a nucleotide sequence selected from the group comprising GCYR and GCWG, and
the second terminal stretch of nucleotides comprises a nucleotide sequence selected from the group comprising GCAGCAC, GCRSMC, GSVVM and GCNV,
whereby the type 4 MCP-1 binding nucleic acid molecule comprises in 5'->3' direction a first terminal stretch of nucleotides, a central stretch of nucleotides and a second terminal stretch of nucleotides, whereby
the first terminal stretch of nucleotides comprises a nucleotide sequence selected from the group comprising AGCGUGDU, GCGCGAG, CSKSUU, GUGUU, and UGUU;
the central stretch of nucleotides comprises a nucleotide sequence selected from the group comprising AGNDRDGBKGGURGYARGUAAAG, AGGUGGGUGGUAGUAAGUAAAG and CAGGUGGGUGGUAGAAUGUAAAGA, and
the second terminal stretch of nucleotides comprises a nucleotide sequence selected from the group comprising GNCASGCU, CUCGCGUC, GRSMSG, GRCAC, and GGCA,
whereby the type 1A MCP-1 binding nucleic acid molecule comprises in 5'->3' direction a first terminal stretch of nucleotides, a first central stretch of nucleotides, a second central stretch of nucleotides, a third central stretch of nucleotides, a fourth central stretch of nucleotides, a fifth central stretch of nucleotides and a second terminal stretch of nucleotides, whereby
the first terminal stretch of nucleotides comprises a nucleotide sequence of AGCRUG,
the first central stretch of nucleotides comprises a nucleotide sequence of CCCGGW,
the second central stretch of nucleotides comprises a nucleotide sequence of GUR,
the third central stretch of nucleotides comprises a nucleotide sequence of RYA,
the fourth central stretch of nucleotides comprises a nucleotide sequence of GGGGGRCGCGAYC,
the fifth central stretch of nucleotides comprises a nucleotide sequence of UGCAAUAAUG or URYAWUUG, and
the second terminal stretch of nucleotides comprises a nucleotide sequence of CRYGCU,
whereby the type 1B MCP-1 binding nucleic acid molecule comprises in 5'->3' direction a first terminal stretch of nucleotides, a first central stretch of nucleotides, a second central stretch of nucleotides, a third central stretch of nucleotides, a fourth central stretch of nucleotides, a fifth central stretch of nucleotides and a second terminal stretch of nucleotides, whereby
the first terminal stretch of nucleotides comprises a nucleotide sequence of AGYRUG,
the first central stretch of nucleotides comprises a nucleotide sequence of CCAGCU or CCAGY,
the second central stretch of nucleotides comprises a nucleotide sequence of GUG,
the third central stretch of nucleotides, comprises a nucleotide sequence of AUG,
the fourth central stretch of nucleotides comprises a nucleotide sequence of GGGGGGCGCGACC,
the fifth central stretch of nucleotides comprises a nucleotide sequence of CAUUUUA or CAUUUA, and
the second terminal stretch of nucleotides comprises a nucleotide sequence of CAYRCU,
whereby the type 5 MCP-1 binding nucleic acid molecule comprises a nucleotide sequence according to any one of SEQ.ID.NOs 87 to 115,
wherein preferably
a) the type 2 MCP-1 binding nucleic acid molecule comprises a nucleic acid sequence according to SEQ.ID.No 37, SEQ.ID.No 116, SEQ.ID.No 117 or SEQ.ID.No 228;
b) the type 3 MCP-1 binding nucleic acid molecule comprises a nucleic acid sequence selected from the group comprising the nucleic acid sequences according to SEQ ID NO. 56, SEQ.ID.No 57 to 61, SEQ.ID.No 67 to 71 and SEQ.ID.No 73;
c) the type 4 MCP-1 binding nucleic acid molecule comprises a nucleic acid sequence according to SEQ.ID.No 80 or SEQ.ID.No 81; and
d) the type 1 A MCP-1 binding nucleic acid molecule comprises a nucleic acid sequence according to SEQ.ID. No 21; and e) the type 1B MCP-1 binding nucleic acid molecule comprises a nucleic acid sequence according to SEQ.ID.No 28 or SEQ.ID.No 27.

2. The antagonist for use according to claim 1, wherein ACR of the subject is at least 100 mg/g and glomerular filtration rate of the subject is
a) at least 90 ml/min/1.73 m², or
b) 60 - 89 ml/min/1.73 m², or
c) 45 - 59 ml/min/1.73 m², or
d) 30 - 44 ml/min/1.73 m², or
e) 15 - 29 ml/min/1.73 m², or
f)< 15 ml/min/1.73 m².

3. The antagonist for use according to any one of claims 1 to 2, wherein
a) ACR of the subject is at least 300 mg/g and glomerular filtration rate of the subject is at least 90 ml/min/1.73 m², or
b) ACR of the subject is at least 300 mg/g, preferably at least 303 mg/g and more preferably at least 695 mg/g, and glomerular filtration rate of the subject is 60 - 89 ml/min/1.73 m², or
c) ACR of the subject is at least 300 mg/g, preferably at least 303 mg/g and more preferably at least 695 mg/g, and glomerular filtration rate of the subject is 45 - 59 ml/min/1.73 m², or
d) ACR of the subject is at least 300 mg/g, preferably at least 303 mg/g and more preferably at least 695 mg/g, and glomerular filtration rate of the subject is 30 - 44 ml/min/1.73 m², or
e) ACR of the subject is at least 300 mg/g, preferably at least 303 mg/g and more preferably at least 695 mg/g, and glomerular filtration rate of the subject is 15 - 29 ml/min/1.73 m², or
f) ACR of the subject is at least 300 mg/g, preferably at least 303 mg/g and more preferably at least 695 mg/g, and glomerular filtration rate of the subject is < 15 ml/min/1.73 m².

4. The antagonist for use according to any one of claims 1 to 3, wherein the disease is a renal disease, a cardiovascular disease, primary and secondary amyloidosis, focal-segmntal glomerulosclerosis, lupus nephritis, Fabry disease, glomerulonephritis, membranous glomerulopathy, hepatorenal syndrome, IgA nephropathy, cryoglobulinemia, multiple myeloma, Nagel-Patella syndrome, hereditary nephritis, polyarteriitis nodosa, purpura Schoenlein-Henoch, ANCA-associated vasculitides, nephrotic syndrome, rapid progressive glomerulonephritides or diabetes, wherein preferably diabetes is diabetes mellitus and more preferably diabetes is diabetes mellitus type 2.

5. The antagonist for use according to any one of claims 1 to 4, wherein the HbA1c value of the subject is above 7.95 %.

6. The antagonist for use according to any one of claims 1 to 5, wherein the subject has at least one of the following characteristics:
(i) the subject is diagnosed type 2 diabetes mellitus according to the American Diabetes Association (ADA) definition;
(ii) the subject is on stable treatment to control hypertension, hyperglycemia and/or dyslipidemia; and
(iii) the subject is on stable treatment with angiotensin-converting enzyme inhibitors (ACEi) and/or Angiotensin II receptor blockers (ARBs).

7. The antagonist for use according to any one of claims 1 to 6, wherein the subject has at least one of following characteristics:
(i) the subject is not suffering from type 1 diabetes mellitus;
(ii) the eGFR of the subject is not ≤ 25 ml/min/1.73 m²;
(iii) the subject did not have any cardiovascular event within 3 months prior to the onset of the administration of the antagonist;
(iv) the subject is not suffering from uncontrolled hypertension, preferably the upper limit of the blood pressure of the subject is 180/110 mm Hg;
(v) the subject was not subject to dialysis within 3 months prior to the onset of the administration of the antagonist;
(vi) the subject did not experience any acute kidney injury within 3 months prior to the onset of the administration of the antagonist;
(vii) the subject does not have or undergo any significant edema, leg ulcer and infectious disease;
(viii) the subject does not use a drug selected from the group consisting of a thiazolidinedione class drug and an immune suppressant;
(ix) the subject does not undergo steroid therapy except a steroid therapy for topical use or inhalation; and
(x) the subject does not chronically use non-steroidal anti-inflammatory drug (NSAIDs), cyclooxygenase type 2 (COX-2) inhibitors, two or more diuretic drugs and/or aliskiren.

8. The antagonist for use according to any one of claims 1 to 7, wherein the nucleuc acid molecule is a Spiegelmer.

9. The antagonist for use according to any one of claims 1 to 8, wherein the nucleic acid molecule comprises a modification, whereby more preferably the modification is a high molecular weight moiety and/or the modification allows to modify the characteristics of the nucleic acid molecule in terms of residence time in an animal or human body.

10. The antagonist for use according to any one of claims 1 to 9, wherein the level of proteinuria shown by the subject after termination of the administration of the antagonist to the subject is lower compared to the level of proteinuria shown by the subject prior to the administration of the antagonist, wherein preferably the level of proteinuria shown by the subject after termination of the administration of the antagonist to the subject is lower compared to the level of proteinuria shown by the subject prior to the administration of the antagonist for one month, for two months, for three months, for four months, for five months, for six months, for seven months, for eight months, for nine months, for ten months, for eleven months or for twelve months after termination of the administration of the antagonist to the subject.

11. An *in vitro* method for determining whether a human subject is susceptible for treatment with an antagonist of CCL2 as defined in any of the preceding claims, preferably any one of claims 1 to 9, wherein the method comprises determining whether the subject is suffering from proteinuria as defined in any of the preceding claims, preferably any one of claims 1 to 9, and wherein in case the subject is suffering from proteinuria as defined in any of the preceding claims, the subject is susceptible to treatment with an antagonist of CCL2 as defined in any of the preceding claims, preferably any one of claims 1 to 9.

12. An antagonist of CCL2 as defined in any one of claims 1 to 9 for use in a method for *in situ* improvement of glomerular filtration of kidney in a human subject, wherein the method comprises administering to the subject the antagonist of CCL2, wherein the subject is suffering from proteinuria as defined in any one of claims 1 to 9, wherein the improvement of glomerular filtration is achieved if the level of proteinuria shown by the subject after termination of the administration of the antagonist of CCL2 to the subject is lower compared to the level of proteinuria shown by the subject prior to the administration of the antagonist of CCL2 for one month, for two months, for three months, for four months, for five months, for six months, for seven months, for eight months, for nine months, for ten months, for eleven months or for twelve months after termination of the administration of the antagonist of CCL2 to the subject.

13. An antagonist of CCL2 as defined in any one of claims 1 to 9 for use in a method for *in situ* repair of kidney in a human subject, wherein the method comprises administering to the subject the antagonist of CCL2, wherein the subject is suffering from proteinuria as defined in any one of claims 1 to 9, wherein the kidney is deemed repaired if the level of proteinuria shown by the subject after termination of the administration of the antagonist of CCL2 to the subject is lower compared to the level of proteinuria shown by the subject prior to the administration of the antagonist of CCL2 for one month, for two months, for three months, for four months, for five months, for six months, for seven months, for eight months, for nine months, for ten months, for eleven months or for twelve months after termination of the administration of the antagonist of CCL2 to the subject.

## Patentansprüche

1. Antagonist von CCL2 zur Verwendung in einem Verfahren zur Behandlung und/oder Prävention von Proteinurie, wobei der Verfahren umfasst Verabreichen des Antagonisten an ein menschliches Lebewesen, wobei das menschliche Lebewesen unter einer Erkrankung leidet, wobei Proteinurie ausgedrückt ist als ACR (Verhältnis von Albumin im Urin/Kreatinin), und wobei das ACR des Lebewesens wenigstens 30 mg/g beträgt, bevorzugterweise wenigstens 88 mg/g, und die glomeruläre Filtrationsrate des Lebewesens
a) wenigstens 90 ml/min/1,73 m², oder
b) 60 - 89 ml/min/1,73 m², oder
c) 45 - 59 ml/min/1,73 m², oder
d) 30 - 44 ml/min/1,73 m², oder
e) 15 - 29 ml/min/1,73 m², oder
f) < 15 ml/min/1,73 m²
beträgt; und
wobei der Antagonist ein Nukleinsäuremolekül ist, das ausgewählt ist aus der Gruppe umfassend ein MCP-1-bindendes Nukleinsäuremolekül vom Typ 2, ein MCP-1-bindendes Nukleinsäuremolekül vom Typ 3, ein MCP-1-bindendes Nukleinsäuremolekül vom Typ 4, ein MCP-1-bindendes Nukleinsäuremolekül vom Typ 1A, ein MCP-1-bindendes Nukleinsäuremolekül vom Typ 1B und ein MCP-1-bindendes Nukleinsäuremolekül vom Typ 5,
wobei das MCP-1-bindende Nukleinsäuremolekül vom Typ 2 in 5'->3' - Richtung eine erste terminale Abfolge von Nukleotiden, eine mittige Abfolge von Nukleotiden und eine zweite terminal Abfolge von Nukleotiden umfasst, wobei
die erste terminale Abfolge von Nukleotiden eine Nukleotidsequenz umfasst, die ausgewählt ist aus der Gruppe umfassend
ACGCA, CGCA und GCA,
die mittige Abfolge von Nukleotiden eine Nukleotidsequenz aus CSUCCCUCACCGGUGCAAGUGAAGCCGYGGCUC umfasst, und
die zweite terminale Abfolge von Nukleotiden eine Nukleotidsequenz umfassst, die aus der Gruppe umfasssend UGCGU, UGCG und UGC,
wobei das MCP-1-bindende Nukleinsäuremolekül vom Typ 3 in 5'->3' - Richtung eine erste terminale Abfolge von Nukleotiden, eine erste mittige Abfolge von Nukleotiden, eine zweite mittige Abfolge von Nukleotiden, eine dritte mittige Abfolge von Nukleotiden, eine vierte mittige Abfolge von Nukleotiden, eine fünfte mittige Abfolge von Nukleotiden, eine sechste mittige Abfolge von Nukleotiden, eine siebte mittige Abfolge von Nukleotiden und eine zweite terminale Abfolge von Nukleotiden umfasst, wobei
die erste terminale Abfolge von Nukleotiden eine Nukleotidsequenz umfasst, die ausgewählt ist aus der Gruppe umfassend GURCUGC, GKSYGC, KBBSC und BNGC,
die erste mittige Abfolge von Nukleotiden eine Nukleotidsequenz aus GKMGU umfasst,
die zweite mittige Abfolge von Nukleotiden eine Nukleotidsequenz aus KRRAR umfasst,
die dritte mittige Abfolge von Nukleotiden eine Nukleotidsequenz aus ACKMC umfasst,
die vierte mittige Abfolge von Nukleotiden eine Nukleotidsequenz umfasst, die ausgewählt ist aus der Gruppe umfassend CURYGA, CUWAUGA, CWRMGACW und UGCCAGUG,
die fünfte mittige Abfolge von Nukleotiden eine Nukleotidsequenz umfasst, die ausgewählt ist aus der Gruppe umfassend GGY und CWGC,
die sechste mittige Abfolge von Nukleotiden eine Nukleotidsequenz umfasst, die ausgewählt ist aus der Gruppe umfassend YAGA, CKAAU und CCUUUAU,
die siebte mittige Abfolge von Nukleotiden eine Nukleotidsequenz umfasst, die ausgewählt ist aus der Gruppe umfassend GCYR und GCWG, und
die zweite terminale Abfolge von Nukleotiden eine Nukleotidsequenz umfasst, die ausgewählt ist aus der Gruppe umfassend GCAGCAC, GCRSMC, GSVVM und GCNV,
wobei das MCP-1-bindende Nukleinsäuremolekül vom Typ 4 in 5'->3' -Richtung eine erste terminale Abfolge von Nukleotiden, eine mittige Abfolge von Nukleotiden und eine zweite terminale Abfolge von Nukleotiden umfasst, wobei
die erste terminale Abfolge von Nukleotiden eine Nukleotidsequenz umfasst, die ausgewählt ist aus der Gruppe umfassend AGCGUGDU, GCGCGAG, CSKSUU, GUGUU und UGUU;
die mittige Abfolge von Nukleotiden eine Nukleotidsequenz umfasst, die ausgewählt ist aus der Gruppe umfassend AGNDRDGBKGGURGYARGUAAAG, AGGUGGGUGGUAGUAAGUAAAG und CAGGUGGGUGGUAGAAUGUAAAGA, und
die zweite terminale Abfolge von Nukleotiden eine Nukleotidsequenz umfasst, die ausgewählt ist aus der Gruppe umfassend GNCASGCU, CUCGCGUC, GRSMSG, GRCAC und GGCA,
wobei das MCP-1-bindende Nukleinsäuremolekül vom Typ 1A in 5'->3' -Richtung eine erste terminale Abfolge von Nukleotiden, eine erste mittige Abfolge von Nukleotiden, eine zweite mittige Abfolge von Nukleotiden, eine dritte mittige Abfolge von Nukleotiden, eine vierte mittige Abfolge von Nukleotiden, eine fünfte mittige Abfolge von Nukleotiden und eine zweite terminale Abfolge von Nukleotiden umfasst, wobei
die erste terminale Abfolge von Nukletoiden eine Nukleotidsequenz aus AGCRUG umfasst,
die erste mittige Abfolge von Nukleotiden eine Nukleotidsequenz aus CCCGGW umfasst,
die zweite mittige Abfolge von Nukleotiden eine Nukleotidsequenz aus GUR umfasst,
die dritte mittige Abfolge von Nukleotiden eine Nukleotidsequenz aus RYA umfasst,
die vierte mittige Abfolge von Nukleotiden eine Nukleotidsequenz aus GGGGGRCGCGAYC umfasst,
die fünfte mittige Abfolge von Nukleotiden eine Nukleotidsequenz aus UGCAAUAAUG oder URYAWUUG umfasst, und
die zweite terminale Abfolge von Nukleotiden eine Nukleotidsequenz aus CRYGCU umfasst,
wobei das MCP-1-bindende Nukleinsäuremolekül vom Typ 1B in 5'->3' -Richtung eine erste terminale Abfolge von Nukleotiden, eine erste mittige Abfolge von Nukleotiden, eine zweite mittige Abfolge von Nukleotiden, eine dritte mittige Abfolge von Nukleotiden, eine vierte mittige Abfolge von Nukleotiden, eine fünfte mittige Abfolge von Nukleotiden und eine zweite terminale Abfolge von Nukleotiden umfasst, wobei
die erste terminale Abfolge von Nukleotiden eine Nukleotidsequenz aus AGYRUG umfasst,
die erste mittige Abfolge von Nukleotiden eine Nukleotidsequenz aus CCAGCU oder CCAGY umfasst,
die zweite mittige Abfolge von Nukleotiden eine Nukleotidsequenz aus GUG umfasst,
die dritte mittige Abfolge von Nukleotiden eine Nukleotidsequenz aus AUG umfasst,
die vierte mittige Abfolge von Nukleotiden eine Nukleotidsequenz aus GGGGGGCGCGACC umfasst,
die fünfte mittige Abfolge von Nukleotiden eine Nukleotidsequenz aus CAUUUUA oder CAUUUA umfasst, und
die zweite terminale Abfolge von Nukleotiden eine Nukleotidsequenz aus CAYRCU umfasst,
wobei das MCP-1-bindende Nukleinsäuremolekül vom Typ 5 eine Nukleotidsequenz gemäß einer der Nukleotidsequenzen gemäß SEQ.ID.NOs 87 bis 115 umfasst,
wobei bevorzugterweise
a) das MCP-1-bindende Nukleinsäuremolekül vom Typ 2 eine Nukleinsäuresequenz gemäß SEQ.ID.No 37, SEQ.ID.No 116, SEQ.ID.No 117 oder SEQ.ID.No 228 umfasst;
b) das MCP-1-bindende Nukleinsäuremolekül vom Typ 3 eine Nukleinsäuresequenz umfasst, die ausgewählt ist aus der Gruppe umfassend die Nukleinsäuresequenzen gemäß SEQ ID NO. 56, SEQ.ID.No 57 bis 61, SEQ.ID.No 67 bis 71 und SEQ.ID.No 73;
c) das MCP-1-bindende Nukleinsäuremolekül vom Typ 4 eine Nukleinsäuresequenz gemäß SEQ.ID.No 80 oder SEQ.ID.No 81 umfasst; und
d) das MCP-1-bindende Nukleinsäuremolekül vom Typ 1 A eine Nukleinsäuresequenz gemäß SEQ.ID. No 21 umfassst; und
e) das MCP-1-bindende Nukleinsäuresäuremolekül vom Typ 1B eine Nukleinsäuresequenz gemäß SEQ.ID.No 28 oder SEQ.ID.No 27 umfasst.

2. Antagonist zur Verwendung nach Anspruch 1, wobei das ACR des Lebewensens wenigstens 100 mg/g beträgt und die glomeruläre Filtrationsrate
a) wenigstens 90 ml/min/1,73 m², oder
b) 60 - 89 ml/min/1,73 m², oder
c) 45 - 59 ml/min/1,73 m², oder
d) 30 - 44 ml/min/1,73 m², oder
e) 15 - 29 ml/min/1,73 m², oder
f) < 15 ml/min/1,73 m² beträgt.

3. Antagonist zur Verwendung nach einem der Ansprüche 1 bis 2, wobei
a) das ACR des Lebewesens wenigstens 300 mg/g beträgt, und die glomeruläre Filtrationsrate des Lebewesens wenigstens 90 ml/min/1,73 m² beträgt, oder
b) das ACR des Lebewesens wenigstens 300 mg/g, bevorzugterweise wenigstens 303 mg/g und bevorzugtererweise wenigstens 695 mg/g beträgt, und die glomeruläre Filtrationsrate des Lebewesens 60 - 89 ml/min/1,73 m² beträgt, oder
c) das ACR des Lebewesens wenigstens 300 mg/g, bevorzugterweise wenigstens 303 mg/g und bevorzugtererweise wenigstens 695 mg/g beträgt, und die glomeruläre Filtrationsrate des Lebewesens 45 - 59 ml/min/1,73 m² beträgt, oder
d) das ACR des Lebewesens wenigstens 300 mg/g, bevorzugterweise wenigstens 303 mg/g und bevorzugtererweise wenigstens 695 mg/g beträgt, und die glomeruläre Filtrationsrate des Lebewesens 30 - 44 ml/min/1,73 m² beträgt, oder
e) das ACR des Lebewesens wenigstens 300 mg/g, bevorzugterweise wenigstens 303 mg/g und bevorzugtererweise wenigstens 695 mg/g beträgt, und die glomeruläre Filtrationsrate des Lebewesens 15 - 29 ml/min/1,73 m² beträgt, oder
f) das ACR des Lebewesens wenigstens 300 mg/g, bevorzugtererweise wenigstens 303 mg/g und bevorzugtererweise wenigstens 695 mg/g beträgt, und die glomeruläre Filtrationsrate des Lebewesens < 15 ml/min/1,73 m² beträgt.

4. Antagonist zur Verwendung nach einem der Ansprüche 1 bis 3, wobei die Erkrankung eine Nierenerkrankung, eine cardiovaskuläre Erkrankung, primäre und sekundäre Amyloidose, fokal-segmentale Glomerulosklerose, Lupus nephritis, Fabry-Erkrankung, Glomerulonephritis, membranöse Glomerulopathie, hepatorenales Syndrom, IgA-Nephropathie, Kryoglobulinämie, multiples Myelom, Nagel-Patella-Syndrom, hereditäre Nephritis, Polyarteriitis nodosa, Purpura Schoenlein-Henoch, ANCA-assoziierte Vaskulitiden, nephrotisches Syndrom, schnell fortschreitende Glomerulonephritiden oder Diabetes ist, wobei bevorzugterweise Diabetes Diabetes mellitus und bevorzugtererweise Diabetes Diabetes mellitus vom Typ 2 ist.

5. Antagonist zur Verwendung nach einem der Ansprüche 1 bis 4, wobei der HbA1c-Wert des Lebewesens höher als 7.95 % ist.

6. Antagonist zur Verwendung nach einem der Ansprüche 1 bis 5, wobei das Lebewesen wenigstens eines der folgenden Merkmale aufweist:
(i) das Lebewesen ist gemäß der American Diabetes Association (ADA) -Definition als Diabetes mellitus vom Typ 2 diagnostiziert;
(ii) das Lebewesen wird stabil behandelt, um Bluthochdruck, Hyperglykämie und/oder Dyslipidämie zu kontrollieren; und
(iii) das Lebewesen wird stabil behandelt mit Angiotensin-Converting-Enzyme-Hemmern (ACEi) und/oder Angiotensin II-Rezeptor-Blockern (ARBs).

7. Antagonist zur Verwendung nach einem der Ansprüche 1 bis 6, wobei das Lebewesen wenigstens eines der folgenden Merkmale aufweist:
(i) das Lebewesen leidet nicht an Diabetes mellitus vom Typ 1;
(ii) der eGFR des Lebewesnes is nicht ≤ 25 ml/min/1,73 m²;
(iii) das Lebewesen hatte kein kardiovaskuläres Ereignis innerhalb von 3 Monaten vor dem Beginn der Verabreichung des Antagonisten;
(iv) das Lebewesen leidet nicht an unkontrolliertem Bluthochdruck, bevorzugterweise ist der obere Grenzwert des Bluthochdrucks des Lebewesens 180/110 mm Hg;
(v) das Lebewesen hatte innerhalb von 3 Monaten vor dem Beginn der Verabreichung des Antagonisten keine Dialyse;
(vi) das Lebewesen hatte innerhalb von 3 Monaten vor dem Beginn der Verabreichung des Antagonisten keine akute Nierenverletzung;
(vii) das Lebewesen hatte kein signifikantes Ödem, kein signifikantes Beingeschwür und keine signifikanten Infektionskrankheiten;
(viii) das Lebewesen verwendet keine Wirkstoffe, die ausgewählt sind aus der Gruppe bestehend aus einem Wirkstoff der Klasse der Thiazolidindione und einem immunsupprimierenden Mittel;
(ix) das Lebewesen erhält keine Steroidtherapie mit Ausnahme einer Steroidtherapie für die topische Anwendung oder Inhalation; und
(x) das Lebewesn verwendet nicht-steroidale anti-endzündliche Wirkstoffe (NSAIDs) vom Typ, Cyclooxygenase-Typ-2 (COX-2)-Inhibitoren, zwei oder mehr diuretische Mittel und/oder Aliskiren nicht chronisch.

8. Antagonist zur Verwendung nach einem der Ansprüche 1 bis 7, wobei das Nukleinsäuremolekül ein Spiegelmer ist.

9. Antagonist zur Verwendung nach einem der Ansprüche 1 bis 8, wobei das Nukleinsäuremolekül eine Modifikation umfasst, wobei bevorzugtererweise die Modifikation ein Teil mit einem hohen Molekulargewicht ist und/oder die Modifikation erlaubt, die Eigenschaften des Nukleinsäuremoleküls bezüglich Verweilzeit in einem tierischen oder einem menschlichen Körper zu modifizieren.

10. Antagonist zur Verwendung nach einem der Ansprüche 1 bis 9, wobei das von dem Lebewesen nach Beendig ung der Verabreichung des Antagonisten an das Lebewesen gezeigte Ausmass an Proteinurie geringer ist als das von dem Lebewesen vor der Verabreichung des Antagonisten gezeigte Ausmass an Proteinurie, wobei bevorzugterweise das von dem Lebewesen nach Beendigung der Verabreichung des Antagonisten an das Lebewesen gezeigte Ausmass an Proteinurie für einen Monat, für zwei Monate, für drei Monate, für vier Monate, für fünf Monate, für sechs Monate, für sieben Monate, für acht Monate, für neun Monate, für zehn Monate, für elf Monate oder für zwölf Monate nach Beendigung der Verabreichung des Antagonisten an das Lebewesen geringer ist verglichen mit dem von dem Lebewesen vor der Verabreichung des Antagonisten gezeigten Ausmass an Proteinurie.

11. *In vitro-*Verfahren zum Bestimmen, ob ein menschliches Lebewesen für eine Behandlung mit einem CCL2-Antagonisten, wie in einem der vorangehenden Ansprüche definiert, bevorzugterweise einem der Ansprüche 1 bis 9, empfänglich ist, wobei das Verfahren umfasst Bestimmen, ob das Lebewesen an Proteinurie, wie in einem der vorangehenden Ansprüche definiert, bevorzugerweise einem der Ansprüche 1 bis 9, leidet, und wobei für den Fall, dass das Lebewesen an Proteinurie, wie in einem der Ansprüche 1 bis 9 definiert, leidet, das Lebewesen für Behandlung mit einem CCL2-Antagonisten, wie in einem der vorangehenden Ansprüche definiert, bevorzugterweise einem der Ansprüche 1 bis 9, empfänglich ist.

12. CCL2-Antagonist, wie in einem der Ansprüche 1 bis 9 definiert, zur Verwendung in einem Verfahren zur *in* situ-Verbesserung von glomerulärer Filtration einer Niere in einem menschlichen Lebewesen, wobei das Verfahren umfasst Verabreichen des CCL2-Antagonisten an das Lebewesen, wobei das Lebewesen an Proteinurie, wie in einem der Ansprüche 1 bis 9 definiert, leidet, wobei die Verbesserung von glomerulärer Filtration erreicht ist, wenn das von dem Lebewesen gezeigte Ausmass an Proteinurie nach Beendigung der Verabreichung des CCL2-Antagonisten an das Lebewesen für einen Monat, für zwei Monate, für drei Monate, für vier Monate, für fünf Monate, für sechs Monate, für sieben Monate, für acht Monate, für neun Monate, für zehn Monate, für elf Monate oder für zwölf Monate nach Beendigung der Verabreichung des CCL2-Antagonisten an das Lebewesen geringer ist verglichen mit dem von dem Lebewesen vor der Verabreichung des CCL2-Antagonisten gezeigten Ausmass an Proteinurie.

13. CCL2-Antagonist, wie in einem der Ansprüche 1 bis 9 definiert, zur Verwendung in einem Verfahren für die *in situ*-Reparatur einer Niere in einem menschlichen Lebewesen, wobei das Verfahren umfasst Verabreichen des CCL2-Antagonisten an das Lebewesen, wobei das Lebewesen an Proteinurie, wie in einem der Ansprüche 1 bis 9 definiert, leidet, wobei die Niere als repariert erachtet wird, wenn das Ausmass an von dem Lebewesen nach Beendigung der Verabreichung des CCL2-Antagonisten an das Lebewesen gezeigter Proteinurie für einen Monat, für zwei Monate, für drei Monate, für vier Monate, für fünf Monate, für sechs Monate, für sieben Monate, für acht Monate, für neun Monate, für zehn Monate, für elf Monate oder für zwölf Monate nach Beendigung der Verabreichung des CCL2-Antagonisten an das Lebewesen geringer ist verglichen mit dem von dem Lebewesen vor der Verabreichung des CCL2-Antagonisten gezeigten Ausmass an Proteinurie.

## Revendications

1. Antagoniste de CCL2 pour une utilisation dans un procédé pour le traitement et/ou la prévention de la protéinurie, dans lequel le procédé comprend l'administration de l'antagoniste à un sujet humain, dans lequel le sujet humain souffre d'une maladie, dans lequel la protéinurie est exprimée en ACR (rapport albumine urinaire/créatinine) et dans lequel l'ACR du sujet est d'au moins 30 mg/g, de préférence d'au moins 88 mg/g, et le taux de filtration glomérulaire du sujet est
a) d'au moins 90 ml/min/1,73 m², ou
b) de 60 à 89 ml/min/1,73 m², ou
c) de 45 à 59 ml/min/1,73 m², ou
d) de 30 à 44 ml/min/1,73 m², ou
e) de 1.5 à 29 ml/min/1,73 m², ou
f) < 15 ml/min/1,73 m² ; et
dans lequel l'antagoniste est une molécule d'acide nucléique choisie dans le groupe comprenant une molécule d'acide nucléique de liaison MCP-1 de type 2, une molécule d'acide nucléique de liaison MCP-1 de type 3, une molécule d'acide nucléique de liaison MCP-1 de type 4, une molécule d'acide nucléique de liaison MCP-1 de type 1A, une molécule d'acide nucléique de liaison MCP-1 de type 1B et une molécule d'acide nucléique de liaison MCP-1 de type 5,
moyennant quoi la molécule d'acide nucléique de liaison MCP-1 de type 2 comprend, dans la direction 5'->3', un premier tronçon terminal de nucléotides, un tronçon central de nucléotides et un second tronçon terminal de nucléotides, moyennant quoi
le premier tronçon terminal de nucléotides comprend une séquence nucléotidique choisie dans le groupe comprenant ACGCA, CGCA et GCA,
le tronçon central de nucléotides comprend une séquence nucléotidique de CSUCCCUCACCGGUGCAAGUGAAGCCGYGGCUC, et
le second tronçon terminal de nucléotides comprend une séquence nucléotidique choisie dans le groupe comprenant UGCGU, UGCG et UGC,
moyennant quoi la molécule d'acide nucléique de liaison MCP-1 de type 3 comprend, dans la direction 5'->3', un premier tronçon terminal de nucléotides, un premier tronçon central de nucléotides, un deuxième tronçon central de nucléotides, un troisième tronçon central de nucléotides, un quatrième tronçon central de nucléotides, un cinquième tronçon central de nucléotides, un sixième tronçon central de nucléotides, un septième tronçon central de nucléotides et un second tronçon terminal de nucléotides, moyennant quoi
le premier tronçon terminal de nucléotides comprend une séquence nucléotidique qui est choisie dans le groupe comprenant GURCUGC, GKSYGC, KBBSC et BNGC,
le premier tronçon central de nucléotides comprend une séquence nucléotidique de GKMGU,
le deuxième tronçon central de nucléotides comprend une séquence nucléotidique de KRRAR,
le troisième tronçon central de nucléotides comprend une séquence nucléotidique d'ACKMC,
le quatrième tronçon central de nucléotides comprend une séquence nucléotidique choisie dans le groupe comprenant CURYGA, CUWAUGA, CWRMGACW et UGCCAGUG,
le cinquième tronçon central de nucléotides comprend une séquence nucléotidique choisie dans le groupe comprenant GGY et CWGC,
le sixième tronçon central de nucléotides comprend une séquence nucléotidique choisie dans le groupe comprenant YAGA, CKAAU et CCUUUAU,
le septième tronçon central de nucléotides comprend une séquence nucléotidique choisie dans le groupe comprenant GCYR et GCWG, et
le second tronçon terminal de nucléotides comprend une séquence nucléotidique choisie dans le groupe comprenant GCAGCAC, GCRSMC, GSVVM et GCNV,
moyennant quoi la molécule d'acide nucléique de liaison MCP-1 de type 4 comprend, dans la direction 5'->3', un premier tronçon terminal de nucléotides, un tronçon central de nucléotides et un second tronçon terminal de nucléotides, moyennant quoi
le premier tronçon terminal de nucléotides comprend une séquence nucléotidique choisie dans le groupe comprenant AGCGUGDU, GCGCGAG, CSKSUU, GUGUU, et UGUU ;
le tronçon central de nucléotides comprend une séquence nucléotidique choisie dans le groupe comprenant AGNDRDGBKGGURGYARGUAAAG, AGGUGGGUGGUAGAAGUAAAG et CAGGUGGGUGGUAGAAUGUAAAGA, et
le second tronçon terminal de nucléotides comprend une séquence nucléotidique choisie dans le groupe comprenant GNCASGCU, CUCGCGUC, GRSMSG, GRCAC, et GGCA,
moyennant quoi la molécule d'acide nucléique de liaison MCP-1 de type 1A comprend, dans la direction 5'->3', un premier tronçon terminal de nucléotides, un premier tronçon central de nucléotides, un deuxième tronçon central de nucléotides, un troisième tronçon central de nucléotides, un quatrième tronçon central de nucléotides, un cinquième tronçon central de nucléotides et un second tronçon terminal de nucléotides, moyennant quoi
le premier tronçon terminal de nucléotides comprend une séquence nucléotidique d'AGCRUG,
le premier tronçon central de nucléotides comprend une séquence nucléotidique de CCCGGW,
le deuxième tronçon central de nucléotides comprend une séquence nucléotidique de GUR,
le troisième tronçon central de nucléotides comprend une séquence nucléotidique de RYA,
le quatrième tronçon central de nucléotides comprend une séquence nucléotidique de GGGGGRCGCGAYC,
le cinquième tronçon central de nucléotides comprend une séquence nucléotidique d'UGCAAUAAUG ou d'URYAWUUG, et
le second tronçon terminal de nucléotides comprend une séquence nucléotidique de CRYGCU,
moyennant quoi la molécule d'acide nucléique de liaison MCP-1 de type 1B comprend, dans la direction 5'->3', un premier tronçon terminal de nucléotides, un premier tronçon central de nucléotides, un deuxième tronçon central de nucléotides, un troisième tronçon central de nucléotides, un quatrième tronçon central de nucléotides, un cinquième tronçon central de nucléotides et un second tronçon terminal de nucléotides, moyennant quoi
le premier tronçon terminal de nucléotides comprend une séquence nucléotidique d'AGYRUG,
le premier tronçon central de nucléotides comprend une séquence nucléotidique de CCAGCU ou CCAGY,
le deuxième tronçon central de nucléotides comprend une séquence nucléotidique de GUG,
le troisième tronçon central de nucléotides comprend une séquence nucléotidique d'AUG,
le quatrième tronçon central de nucléotides comprend une séquence nucléotidique de GGGGGGCGCGACC,
le cinquième tronçon central de nucléotides comprend une séquence nucléotidique de CAUUUUA ou de CAUUUA, et
le second tronçon terminal de nucléotides comprend une séquence nucléotidique de CAYRCU,
moyennant quoi la molécule d'acide nucléique de liaison MCP-1 de type 5 comprend une séquence nucléotidique selon l'une quelconque des SEQ.ID N° 87 à 115,
dans lequel, de préférence
a) la molécule d'acide nucléique de liaison MCP-1 de type 2 comprend une séquence d'acide nucléique selon SEQ.ID N° 37, SEQ.ID N° 116, SEQ.ID N° 117 ou SEQ.ID N° 228 ;
b) la molécule d'acide nucléique de liaison MCP-1 de type 3 comprend une séquence d'acide nucléique choisie dans le groupe comprenant les séquences d'acide nucléique selon SEQ ID N° 56, SEQ.ID.N° 57 à 61, SEQ.ID.N° 67 à 71 et SEQ.ID.N° 73 ;
c) la molécule d'acide nucléique de liaison MCP-1 de type 4 comprend une séquence d'acide nucléique selon SEQ.ID.N° 80 ou SEQ.ID.N° 81 ; et
d) d) la molécule d'acide nucléique de liaison MCP-1 de type 1A comprend une séquence d'acide nucléique selon SEQ.ID.N° 21 ; et
e) la molécule d'acide nucléique de liaison MCP-1 de type 1B comprend une séquence d'acide nucléique selon SEQ.ID.N° 28 ou SEQ.ID.N° 27.

2. Antagoniste pour une utilisation selon la revendication 1, dans lequel l'ACR du sujet est d'au moins 100 mg/g et le taux de filtration glomérulaire du sujet estd'au moins 90 ml/min/1,73 m², ou60 à 89 ml/min/1,73 m², ou45 à 59 ml/min/1,73 m², ou
d) 30 à 44 ml/min/1,73 m², ou
e) 15 à 29 ml/min/1,73 m², ou
f) < 15 ml/min/1,73 m².

3. Antagoniste pour une utilisation selon l'une quelconque des revendications 1 et 2, dans lequel
a) l'ACR du sujet est d'au moins 300 mg/g et le taux de filtration glomérulaire du sujet est d'au moins 90 ml/min/1,73 m², ou
b) l'ACR du sujet est d'au moins 300 mg/g, de préférence d'au moins 303 mg/g et de manière davantage préférée d'au moins 695 mg/g, et le taux de filtration glomérulaire du sujet est de 60 à 89 ml/min/1,73 m², ou
c) l'ACR du sujet est d'au moins 300 mg/g, de préférence d'au moins 303 mg/g et de manière davantage préférée d'au moins 695 mg/g, et le taux de filtration glomérulaire du sujet est de 45 à 59 ml/min/1,73 m², ou
d) l'ACR du sujet est d'au moins 300 mg/g, de préférence d'au moins 303 mg/g et de manière davantage préférée d'au moins 695 mg/g, et le taux de filtration glomérulaire du sujet est de 30 à 44 ml/min/1,73 m², ou
e) l'ACR du sujet est d'au moins 300 mg/g, de préférence d'au moins 303 mg/g et de manière davantage préférée d'au moins 695 mg/g, et le taux de filtration glomérulaire du sujet est de 15 à 29 ml/min/1,73 m², ou
f) l'ACR du sujet est d'au moins 300 mg/g, de préférence d'au moins 303 mg/g et de manière davantage préférée d'au moins 695 mg/g, et le taux de filtration glomérulaire du sujet est < 15 ml/min/1,73 m².

4. Antagoniste pour une utilisation selon l'une quelconque des revendications 1 à 3, dans lequel la maladie est une maladie rénale, une maladie cardiovasculaire, une amyloïdose primaire et secondaire, une glomérulosclérose focale-segmnale, une néphrite lupique, une maladie de Fabry, une glomérulonéphrite, une glomérulopathie membraneuse, un syndrome hépatorénal, une néphropathie à IgA, une cryoglobulinémie, un myélome multiple, un syndrome de Nagel-Patella, une néphrite héréditaire, une polyartérite noueuse, un purpura de Schönlein-Henoch, des vascularites associées aux ANCA, un syndrome néphrotique, des glomérulonéphritides progressifs rapides ou des diabètes, dans lequel le diabète est de préférence le diabète sucré et de manière davantage préférée le diabète sucré de type 2.

5. Antagoniste pour une utilisation selon l'une quelconque des revendications 1 à 4, dans lequel la valeur HbA1c du sujet est supérieure à 7,95 %.

6. Antagoniste pour une utilisation selon l'une quelconque des revendications 1 à 5, dans lequel le sujet présente au moins l'une des caractéristiques suivantes :
(i) le sujet reçoit un diagnostic de diabète sucré de type 2 selon la définition de l'Association américaine contre le diabète (American Diabetes Association - ADA) ;
(ii) le sujet est sous traitement stable pour contrôler l'hypertension, l'hyperglycémie et/ou la dyslipidémie ; et
(iii) le sujet est sous traitement stable avec des inhibiteurs d'enzyme de conversion d'angiotensine (ACEi) et/ou des antagonistes de récepteurs de l'angiotensine II (ARB).

7. Antagoniste pour une utilisation selon l'une quelconque des revendications 1 à 6, dans lequel le sujet présente au moins l'une des caractéristiques suivantes :
(i) le sujet ne souffre pas de diabète sucré de type 1 ;
(ii) l'eGFR du sujet n'est pas ≤ 25 ml/min/1,73 m² ;
(iii) le sujet n'a eu aucun événement cardiovasculaire dans les 3 mois précédant le début de l'administration de l'antagoniste ;
(iv) le sujet ne souffre pas d'hypertension non contrôlée, de préférence la limite supérieure de la pression sanguine du sujet est de 180/110 mm Hg ;
(v) le sujet n'a pas été soumis à une dialyse dans les 3 mois précédant le début de l'administration de l'antagoniste ;
(vi) le sujet n'a présenté aucune lésion rénale aiguë dans les 3 mois précédant le début de l'administration de l'antagoniste ;
(vii) le sujet ne présente ou ne subit aucun œdème significatif, aucun ulcère de jambe et aucune maladie infectieuse ;
(viii) le sujet n'utilise pas de médicament choisi dans le groupe constitué d'un médicament de la classe des thiazolidinediones et d'un immunosuppresseur ;
(ix) le sujet ne subit pas de thérapie stéroïdienne sauf une thérapie stéroïdienne à usage topique ou par inhalation ; et
(x) le sujet n'utilise pas de manière chronique un anti-inflammatoire non stéroïdien (AINS), des inhibiteurs de cyclooxygénase de type 2 (COX-2), deux médicaments diurétiques ou plus et/ou l'aliskirène.

8. Antagoniste pour une utilisation selon l'une quelconque des revendications 1 à 7, dans lequel la molécule d'acide nucléique est un Spiegelmer.

9. Antagoniste pour une utilisation selon l'une quelconque des revendications 1 à 8, dans lequel la molécule d'acide nucléique comprend une modification, moyennant quoi la modification est de manière davantage préférée une fraction de poids moléculaire élevé et/ou la modification permet de modifier les caractéristiques de la molécule d'acide nucléique en matière de durée de séjour dans un corps animal ou humain.

10. Antagoniste pour une utilisation selon l'une quelconque des revendications 1 à 9, dans lequel le niveau de protéinurie présenté par le sujet après la fin de l'administration de l'antagoniste au sujet est inférieur par rapport au niveau de protéinurie présenté par le sujet avant l'administration de l'antagoniste, dans lequel le niveau de protéinurie présenté par le sujet après la fin de l'administration de l'antagoniste au sujet est de préférence inférieur par rapport au niveau de protéinurie présenté par le sujet avant l'administration de l'antagoniste pendant un mois, pendant deux mois, pendant trois mois, pendant quatre mois, pendant cinq mois, pendant six mois, pendant sept mois, pendant huit mois, pendant neuf mois, pendant dix mois, pendant onze mois ou pendant douze mois après la fin de l'administration de l'antagoniste au sujet.

11. Procédé *in vitro* pour déterminer si un sujet humain est susceptible d'être traité avec un antagoniste de CCL2 tel que défini dans l'une quelconque des revendications précédentes, de préférence l'une quelconque des revendications 1 à 9, dans lequel le procédé comprend le fait de déterminer si le sujet souffre de protéinurie comme défini dans l'une quelconque des revendications précédentes, de préférence l'une quelconque des revendications 1 à 9, et dans lequel, dans le cas où le sujet souffre de protéinurie telle que définie dans l'une quelconque des revendications précédentes, le sujet est susceptible d'être traité avec un antagoniste de CCL2 tel que défini dans l'une quelconque des revendications précédentes, de préférence l'une quelconque des revendications 1 à 9.

12. Antagoniste de CCL2 tel que défini dans l'une quelconque des revendications 1 à 9 pour une utilisation dans un procédé pour l'amélioration *in situ* de la filtration glomérulaire d'un rein chez un sujet humain, dans lequel le procédé comprend l'administration au sujet de l'antagoniste de CCL2, dans lequel le sujet souffre d'une protéinurie telle que définie dans l'une quelconque des revendications 1 à 9, dans lequel l'amélioration de la filtration glomérulaire est obtenue si le niveau de protéinurie présenté par le sujet après la fin de l'administration de l'antagoniste de CCL2 au sujet est inférieur par rapport au niveau de protéinurie présenté par le sujet avant l'administration de l'antagoniste de CCL2 pendant un mois, pendant deux mois, pendant trois mois, pendant quatre mois, pendant cinq mois, pendant six mois, pendant sept mois, pendant huit mois, pendant neuf mois, pendant dix mois, pendant onze mois ou pendant douze mois après la fin de l'administration de l'antagoniste de CCL2 au sujet.

13. Antagoniste de CCL2 tel que défini dans l'une quelconque des revendications 1 à 9 pour une utilisation dans un procédé pour la réparation *in situ* d'un rein chez un sujet humain, dans lequel le procédé comprend l'administration au sujet de l'antagoniste de CCL2, dans lequel le sujet souffre d'une protéinurie telle que définie dans l'une quelconque des revendications 1 à 9, dans lequel le rein est considéré comme réparé si le niveau de protéinurie présenté par le sujet après la fin de l'administration de l'antagoniste de CCL2 au sujet est inférieur par rapport au niveau de protéinurie présenté par le sujet avant l'administration de l'antagoniste de CCL2 pendant un mois, pendant deux mois, pendant trois mois, pendant quatre mois, pendant cinq mois, pendant six mois, pendant sept mois, pendant huit mois, pendant neuf mois, pendant dix mois, pendant onze mois ou pendant douze mois après la fin de l'administration de l'antagoniste de CCL2 au sujet.


### SEQUENCE LISTING

<110> NOXXON Pharma AG
<120> Means and methods for hte treatment of nephropathy
<130> N 10108 PCT
<150> EP 13005191.5
   <151> 2013-11-04
<150> EP 14001242.8
   <151> 2014-04-03
<160> 228
<170> PatentIn version 3.3
<210> 1
   <211> 76
   <212> PRT
   <213> Homo sapiens
<220>
   <221> MISC_FEATURE
   <223> MCP-1
<400> 1
   Gln Pro Asp Ala Ile Asn Ala Pro Val Thr Cys Cys Tyr Asn Phe Thr
   1               5                   10                  15
   Asn Arg Lys Ile Ser Val Gln Arg Leu Ala Ser Tyr Arg Arg Ile Thr
               20                  25                  30
   Ser Ser Lys Cys Pro Lys Glu Ala Val Ile Phe Lys Thr Ile Val Ala
           35                  40                  45
   Lys Glu Ile Cys Ala Asp Pro Lys Gln Lys Trp Val Gln Asp Ser Met
       50                  55                  60
   Asp His Leu Asp Lys Gln Thr Gln Thr Pro Lys Thr
   65                  70                  75
<210> 2
   <211> 33
   <212> RNA
   <213> Artificial
<220>
   <223> Synthetic
<220>
   <221> misc_feature
   <223> L-RNA
<400> 2
   csucccucac cggugcaagu gaagccgygg cuc 33
<210> 3
   <211> 23
   <212> RNA
   <213> Artificial
<220>
   <223> Synthetic
<220>
   <221> misc_feature
   <223> L-RNA
<220>
   <221> misc_feature
   <222> (3)..(3)
   <223> n is a, c, g, or u
<400> 3
   agndrdgbkg gurgyargua aag 23
<210> 4
   <211> 22
   <212> RNA
   <213> Artificial
<220>
   <223> Synthetic
<220>
   <221> misc_feature
   <223> L-RNA
<400> 4
   aggugggugg uaguaaguaa ag 22
<210> 5
   <211> 24
   <212> RNA
   <213> Artificial
<220>
   <223> Synthetic
<220>
   <221> misc_feature
   <223> L-RNA
<400> 5
   caggugggug guagaaugua aaga 24
<210> 6
   <211> 13
   <212> RNA
   <213> Artificial
<220>
   <223> Synthetic
<220>
   <221> misc_feature
   <223> L-RNA
<400> 6
   gggggrcgcg ayc 13
<210> 7
   <211> 10
   <212> RNA
   <213> Artificial
<220>
   <223> Synthetic
<220>
   <221> misc_feature
   <223> L-RNA
<400> 7
   ugcaauaaug 10
<210> 8
   <211> 74
   <212> PRT
   <213> Homo sapiens
<220>
   <221> MISC_FEATURE
   <223> Eotaxin
<400> 8
   Gly Pro Ala Ser Val Pro Thr Thr Cys Cys Phe Asn Leu Ala Asn Arg
   1               5                   10                  15
   Lys Ile Pro Leu Gln Arg Leu Glu Ser Tyr Arg Arg Ile Thr Ser Gly
               20                  25                  30
   Lys Cys Pro Gln Lys Ala Val Ile Phe Lys Thr Lys Leu Ala Lys Asp
           35                  40                  45
   Ile Cys Ala Asp Pro Lys Lys Lys Trp Val Gln Asp Ser Met Lys Tyr
       50                  55                  60
   Leu Asp Gln Lys Ser Pro Thr Pro Lys Pro
   65                  70
<210> 9
   <211> 76
   <212> PRT
   <213> Homo sapiens
<220>
   <221> MISC_FEATURE
   <223> MCP-2
<400> 9
   Gln Pro Asp Ser Val Ser Ile Pro Ile Thr Cys Cys Phe Asn Val Ile
   1               5                   10                  15
   Asn Arg Lys Ile Pro Ile Gln Arg Leu Glu Ser Tyr Thr Arg Ile Thr
               20                  25                  30
   Asn Ile Gln Cys Pro Lys Glu Ala Val Ile Phe Lys Thr Lys Arg Gly
           35                  40                  45
   Lys Glu Val Cys Ala Asp Pro Lys Glu Arg Trp Val Arg Asp Ser Met
       50                  55                  60
   Lys His Leu Asp Gln Ile Phe Gln Asn Leu Lys Pro
   65                  70                  75
<210> 10
   <211> 47
   <212> RNA
   <213> Artificial
<220>
   <223> Synthetic
<220>
   <221> misc_feature
   <223> L-RNA
<400> 10
   agcgugcccg gaguggcagg gggacgcgac cugcaauaau gcacgcu 47
<210> 11
   <211> 45
   <212> RNA
   <213> Artificial
<220>
   <223> Syntetic
<220>
   <221> misc_feature
   <223> L-RNA
<400> 11
   agcgugcccg gaguggcagg gggacgcgac cugcaauugc acgcu 45
<210> 12
   <211> 47
   <212> RNA
   <213> Artificial
<220>
   <223> Synthetic
<220>
   <221> misc_feature
   <223> L-RNA
<400> 12
   agcgugcccg gaguggcagg gggacgcgac cuguaauaau gcacgcu 47
<210> 13
   <211> 47
   <212> RNA
   <213> Artificial
<220>
   <223> Synthetic
<220>
   <221> misc_feature
   <223> L-RNA
<400> 13
   agcgugcccg guguggcagg gggacgcgac cugcaauaau gcgcgcu 47
<210> 14
   <211> 47
   <212> RNA
   <213> Artificial
<220>
   <223> Synthetic
<220>
   <221> misc_feature
   <223> L-RNA
<400> 14
   agcgugcccg gaguagcagg ggggcgcgac cugcaauaau gcacgcu 47
<210> 15
   <211> 45
   <212> RNA
   <213> Artificial
<220>
   <223> Synthetic
<220>
   <221> misc_feature
   <223> L-RNA
<400> 15
   agcgugcccg gugugguagg ggggcgcgau cuacaauugc acgcu 45
<210> 16
   <211> 45
   <212> RNA
   <213> Artificial
<220>
   <223> Synthetic
<220>
   <221> misc_feature
   <223> L-RNA
<400> 16
   agcgugcccg gugugacagg ggggcgcgac cugcauuugc acgcu 45
<210> 17
   <211> 45
   <212> RNA
   <213> Artificial
<220>
   <223> Synthetic
<220>
   <221> misc_feature
   <223> L-RNA
<400> 17
   agcgugcccg guguggcagg ggggcgcgac cuguauuugc acgcu 45
<210> 18
   <211> 47
   <212> RNA
   <213> Artificial
<220>
   <223> Synthetic
<220>
   <221> misc_feature
   <223> L-RNA
<400> 18
   agcgugcccg gaguggcagg ggggcgcgac cugcaauaau gcacgcu 47
<210> 19
   <211> 45
   <212> RNA
   <213> Artificial
<220>
   <223> Synthetic
<220>
   <221> misc_feature
   <223> L-RNA
<400> 19
   agcgugcccg guguggcagg ggggcgcgac cugcaauugc acgcu 45
<210> 20
   <211> 45
   <212> RNA
   <213> Artificial
<220>
   <223> Synthetic
<220>
   <221> misc_feature
   <223> L-RNA
<400> 20
   agcaugcccg guguggcagg ggggcgcgac cugcauuugc augcu 45
<210> 21
   <211> 45
   <212> RNA
   <213> Artificial
<220>
   <223> Synthetic
<220>
   <221> misc_feature
   <223> L-RNA
<400> 21
   agcgugcccg gugugguagg ggggcgcgac cuacauuugc acgcu 45
<210> 22
   <211> 44
   <212> RNA
   <213> Artificial
<220>
   <223> Synthetic
<220>
   <221> misc_feature
   <223> L-RNA
<400> 22
   agugugccag cugugauggg ggggcgcgac ccauuuuaca cacu 44
<210> 23
   <211> 43
   <212> RNA
   <213> Artificial
<220>
   <223> Synthetic
<220>
   <221> misc_feature
   <223> L-RNA
<400> 23
   agugugccag cgugaugggg gggcgcgacc cauuuuacac acu 43
<210> 24
   <211> 43
   <212> RNA
   <213> Artificial
<220>
   <223> Synthetic
<220>
   <221> misc_feature
   <223> L-RNA
<400> 24
   agugugcgag cgugaugggg gggcgcgacc cauuuuacau acu 43
<210> 25
   <211> 43
   <212> RNA
   <213> Artificial
<220>
   <223> Synthetic
<220>
   <221> misc_feature
   <223> L-RNA
<400> 25
   agugugccag cgugaugggg gggcgcgacc cauuuuacau acu 43
<210> 26
   <211> 42
   <212> RNA
   <213> Artificial
<220>
   <223> Synthetic
<220>
   <221> misc_feature
   <223> L-RNA
<400> 26
   aguaugccag cgugaugggg gggcgcgacc cauuuacaua cu 42
<210> 27
   <211> 43
   <212> RNA
   <213> Artificial
<220>
   <223> Synthetic
<220>
   <221> misc_feature
   <223> L-RNA
<400> 27
   agugugccag ugugaugggg gggcgcgacc cauuuuacac acu 43
<210> 28
   <211> 43
   <212> RNA
   <213> Artificial
<220>
   <223> Synthetic
<220>
   <221> misc_feature
   <223> L-RNA
<400> 28
   agcgugccag ugugaugggg gggcgcgacc cauuuuacac gcu 43
<210> 29
   <211> 43
   <212> RNA
   <213> Artificial
<220>
   <223> Synthetic
<220>
   <221> misc_feature
   <223> L-RNA
<400> 29
   acgcacgucc cucaccggug caagugaagc cgcggcucug cgu 43
<210> 30
   <211> 43
   <212> RNA
   <213> Artificial
<220>
   <223> Synthetic
<220>
   <221> misc_feature
   <223> L-RNA
<400> 30
   acgcaccucc cucaccggug caagugaagc cguggcucug cgc 43
<210> 31
   <211> 43
   <212> RNA
   <213> Artificial
<220>
   <223> Synthetic
<220>
   <221> misc_feature
   <223> L-RNA
<400> 31
   acgcacgucc cucaccggug caagugaagc cguggcucug cgu 43
<210> 32
   <211> 41
   <212> RNA
   <213> Artificial
<220>
   <223> Synthetic
<220>
   <221> misc_feature
   <223> L-RNA
<400> 32
   gcacgucccu caccggugca agugaagccg uggcucugcg u 41
<210> 33
   <211> 41
   <212> RNA
   <213> Artificial
<220>
   <223> Synthetic
<220>
   <221> misc_feature
   <223> L-RNA
<400> 33
   acgcacgucc cucaccggug caagugaagc cguggcucug c 41
<210> 34
   <211> 39
   <212> RNA
   <213> Artificial
<220>
   <223> Synthetic
<220>
   <221> misc_feature
   <223> L-RNA
<400> 34
   gcacgucccu caccggugca agugaagccg uggcucugc 39
<210> 35
   <211> 42
   <212> RNA
   <213> Artificial
<220>
   <223> Synthetic
<220>
   <221> misc_feature
   <223> L-RNA
<400> 35
   cgcacguccc ucaccggugc aagugaagcc guggcucugc gu 42
<210> 36
   <211> 41
   <212> RNA
   <213> Artificial
<220>
   <223> Synthetic
<220>
   <221> misc_feature
   <223> L-RNA
<400> 36
   cgcacguccc ucaccggugc aagugaagcc guggcucugc g 41
<210> 37
   <211> 40
   <212> RNA
   <213> Artificial
<220>
   <223> Synthetic
<220>
   <221> misc_feature
   <223> L-RNA
<400> 37
   gcacgucccu caccggugca agugaagccg uggcucugcg 40
<210> 38
   <211> 49
   <212> RNA
   <213> Artificial
<220>
   <223> Synthetic
<220>
   <221> misc_feature
   <223> L-RNA
<400> 38
   gugcugcgua guggaagacu accuaaugac agccgaaugc uggcagcac 49
<210> 39
   <211> 49
   <212> RNA
   <213> Artificial
<220>
   <223> Synthetic
<220>
   <221> misc_feature
   <223> L-RNA
<400> 39
   gugcugcgua guggaagacu accuaaugac agccuaaugc uggcagcac 49
<210> 40
   <211> 49
   <212> RNA
   <213> Artificial
<220>
   <223> Synthetic
<220>
   <221> misc_feature
   <223> L-RNA
<400> 40
   gugcugcgua guggaagacu accuuaugac agccgaaugc uggcagcac 49
<210> 41
   <211> 48
   <212> RNA
   <213> Artificial
<220>
   <223> Synthetic
<220>
   <221> misc_feature
   <223> L-RNA
<400> 41
   gugcugcgua gugaaaaacu acugccagug ggucagagcu agcagcac 48
<210> 42
   <211> 48
   <212> RNA
   <213> Artificial
<220>
   <223> Synthetic
<220>
   <221> misc_feature
   <223> L-RNA
<400> 42
   gugcugcgga guuaaaaacu cccuaagaca ggccagagcc ggcagcac 48
<210> 43
   <211> 48
   <212> RNA
   <213> Artificial
<220>
   <223> Synthetic
<220>
   <221> misc_feature
   <223> L-RNA
<400> 43
   gugcugcgga guugaaaacu cccuaagaca ggccagagcc ggcagcac 48
<210> 44
   <211> 48
   <212> RNA
   <213> Artificial
<220>
   <223> Synthetic
<220>
   <221> misc_feature
   <223> L-RNA
<400> 44
   gugcugcgua guggaagacu accuaugaca gccuaaugcu ggcagcac 48
<210> 45
   <211> 48
   <212> RNA
   <213> Artificial
<220>
   <223> Synthetic
<220>
   <221> misc_feature
   <223> L-RNA
<400> 45
   gugcugcgga guuaaaaacu cccuaagaca ggcuagagcc ggcagcac 48
<210> 46
   <211> 50
   <212> RNA
   <213> Artificial
<220>
   <223> Synthetic
<220>
   <221> misc_feature
   <223> L-RNA
<400> 46
   gugcugcggc gugaaaaacg cccugcgacu gcccuuuaug caggcagcac 50
<210> 47
   <211> 48
   <212> RNA
   <213> Artificial
<220>
   <223> Synthetic
<220>
   <221> misc_feature
   <223> L-RNA
<400> 47
   gugcugcgua gugaaaaacu accaacgacu ggcuagagcc ggcagcac 48
<210> 48
   <211> 48
   <212> RNA
   <213> Artificial
<220>
   <223> Synthetic
<220>
   <221> misc_feature
   <223> L-RNA
<400> 48
   gugcugcgua gugaaagacu accugugaca gccgaaugcu ggcagcac 48
<210> 49
   <211> 48
   <212> RNA
   <213> Artificial
<220>
   <223> Synthetic
<220>
   <221> misc_feature
   <223> L-RNA
<400> 49
   guacugcgua guuaaaaacu accaacgacu ggcuagagcc ggcagcac 48
<210> 50
   <211> 48
   <212> RNA
   <213> Artificial
<220>
   <223> Synthetic
<220>
   <221> misc_feature
   <223> L-RNA
<400> 50
   gugcugcgua guuaaaaacu accaacgacu ggcuagagcc ggcagcac 48
<210> 51
   <211> 48
   <212> RNA
   <213> Artificial
<220>
   <223> Synthetic
<220>
   <221> misc_feature
   <223> L-RNA
<400> 51
   gugcugcgua guuaaaaacu accagcgaca ggcuagagcc ggcagcac 48
<210> 52
   <211> 48
   <212> RNA
   <213> Artificial
<220>
   <223> Synthetic
<220>
   <221> misc_feature
   <223> L-RNA
<400> 52
   gugcugcgua guuaaaaacu accagcgacu ggcuagagcc ggcagcac 48
<210> 53
   <211> 48
   <212> RNA
   <213> Artificial
<220>
   <223> Synthetic
<220>
   <221> misc_feature
   <223> L-RNA
<400> 53
   gugcugcgua gugagaaacu accaacgacu ggcuagagcc ggcagcac 48
<210> 54
   <211> 46
   <212> RNA
   <213> Artificial
<220>
   <223> Synthetic
<220>
   <221> misc_feature
   <223> L-RNA
<400> 54
   ggcugcguag uuaaaaacua ccagcgacug gcuagagccg gcagcc 46
<210> 55
   <211> 44
   <212> RNA
   <213> Artificial
<220>
   <223> Synthetic
<220>
   <221> misc_feature
   <223> L-RNA
<400> 55
   ggcgcguagu uaaaaacuac cagcgacugg cuagagccgg cgcc 44
<210> 56
   <211> 46
   <212> RNA
   <213> Artificial
<220>
   <223> Synthetic
<220>
   <221> misc_feature
   <223> L-RNA
<400> 56
   gugcgcguag uuaaaaacua ccagcgacug gcuagagccg gcgcac 46
<210> 57
   <211> 46
   <212> RNA
   <213> Artificial
<220>
   <223> Synthetic
<220>
   <221> misc_feature
   <223> L-RNA
<400> 57
   gugcgcguag ugagaaacua ccaacgacug gcuagagccg gcgcac 46
<210> 58
   <211> 44
   <212> RNA
   <213> Artificial
<220>
   <223> Synthetic
<220>
   <221> misc_feature
   <223> L-RNA
<400> 58
   gugccguagu gagaaacuac caacgacugg cuagagccgg gcac 44
<210> 59
   <211> 44
   <212> RNA
   <213> Artificial
<220>
   <223> Synthetic
<220>
   <221> misc_feature
   <223> L-RNA
<400> 59
   guggcguagu gagaaacuac caacgacugg cuagagccgg ccac 44
<210> 60
   <211> 44
   <212> RNA
   <213> Artificial
<220>
   <223> Synthetic
<220>
   <221> misc_feature
   <223> L-RNA
<400> 60
   gucgcguagu gagaaacuac caacgacugg cuagagccgg cgac 44
<210> 61
   <211> 44
   <212> RNA
   <213> Artificial
<220>
   <223> Synthetic
<220>
   <221> misc_feature
   <223> L-RNA
<400> 61
   ugcgcguagu gagaaacuac caacgacugg cuagagccgg cgca 44
<210> 62
   <211> 44
   <212> RNA
   <213> Artificial
<220>
   <223> Synthetic
<220>
   <221> misc_feature
   <223> L-RNA
<400> 62
   gcugcguagu gagaaacuac caacgacugg cuagagccgg cagc 44
<210> 63
   <211> 44
   <212> RNA
   <213> Artificial
<220>
   <223> Synthetic
<220>
   <221> misc_feature
   <223> L-RNA
<400> 63
   gcugcguagu gagaaacuac caacgacugg cuagagccgg cagc 44
<210> 64
   <211> 44
   <212> RNA
   <213> Artificial
<220>
   <223> Synthetic
<220>
   <221> misc_feature
   <223> L-RNA
<400> 64
   ggugcguagu gagaaacuac caacgacugg cuagagccgg cacc 44
<210> 65
   <211> 42
   <212> RNA
   <213> Artificial
<220>
   <223> Synthetic
<220>
   <221> misc_feature
   <223> L-RNA
<400> 65
   uggcguagug agaaacuacc aacgacuggc uagagccggc ca 42
<210> 66
   <211> 42
   <212> RNA
   <213> Artificial
<220>
   <223> Synthetic
<220>
   <221> misc_feature
   <223> L-RNA
<400> 66
   gcgcguagug agaaacuacc aacgacuggc uagagccggc gc 42
<210> 67
   <211> 42
   <212> RNA
   <213> Artificial
<220>
   <223> Synthetic
<220>
   <221> misc_feature
   <223> L-RNA
<400> 67
   gugcguagug agaaacuacc aacgacuggc uagagccggc ac 42
<210> 68
   <211> 42
   <212> RNA
   <213> Artificial
<220>
   <223> Synthetic
<220>
   <221> misc_feature
   <223> L-RNA
<400> 68
   gggcguagug agaaacuacc aacgacuggc uagagccggc cc 42
<210> 69
   <211> 42
   <212> RNA
   <213> Artificial
<220>
   <223> Synthetic
<220>
   <221> misc_feature
   <223> L-RNA
<400> 69
   gagcguagug agaaacuacc aacgacuggc uagagccggc uc 42
<210> 70
   <211> 42
   <212> RNA
   <213> Artificial
<220>
   <223> Synthetic
<220>
   <221> misc_feature
   <223> L-RNA
<400> 70
   cggcguagug agaaacuacc aacgacuggc uagagccggc cg 42
<210> 71
   <211> 42
   <212> RNA
   <213> Artificial
<220>
   <223> Synthetic
<220>
   <221> misc_feature
   <223> L-RNA
<400> 71
   ccgcguagug agaaacuacc aacgacuggc uagagccggc gg 42
<210> 72
   <211> 42
   <212> RNA
   <213> Artificial
<220>
   <223> Synthetic
<220>
   <221> misc_feature
   <223> L-RNA
<400> 72
   cagcguagug agaaacuacc aacgacuggc uagagccggc ug 42
<210> 73
   <211> 42
   <212> RNA
   <213> Artificial
<220>
   <223> Synthetic
<220>
   <221> misc_feature
   <223> L-RNA
<400> 73
   cugcguagug agaaacuacc aacgacuggc uagagccggc ag 42
<210> 74
   <211> 39
   <212> RNA
   <213> Artificial
<220>
   <223> Synthetic
<220>
   <221> misc_feature
   <223> L-RNA
<400> 74
   agcguguuag ugaagugggu ggcagguaaa ggacacgcu 39
<210> 75
   <211> 39
   <212> RNA
   <213> Artificial
<220>
   <223> Synthetic
<220>
   <221> misc_feature
   <223> L-RNA
<400> 75
   agcgugguag cggugugggu gguagguaaa ggccacgcu 39
<210> 76
   <211> 39
   <212> RNA
   <213> Artificial
<220>
   <223> Synthetic
<220>
   <221> misc_feature
   <223> L-RNA
<400> 76
   agcgugauag aagagcgggu gguagguaaa ggucaggcu 39
<210> 77
   <211> 39
   <212> RNA
   <213> Artificial
<220>
   <223> Synthetic
<220>
   <221> misc_feature
   <223> L-RNA
<400> 77
   agcguguuag guaggguggu aguaaguaaa ggacacgcu 39
<210> 78
   <211> 38
   <212> RNA
   <213> Artificial
<220>
   <223> Synthetic
<220>
   <221> misc_feature
   <223> L-RNA
<400> 78
   agcguguuag guggguggua guaaguaaag gacacgcu 38
<210> 79
   <211> 38
   <212> RNA
   <213> Artificial
<220>
   <223> Synthetic
<220>
   <221> misc_feature
   <223> L-RNA
<400> 79
   agcguguuag guggguggua guaaguaaag ggcacgcu 38
<210> 80
   <211> 34
   <212> RNA
   <213> Artificial
<220>
   <223> Synthetic
<220>
   <221> misc_feature
   <223> L-RNA
<400> 80
   ccgcuuaggu gggugguagu aaguaaaggg gcgg 34
<210> 81
   <211> 39
   <212> RNA
   <213> Artificial
<220>
   <223> Synthetic
<220>
   <221> misc_feature
   <223> L-RNA
<400> 81
   gcgcgagcag guggguggua gaauguaaag acucgcguc 39
<210> 82
   <211> 34
   <212> RNA
   <213> Artificial
<220>
   <223> Synthetic
<220>
   <221> misc_feature
   <223> L-RNA
<400> 82
   cguguuaggu gggugguagu aaguaaagga cacg 34
<210> 83
   <211> 32
   <212> RNA
   <213> Artificial
<220>
   <223> Synthetic
<220>
   <221> misc_feature
   <223> L-RNA
<400> 83
   guguuaggug ggugguagua aguaaaggac ac 32
<210> 84
   <211> 34
   <212> RNA
   <213> Artificial
<220>
   <223> Synthetic
<220>
   <221> misc _feature
   <223> L-RNA
<400> 84
   cguguuaggu gggugguagu aaguaaaggg cacg 34
<210> 85
   <211> 32
   <212> RNA
   <213> Artificial
<220>
   <223> Synthetic
<220>
   <221> misc _feature
   <223> L-RNA
<400> 85
   guguuaggug ggugguagua aguaaagggc ac 32
<210> 86
   <211> 30
   <212> RNA
   <213> Artificial
<220>
   <223> Synthetic
<220>
   <221> misc_feature
   <223> L-RNA
<400> 86
   uguuaggugg gugguaguaa guaaagggca 30
<210> 87
   <211> 52
   <212> RNA
   <213> Artificial
<220>
   <223> Synthetic
<220>
   <221> misc _feature
   <223> L-RNA
<400> 87
   ggacgagagu gacaaaugau auaaccuccu gacuaacgcu gcgggcgaca gg 52
<210> 88
   <211> 52
   <212> RNA
   <213> Artificial
<220>
   <223> Synthetic
<220>
   <221> misc _feature
   <223> L-RNA
<400> 88
   ggaccuaucg cuaagacaac gcgcagucua cgggacauuc uccgcggaca gg 52
<210> 89
   <211> 52
   <212> RNA
   <213> Artificial
<220>
   <223> Synthetic
<220>
   <221> misc _feature
   <223> L-RNA
<400> 89
   ggacaauugu uacccccgag agagacaaau gagacaaccu ccugaagaca gg 52
<210> 90
   <211> 51
   <212> RNA
   <213> Artificial
<220>
   <223> Synthetic
<220>
   <221> misc _feature
   <223> L-RNA
<400> 90
   ggacgaaagu gagaaaugau acaaccuccu guugcugcga auccggacag g 51
<210> 91
   <211> 50
   <212> RNA
   <213> Artificial
<220>
   <223> Synthetic
<220>
   <221> misc _feature
   <223> L-RNA
<400> 91
   ggacguaaaa gacgcuaccc gaaagaaugu caggagggua gaccgacagg 50
<210> 92
   <211> 50
   <212> RNA
   <213> Artificial
<220>
   <223> Synthetic
<220>
   <221> misc _feature
   <223> L-RNA
<400> 92
   ggacuagaaa cuacaauagc ggccaguugc accgcguuau caacgacagg 50
<210> 93
   <211> 50
   <212> RNA
   <213> Artificial
<220>
   <223> Synthetic
<220>
   <221> misc _feature
   <223> L-RNA
<400> 93
   ggacuaguca gccagugugu auaucggacg cggguuuauu uacugacagg 50
<210> 94
   <211> 50
   <212> RNA
   <213> Artificial
<220>
   <223> Synthetic
<220>
   <221> misc _feature
   <223> L-RNA
<400> 94
   ggacuguccg gagugugaaa cuccccgaga ccgccagaag cggggacagg 50
<210> 95
   <211> 50
   <212> RNA
   <213> Artificial
<220>
   <223> Synthetic
<220>
   <221> misc _feature
   <223> L-RNA
<400> 95
   ggacuucuau ccaggugggu gguaguaugu aaagagauag aagugacagg 50
<210> 96
   <211> 50
   <212> RNA
   <213> Artificial
<220>
   <223> Synthetic
<220>
   <221> misc _feature
   <223> L-RNA
<400> 96
   ggacgagagc gaacaaugau auaaccuccu gacggaaaga gaucgacagg 50
<210> 97
   <211> 48
   <212> RNA
   <213> Artificial
<220>
   <223> Syntethic
<220>
   <221> misc _feature
   <223> L-RNA
<400> 97
   ccugugcuac acgcaguaag aagugaacgu ucaguaugug ugcacagg 48
<210> 98
   <211> 48
   <212> RNA
   <213> Artificial
<220>
   <223> Synthetic
<220>
   <221> misc _feature
   <223> L-RNA
<400> 98
   cgugagccag gcaccgaggg cguuaacugg cugauuggac acgacacg 48
<210> 99
   <211> 48
   <212> RNA
   <213> Artificial
<220>
   <223> Synthetic
<220>
   <221> misc _feature
   <223> L-RNA
<400> 99
   cgugaacaug caagcuaagc ggggcuguug guugcuuggc ccgccacg 48
<210> 100
   <211> 48
   <212> RNA
   <213> Artificial
<220>
   <223> Synthetic
<220>
   <221> misc_feature
   <223> L-RNA
<400> 100
   cgugcagaga gagaccaacc acguaaaauc aaccuaaugg gccgcacg 48
<210> 101
   <211> 48
   <212> RNA
   <213> Artificial
<220>
   <223> Synthetic
<220>
   <221> misc _feature
   <223> L-RNA
<400> 101
   cgugcagaga gagaccaacc acguaaaauc aaccuaaugg gccgcacg 48
<210> 102
   <211> 48
   <212> RNA
   <213> Artificial
<220>
   <223> Synthetic
<220>
   <221> misc _feature
   <223> L-RNA
<400> 102
   cgugaacauu caagcuaagc ggggcuguug guugcuuggc ccgccacg 48
<210> 103
   <211> 48
   <212> RNA
   <213> Artificial
<220>
   <223> Synthetic
<220>
   <221> misc _feature
   <223> L-RNA
<400> 103
   cgugccgagg cggcgaccag cguuacuuag agaggcuuug gcaccacg 48
<210> 104
   <211> 47
   <212> RNA
   <213> Artificial
<220>
   <223> Synthetic
<220>
   <221> misc_feature
   <223> L-RNA
<400> 104
   cgugauaaca gccgucgguc aagaaaacaa aguucgggcg gcgcacg 47
<210> 105
   <211> 47
   <212> RNA
   <213> Artificial
<220>
   <223> Synthetic
<220>
   <221> misc _feature
   <223> L-RNA
<400> 105
   cguggguggc gcaccgaggg cgaaaagcca ccaguaaaga uagaccg 47
<210> 106
   <211> 45
   <212> RNA
   <213> Artificial
<220>
   <223> Synthetic
<220>
   <221> misc _feature
   <223> L-RNA
<400> 106
   cgugugaucu ccuuuggggu gauuagcuua gagacuuccc acacg 45
<210> 107
   <211> 45
   <212> RNA
   <213> Artificial
<220>
   <223> Synthetic
<220>
   <221> misc _feature
   <223> L-RNA
<400> 107
   gcaccuucgc cuaauacacg ugccggcuag cuaauacucg uccgc 45
<210> 108
   <211> 45
   <212> RNA
   <213> Artificial
<220>
   <223> Synthetic
<220>
   <221> misc_feature
   <223> L-RNA
<400> 108
   gcacgacuug ggcgaccagu gauacuuaga gagcaagucg ucggc 45
<210> 109
   <211> 44
   <212> RNA
   <213> Artificial
<220>
   <223> Synthetic
<220>
   <221> misc _feature
   <223> L-RNA
<400> 109
   gcgcgcgcuc aguaagaaau ugaaaguuca gaaugucguc gcgc 44
<210> 110
   <211> 39
   <212> RNA
   <213> Artificial
<220>
   <223> Synthetic
<220>
   <221> misc _feature
   <223> L-RNA
<400> 110
   aguguguggc aggcuaagga gauauuccga gaccacgcu 39
<210> 111
   <211> 39
   <212> RNA
   <213> Artificial
<220>
   <223> Synthetic
<220>
   <221> misc _feature
   <223> L-RNA
<400> 111
   aguguguggc agacuaugga uagacuccga gaccacgcu 39
<210> 112
   <211> 39
   <212> RNA
   <213> Artificial
<220>
   <223> Synthetic
<220>
   <221> misc _feature
   <223> L-RNA
<400> 112
   agcgugaggc gaccagcgga uuacuuagag agucacgcu 39
<210> 113
   <211> 39
   <212> RNA
   <213> Artificial
<220>
   <223> Synthetic
<220>
   <221> misc _feature
   <223> L-RNA
<400> 113
   agcgugaagg ggaccagcgu uacuuacaga guucacgcu 39
<210> 114
   <211> 39
   <212> RNA
   <213> Artificial
<220>
   <223> Synthetic
<220>
   <221> misc _feature
   <223> L-RNA
<400> 114
   agcgugugau guauguagca ccguaucaga ggacacgcu 39
<210> 115
   <211> 37
   <212> RNA
   <213> Artificial
<220>
   <223> Synthetic
<220>
   <221> misc _feature
   <223> L-RNA
<400> 115
   agcgugaggc gacccguguu ucguagagag ucacgcu 37
<210> 116
   <211> 40
   <212> RNA
   <213> Artificial
<220>
   <223> Synthetic
<220>
   <221> misc _feature
   <223> L-RNA
<220>
   <221> misc _feature
   <223> PEG moiety at 5' end
<400> 116
   gcacgucccu caccggugca agugaagccg uggcucugcg 40
<210> 117
   <211> 40
   <212> RNA
   <213> Artificial
<220>
   <223> Synthetic
<220>
   <221> misc _feature
   <223> L-RNA
<220>
   <221> misc _feature
   <223> PEG moiety at 3' end
<400> 117
   gcacgucccu caccggugca agugaagccg uggcucugcg 40
<210> 118
   <211> 13
   <212> RNA
   <213> Artificial
<220>
   <223> Synthetic
<400> 118
   ggggggcgcg acc 13
<210> 119
   <211> 76
   <212> PRT
   <213> Homo sapiens
<220>
   <221> MISC_FEATURE
   <223> D-MCP-1
<220>
   <221> MISC _FEATURE
   <223> Biotin at N-terminal amino acid
<400> 119
   Gln Pro Asp Ala Ile Asn Ala Pro Val Thr Cys Cys Tyr Asn Phe Thr
   1               5                   10                  15
   Asn Arg Lys Ile Ser Val Gln Arg Leu Ala Ser Tyr Arg Arg Ile Thr
               20                  25                  30
   Ser Ser Lys Cys Pro Lys Glu Ala Val Ile Phe Lys Thr Ile Val Ala
           35                  40                  45
   Lys Glu Ile Cys Ala Asp Pro Lys Gln Lys Trp Val Gln Asp Ser Met
       50                  55                  60
   Asp His Leu Asp Lys Gln Thr Gln Thr Pro Lys Thr
   65                  70                  75
<210> 120
   <211> 33
   <212> RNA
   <213> Artificial
<220>
   <223> Synthetic
<220>
   <221> misc _feature
   <223> L-RNA
<400> 120
   cgucccucac cggugcaagu gaagccgugg cuc 33
<210> 121
   <211> 47
   <212> RNA
   <213> Artificial
<220>
   <223> Synthetic
<220>
   <221> misc _feature
   <223> D-RNA
<400> 121
   agcgugcccg gaguggcagg gggacgcgac cugcaauaau gcacgcu 47
<210> 122
   <211> 45
   <212> RNA
   <213> Artificial
<220>
   <223> Synthetic
<220>
   <221> misc _feature
   <223> D-RNA
<400> 122
   agcgugcccg gaguggcagg gggacgcgac cugcaauugc acgcu 45
<210> 123
   <211> 47
   <212> RNA
   <213> Artificial
<220>
   <223> Synthetic
<220>
   <221> misc _feature
   <223> D-RNA
<400> 123
   agcgugcccg gaguggcagg gggacgcgac cuguaauaau gcacgcu 47
<210> 124
   <211> 47
   <212> RNA
   <213> Artificial
<220>
   <223> Synthetic
<220>
   <221> misc_feature
   <223> D-RNA
<400> 124
   agcgugcccg guguggcagg gggacgcgac cugcaauaau gcgcgcu 47
<210> 125
   <211> 47
   <212> RNA
   <213> Artificial
<220>
   <223> Synthetic
<220>
   <221> misc _feature
   <223> D-RNA
<400> 125
   agcgugcccg gaguagcagg ggggcgcgac cugcaauaau gcacgcu 47
<210> 126
   <211> 45
   <212> RNA
   <213> Artificial
<220>
   <223> Synthetic
<220>
   <221> misc _feature
   <223> D-RNA
<400> 126
   agcgugcccg gugugguagg ggggcgcgau cuacaauugc acgcu 45
<210> 127
   <211> 45
   <212> RNA
   <213> Artificial
<220>
   <223> Synthetic
<220>
   <221> misc _feature
   <223> D-RNA
<400> 127
   agcgugcccg gugugacagg ggggcgcgac cugcauuugc acgcu 45
<210> 128
   <211> 45
   <212> RNA
   <213> Artificial
<220>
   <223> Synthetic
<220>
   <221> misc _feature
   <223> D-RNA
<400> 128
   agcgugcccg guguggcagg ggggcgcgac cuguauuugc acgcu 45
<210> 129
   <211> 47
   <212> RNA
   <213> Artificial
<220>
   <223> Synthetic
<220>
   <221> misc _feature
   <223> D-RNA
<400> 129
   agcgugcccg gaguggcagg ggggcgcgac cugcaauaau gcacgcu 47
<210> 130
   <211> 45
   <212> RNA
   <213> Artificial
<220>
   <223> Synthetic
<220>
   <221> misc _feature
   <223> D-RNA
<400> 130
   agcgugcccg guguggcagg ggggcgcgac cugcaauugc acgcu 45
<210> 131
   <211> 45
   <212> RNA
   <213> Artificial
<220>
   <223> Syntehtic
<220>
   <221> misc _feature
   <223> D-RNA
<400> 131
   agcaugcccg guguggcagg ggggcgcgac cugcauuugc augcu 45
<210> 132
   <211> 45
   <212> RNA
   <213> Artificial
<220>
   <223> Synthetic
<220>
   <221> misc _feature
   <223> D-RNA
<400> 132
   agcgugcccg gugugguagg ggggcgcgac cuacauuugc acgcu 45
<210> 133
   <211> 44
   <212> RNA
   <213> Artificial
<220>
   <223> Synthetic
<220>
   <221> misc _feature
   <223> D-RNA
<400> 133
   agugugccag cugugauggg ggggcgcgac ccauuuuaca cacu 44
<210> 134
   <211> 43
   <212> RNA
   <213> Artificial
<220>
   <223> Synthetic
<220>
   <221> misc _feature
   <223> D-RNA
<400> 134
   agugugccag cgugaugggg gggcgcgacc cauuuuacac acu 43
<210> 135
   <211> 43
   <212> RNA
   <213> Artificial
<220>
   <223> Synthetic
<220>
   <221> misc _feature
   <223> D-RNA
<400> 135
   agugugcgag cgugaugggg gggcgcgacc cauuuuacau acu 43
<210> 136
   <211> 43
   <212> RNA
   <213> Artificial
<220>
   <223> Synthetic
<220>
   <221> misc _feature
   <223> D-RNA
<400> 136
   agugugccag cgugaugggg gggcgcgacc cauuuuacau acu 43
<210> 137
   <211> 42
   <212> RNA
   <213> Artificial
<220>
   <223> Synthetic
<220>
   <221> misc _feature
   <223> D-RNA
<400> 137
   aguaugccag cgugaugggg gggcgcgacc cauuuacaua cu 42
<210> 138
   <211> 43
   <212> RNA
   <213> Artificial
<220>
   <223> Synthetic
<220>
   <221> misc _feature
   <223> D-RNA
<400> 138
   agugugccag ugugaugggg gggcgcgacc cauuuuacac acu 43
<210> 139
   <211> 43
   <212> RNA
   <213> Artificial
<220>
   <223> Synthetic
<220>
   <221> misc _feature
   <223> D-RNA
<400> 139
   agcgugccag ugugaugggg gggcgcgacc cauuuuacac gcu 43
<210> 140
   <211> 43
   <212> RNA
   <213> Artificial
<220>
   <223> Synthetic
<220>
   <221> misc _feature
   <223> D-RNA
<400> 140
   acgcacgucc cucaccggug caagugaagc cgcggcucug cgu 43
<210> 141
   <211> 43
   <212> RNA
   <213> Artificial
<220>
   <223> Synthetic
<220>
   <221> misc _feature
   <223> D-RNA
<400> 141
   acgcaccucc cucaccggug caagugaagc cguggcucug cgc 43
<210> 142
   <211> 43
   <212> RNA
   <213> Artificial
<220>
   <223> Synthetic
<220>
   <221> misc _feature
   <223> D-RNA
<400> 142
   acgcacgucc cucaccggug caagugaagc cguggcucug cgu 43
<210> 143
   <211> 41
   <212> RNA
   <213> Artificial
<220>
   <223> Synthetic
<220>
   <221> misc _feature
   <223> D-RNA
<400> 143
   gcacgucccu caccggugca agugaagccg uggcucugcg u 41
<210> 144
   <211> 41
   <212> RNA
   <213> Artificial
<220>
   <223> Synthetic
<220>
   <221> misc _feature
   <223> D-RNA
<400> 144
   acgcacgucc cucaccggug caagugaagc cguggcucug c 41
<210> 145
   <211> 39
   <212> RNA
   <213> Artificial
<220>
   <223> Synthetic
<220>
   <221> misc _feature
   <223> D-RNA
<400> 145
   gcacgucccu caccggugca agugaagccg uggcucugc 39
<210> 146
   <211> 42
   <212> RNA
   <213> Artificial
<220>
   <223> Synthetic
<220>
   <221> misc _feature
   <223> D-RNA
<400> 146
   cgcacguccc ucaccggugc aagugaagcc guggcucugc gu 42
<210> 147
   <211> 41
   <212> RNA
   <213> Artificial
<220>
   <223> Synthetic
<220>
   <221> misc _feature
   <223> D-RNA
<400> 147
   cgcacguccc ucaccggugc aagugaagcc guggcucugc g 41
<210> 148
   <211> 40
   <212> RNA
   <213> Artificial
<220>
   <223> Synthetic
<220>
   <221> misc _feature
   <223> D-RNA
<400> 148
   gcacgucccu caccggugca agugaagccg uggcucugcg 40
<210> 149
   <211> 49
   <212> RNA
   <213> Artificial
<220>
   <223> Synthetic
<220>
   <221> misc _feature
   <223> D-RNA
<400> 149
   gugcugcgua guggaagacu accuaaugac agccgaaugc uggcagcac 49
<210> 150
   <211> 49
   <212> RNA
   <213> Artificial
<220>
   <223> Synthetic
<220>
   <221> misc _feature
   <223> D-RNA
<400> 150
   gugcugcgua guggaagacu accuaaugac agccuaaugc uggcagcac 49
<210> 151
   <211> 49
   <212> RNA
   <213> Artificial
<220>
   <223> Synthetic
<220>
   <221> misc _feature
   <223> D-RNA
<400> 151
   gugcugcgua guggaagacu accuuaugac agccgaaugc uggcagcac 49
<210> 152
   <211> 48
   <212> RNA
   <213> Artificial
<220>
   <223> Synthetic
<220>
   <221> misc _feature
   <223> D-RNA
<400> 152
   gugcugcgua gugaaaaacu acugccagug ggucagagcu agcagcac 48
<210> 153
   <211> 48
   <212> RNA
   <213> Artificial
<220>
   <223> Synthetic
<220>
   <221> misc _feature
   <223> D-RNA
<400> 153
   gugcugcgga guuaaaaacu cccuaagaca ggccagagcc ggcagcac 48
<210> 154
   <211> 48
   <212> RNA
   <213> Artificial
<220>
   <223> Synthetic
<220>
   <221> misc _feature
   <223> D-RNA
<400> 154
   gugcugcgga guugaaaacu cccuaagaca ggccagagcc ggcagcac 48
<210> 155
   <211> 48
   <212> RNA
   <213> Artificial
<220>
   <223> Synthetic
<220>
   <221> misc _feature
   <223> D-RNA
<400> 155
   gugcugcgua guggaagacu accuaugaca gccuaaugcu ggcagcac 48
<210> 156
   <211> 48
   <212> RNA
   <213> Artificial
<220>
   <223> Synthetic
<220>
   <221> misc _feature
   <223> D-RNA
<400> 156
   gugcugcgga guuaaaaacu cccuaagaca ggcuagagcc ggcagcac 48
<210> 157
   <211> 50
   <212> RNA
   <213> Artificial
<220>
   <223> Synthetic
<220>
   <221> misc _feature
   <223> D-RNA
<400> 157
   gugcugcggc gugaaaaacg cccugcgacu gcccuuuaug caggcagcac 50
<210> 158
   <211> 48
   <212> RNA
   <213> Artificial
<220>
   <223> Synthetic
<220>
   <221> misc _feature
   <223> D-RNA
<400> 158
   gugcugcgua gugaaaaacu accaacgacu ggcuagagcc ggcagcac 48
<210> 159
   <211> 48
   <212> RNA
   <213> Artificial
<220>
   <223> Synthetic
<220>
   <221> misc _feature
   <223> D-RNA
<400> 159
   gugcugcgua gugaaagacu accugugaca gccgaaugcu ggcagcac 48
<210> 160
   <211> 48
   <212> RNA
   <213> Artificial
<220>
   <223> Synthetic
<220>
   <221> misc _feature
   <223> D-RNA
<400> 160
   guacugcgua guuaaaaacu accaacgacu ggcuagagcc ggcagcac 48
<210> 161
   <211> 48
   <212> RNA
   <213> Artificial
<220>
   <223> Synthetic
<220>
   <221> misc_feature
   <223> D-RNA
<400> 161
   gugcugcgua guuaaaaacu accaacgacu ggcuagagcc ggcagcac 48
<210> 162
   <211> 48
   <212> RNA
   <213> Artificial
<220>
   <223> Synthetic
<220>
   <221> misc _feature
   <223> D-RNA
<400> 162
   gugcugcgua guuaaaaacu accagcgaca ggcuagagcc ggcagcac 48
<210> 163
   <211> 48
   <212> RNA
   <213> Artificial
<220>
   <223> Synthetic
<220>
   <221> misc _feature
   <223> D-RNA
<400> 163
   gugcugcgua guuaaaaacu accagcgacu ggcuagagcc ggcagcac 48
<210> 164
   <211> 48
   <212> RNA
   <213> Artificial
<220>
   <223> Synthetic
<220>
   <221> misc _feature
   <223> D-RNA
<400> 164
   gugcugcgua gugagaaacu accaacgacu ggcuagagcc ggcagcac 48
<210> 165
   <211> 46
   <212> RNA
   <213> Artificial
<220>
   <223> Synthetic
<220>
   <221> misc _feature
   <223> D-RNA
<400> 165
   ggcugcguag uuaaaaacua ccagcgacug gcuagagccg gcagcc 46
<210> 166
   <211> 44
   <212> RNA
   <213> Artificial
<220>
   <223> Synthetic
<220>
   <221> misc _feature
   <223> D-RNA
<400> 166
   ggcgcguagu uaaaaacuac cagcgacugg cuagagccgg cgcc 44
<210> 167
   <211> 46
   <212> RNA
   <213> Artificial
<220>
   <223> Synthetic
<220>
   <221> misc _feature
   <223> D-RNA
<400> 167
   gugcgcguag uuaaaaacua ccagcgacug gcuagagccg gcgcac 46
<210> 168
   <211> 46
   <212> RNA
   <213> Artificial
<220>
   <223> Synthetic
<220>
   <221> misc _feature
   <223> D-RNA
<400> 168
   gugcgcguag ugagaaacua ccaacgacug gcuagagccg gcgcac 46
<210> 169
   <211> 44
   <212> RNA
   <213> Artificial
<220>
   <223> Synthetic
<220>
   <221> misc_feature
   <223> D-RNA
<400> 169
   gugccguagu gagaaacuac caacgacugg cuagagccgg gcac 44
<210> 170
   <211> 44
   <212> RNA
   <213> Artificial
<220>
   <223> Synthetic
<220>
   <221> misc _feature
   <223> D-RNA
<400> 170
   guggcguagu gagaaacuac caacgacugg cuagagccgg ccac 44
<210> 171
   <211> 44
   <212> RNA
   <213> Artificial
<220>
   <223> Synthetic
<220>
   <221> misc _feature
   <223> D-RNA
<400> 171
   gucgcguagu gagaaacuac caacgacugg cuagagccgg cgac 44
<210> 172
   <211> 44
   <212> RNA
   <213> Artificial
<220>
   <223> Synthetic
<220>
   <221> misc_feature
   <223> D-RNA
<400> 172
   ugcgcguagu gagaaacuac caacgacugg cuagagccgg cgca 44
<210> 173
   <211> 44
   <212> RNA
   <213> Artificial
<220>
   <223> Synthetic
<220>
   <221> misc _feature
   <223> D-RNA
<400> 173
   gcugcguagu gagaaacuac caacgacugg cuagagccgg cage 44
<210> 174
   <211> 44
   <212> RNA
   <213> Artificial
<220>
   <223> Synthetic
<220>
   <221> misc _feature
   <223> D-RNA
<400> 174
   gcugcguagu gagaaacuac caacgacugg cuagagccgg cagc 44
<210> 175
   <211> 44
   <212> RNA
   <213> Artificial
<220>
   <223> Synthetic
<220>
   <221> misc _feature
   <223> D-RNA
<400> 175
   ggugcguagu gagaaacuac caacgacugg cuagagccgg cacc 44
<210> 176
   <211> 42
   <212> RNA
   <213> Artificial
<220>
   <223> Synthetic
<220>
   <221> misc _feature
   <223> D-RNA
<400> 176
   uggcguagug agaaacuacc aacgacuggc uagagccggc ca 42
<210> 177
   <211> 42
   <212> RNA
   <213> Artificial
<220>
   <223> Synthetic
<220>
   <221> misc _feature
   <223> D-RNA
<400> 177
   gcgcguagug agaaacuacc aacgacuggc uagagccggc gc 42
<210> 178
   <211> 42
   <212> RNA
   <213> Artificial
<220>
   <223> Synthetic
<220>
   <221> misc _feature
   <223> D-RNA
<400> 178
   gugcguagug agaaacuacc aacgacuggc uagagccggc ac 42
<210> 179
   <211> 42
   <212> RNA
   <213> Artificial
<220>
   <223> Synthetic
<220>
   <221> misc_feature
   <223> D-RNA
<400> 179
   gggcguagug agaaacuacc aacgacuggc uagagccggc cc 42
<210> 180
   <211> 42
   <212> RNA
   <213> Artificial
<220>
   <223> Synthetic
<220>
   <221> misc _feature
   <223> D-RNA
<400> 180
   gagcguagug agaaacuacc aacgacuggc uagagccggc uc 42
<210> 181
   <211> 42
   <212> RNA
   <213> Artificial
<220>
   <223> Synthetic
<220>
   <221> misc_feature
   <223> D-RNA
<400> 181
   cggcguagug agaaacuacc aacgacuggc uagagccggc cg 42
<210> 182
   <211> 42
   <212> RNA
   <213> Artificial
<220>
   <223> Synthetic
<220>
   <221> misc _feature
   <223> D-RNA
<400> 182
   ccgcguagug agaaacuacc aacgacuggc uagagccggc gg 42
<210> 183
   <211> 42
   <212> RNA
   <213> Artificial
<220>
   <223> Synthetic
<220>
   <221> misc _feature
   <223> D-RNA
<400> 183
   cagcguagug agaaacuacc aacgacuggc uagagccggc ug 42
<210> 184
   <211> 42
   <212> RNA
   <213> Artificial
<220>
   <223> Synthetic
<220>
   <221> misc_feature
   <223> D-RNA
<400> 184
   cugcguagug agaaacuacc aacgacuggc uagagccggc ag 42
<210> 185
   <211> 39
   <212> RNA
   <213> Artificial
<220>
   <223> Synthetic
<220>
   <221> misc _feature
   <223> D-RNA
<400> 185
   agcguguuag ugaagugggu ggcagguaaa ggacacgcu 39
<210> 186
   <211> 39
   <212> RNA
   <213> Artificial
<220>
   <223> Synthetic
<220>
   <221> misc _feature
   <223> D-RNA
<400> 186
   agcgugguag cggugugggu gguagguaaa ggccacgcu 39
<210> 187
   <211> 39
   <212> RNA
   <213> Artificial
<220>
   <223> Synthetic
<220>
   <221> misc _feature
   <223> D-RNA
<400> 187
   agcgugauag aagagcgggu gguagguaaa ggucaggcu 39
<210> 188
   <211> 39
   <212> RNA
   <213> Artificial
<220>
   <223> Synthetic
<220>
   <221> misc _feature
   <223> D-RNA
<400> 188
   agcguguuag guaggguggu aguaaguaaa ggacacgcu 39
<210> 189
   <211> 38
   <212> RNA
   <213> Artificial
<220>
   <223> Synthetic
<220>
   <221> misc _feature
   <223> D-RNA
<400> 189
   agcguguuag guggguggua guaaguaaag gacacgcu 38
<210> 190
   <211> 38
   <212> RNA
   <213> Artificial
<220>
   <223> Synthetic
<220>
   <221> misc _feature
   <223> D-RNA
<400> 190
   agcguguuag guggguggua guaaguaaag ggcacgcu 38
<210> 191
   <211> 34
   <212> RNA
   <213> Artificial
<220>
   <223> Synthetic
<220>
   <221> misc _feature
   <223> D-RNA
<400> 191
   ccgcuuaggu gggugguagu aaguaaaggg gcgg 34
<210> 192
   <211> 39
   <212> RNA
   <213> Artificial
<220>
   <223> Synthetic
<220>
   <221> misc_feature
   <223> D-RNA
<400> 192
   gcgcgagcag guggguggua gaauguaaag acucgcguc 39
<210> 193
   <211> 34
   <212> RNA
   <213> Artificial
<220>
   <223> Synthetic
<220>
   <221> misc _feature
   <223> D-RNA
<400> 193
   cguguuaggu gggugguagu aaguaaagga cacg 34
<210> 194
   <211> 32
   <212> RNA
   <213> Artificial
<220>
   <223> Synthetic
<220>
   <221> misc _feature
   <223> D-RNA
<400> 194
   guguuaggug ggugguagua aguaaaggac ac 32
<210> 195
   <211> 34
   <212> RNA
   <213> Artificial
<220>
   <223> Synthetic
<220>
   <221> misc _feature
   <223> D-RNA
<400> 195
   cguguuaggu gggugguagu aaguaaaggg cacg 34
<210> 196
   <211> 32
   <212> RNA
   <213> Artificial
<220>
   <223> Synthetic
<220>
   <221> misc _feature
   <223> D-RNA
<400> 196
   guguuaggug ggugguagua aguaaagggc ac 32
<210> 197
   <211> 30
   <212> RNA
   <213> Artificial
<220>
   <223> Synthetic
<220>
   <221> misc _feature
   <223> D-RNA
<400> 197
   uguuaggugg gugguaguaa guaaagggca 30
<210> 198
   <211> 52
   <212> RNA
   <213> Artificial
<220>
   <223> Synthetic
<220>
   <221> misc_feature
   <223> D-RNA
<400> 198
   ggacgagagu gacaaaugau auaaccuccu gacuaacgcu gcgggcgaca gg 52
<210> 199
   <211> 52
   <212> RNA
   <213> Artificial
<220>
   <223> Synthetic
<220>
   <221> misc _feature
   <223> D-RNA
<400> 199
   ggaccuaucg cuaagacaac gcgcagucua cgggacauuc uccgcggaca gg 52
<210> 200
   <211> 52
   <212> RNA
   <213> Artificial
<220>
   <223> Synthetic
<220>
   <221> misc _feature
   <223> D-RNA
<400> 200
   ggacaauugu uacccccgag agagacaaau gagacaaccu ccugaagaca gg 52
<210> 201
   <211> 51
   <212> RNA
   <213> Artificial
<220>
   <223> Synthetic
<220>
   <221> misc _feature
   <223> D-RNA
<400> 201
   ggacgaaagu gagaaaugau acaaccuccu guugcugcga auccggacag g 51
<210> 202
   <211> 50
   <212> RNA
   <213> Artificial
<220>
   <223> Synthetic
<220>
   <221> misc_feature
   <223> D-RNA
<400> 202
   ggacguaaaa gacgcuaccc gaaagaaugu caggagggua gaccgacagg 50
<210> 203
   <211> 50
   <212> RNA
   <213> Artificial
<220>
   <223> Synthetic
<220>
   <221> misc _feature
   <223> D-RNA
<400> 203
   ggacuagaaa cuacaauagc ggccaguugc accgcguuau caacgacagg 50
<210> 204
   <211> 50
   <212> RNA
   <213> Artificial
<220>
   <223> Synthetic
<220>
   <221> misc _feature
   <223> D-RNA
<400> 204
   ggacuaguca gccagugugu auaucggacg cggguuuauu uacugacagg 50
<210> 205
   <211> 50
   <212> RNA
   <213> Artificial
<220>
   <223> Synthetic
<220>
   <221> misc _feature
   <223> D-RNA
<400> 205
   ggacuguccg gagugugaaa cuccccgaga ccgccagaag cggggacagg 50
<210> 206
   <211> 50
   <212> RNA
   <213> Artificial
<220>
   <223> Synthetic
<220>
   <221> misc _feature
   <223> D-RNA
<400> 206
   ggacuucuau ccaggugggu gguaguaugu aaagagauag aagugacagg 50
<210> 207
   <211> 50
   <212> RNA
   <213> Artificial
<220>
   <223> Synthetic
<220>
   <221> misc_feature
   <223> D-RNA
<400> 207
   ggacgagagc gaacaaugau auaaccuccu gacggaaaga gaucgacagg 50
<210> 208
   <211> 48
   <212> RNA
   <213> Artificial
<220>
   <223> Synthetic
<220>
   <221> misc _feature
   <223> D-RNA
<400> 208
   ccugugcuac acgcaguaag aagugaacgu ucaguaugug ugcacagg 48
<210> 209
   <211> 48
   <212> RNA
   <213> Artificial
<220>
   <223> Synthetic
<220>
   <221> misc _feature
   <223> D-RNA
<400> 209
   cgugagccag gcaccgaggg cguuaacugg cugauuggac acgacacg 48
<210> 210
   <211> 48
   <212> RNA
   <213> Artificial
<220>
   <223> Synthetic
<220>
   <221> misc _feature
   <223> D-RNA
<400> 210
   cgugaacaug caagcuaagc ggggcuguug guugcuuggc ccgccacg 48
<210> 211
   <211> 48
   <212> RNA
   <213> Artificial
<220>
   <223> Synthetic
<220>
   <221> misc _feature
   <223> D-RNA
<400> 211
   cgugcagaga gagaccaacc acguaaaauc aaccuaaugg gccgcacg 48
<210> 212
   <211> 48
   <212> RNA
   <213> Artificial
<220>
   <223> Synthetic
<220>
   <221> misc _feature
   <223> D-RNA
<400> 212
   cgugcagaga gagaccaacc acguaaaauc aaccuaaugg gccgcacg 48
<210> 213
   <211> 48
   <212> RNA
   <213> Artificial
<220>
   <223> Synthetic
<220>
   <221> misc _feature
   <223> D-RNA
<400> 213
   cgugaacauu caagcuaagc ggggcuguug guugcuuggc ccgccacg 48
<210> 214
   <211> 48
   <212> RNA
   <213> Artificial
<220>
   <223> Synthetic
<220>
   <221> misc _feature
   <223> D-RNA
<400> 214
   cgugccgagg cggcgaccag cguuacuuag agaggcuuug gcaccacg 48
<210> 215
   <211> 47
   <212> RNA
   <213> Artificial
<220>
   <223> Synthetic
<220>
   <221> misc _feature
   <223> D-RNA
<400> 215
   cgugauaaca gccgucgguc aagaaaacaa aguucgggcg gcgcacg 47
<210> 216
   <211> 47
   <212> RNA
   <213> Artificial
<220>
   <223> Synthetic
<220>
   <221> misc _feature
   <223> D-RNA
<400> 216
   cguggguggc gcaccgaggg cgaaaagcca ccaguaaaga uagaccg 47
<210> 217
   <211> 45
   <212> RNA
   <213> Artificial
<220>
   <223> Synthetic
<220>
   <221> misc _feature
   <223> D-RNA
<400> 217
   cgugugaucu ccuuuggggu gauuagcuua gagacuuccc acacg 45
<210> 218
   <211> 45
   <212> RNA
   <213> Artificial
<220>
   <223> Synthetic
<220>
   <221> misc _feature
   <223> D-RNA
<400> 218
   gcaccuucgc cuaauacacg ugccggcuag cuaauacucg uccgc 45
<210> 219
   <211> 45
   <212> RNA
   <213> Artificial
<220>
   <223> Synthetic
<220>
   <221> misc _feature
   <223> D-RNA
<400> 219
   gcacgacuug ggcgaccagu gauacuuaga gagcaagucg ucggc 45
<210> 220
   <211> 44
   <212> RNA
   <213> Artificial
<220>
   <223> Synthetic
<220>
   <221> misc _feature
   <223> D-RNA
<400> 220
   gcgcgcgcuc aguaagaaau ugaaaguuca gaaugucguc gcgc 44
<210> 221
   <211> 39
   <212> RNA
   <213> Artificial
<220>
   <223> Synthetic
<220>
   <221> misc _feature
   <223> D-RNA
<400> 221
   aguguguggc aggcuaagga gauauuccga gaccacgcu 39
<210> 222
   <211> 39
   <212> RNA
   <213> Artificial
<220>
   <223> Synthetic
<220>
   <221> misc _feature
   <223> D-RNA
<400> 222
   aguguguggc agacuaugga uagacuccga gaccacgcu 39
<210> 223
   <211> 39
   <212> RNA
   <213> Artificial
<220>
   <223> Synthetic
<220>
   <221> misc_feature
   <223> D-RNA
<400> 223
   agcgugaggc gaccagcgga uuacuuagag agucacgcu 39
<210> 224
   <211> 39
   <212> RNA
   <213> Artificial
<220>
   <223> Synthetic
<220>
   <221> misc _feature
   <223> D-RNA
<400> 224
   agcgugaagg ggaccagcgu uacuuacaga guucacgcu 39
<210> 225
   <211> 39
   <212> RNA
   <213> Artificial
<220>
   <223> Synthetic
<220>
   <221> misc _feature
   <223> D-RNA
<400> 225
   agcgugugau guauguagca ccguaucaga ggacacgcu 39
<210> 226
   <211> 37
   <212> RNA
   <213> Artificial
<220>
   <223> Synthetic
<220>
   <221> misc _feature
   <223> D-RNA
<400> 226
   agcgugaggc gacccguguu ucguagagag ucacgcu 37
<210> 227
   <211> 82
   <212> PRT
   <213> Homo sapiens
<220>
   <221> MISC _FEATURE
   <223> MCP-4
<400> 227
   Phe Asn Pro Gln Gly Leu Ala Gln Pro Asp Ala Leu Asn Val Pro Ser
   1               5                   10                  15
   Thr Cys Cys Phe Thr Phe Ser Ser Lys Lys Ile Ser Leu Gln Arg Leu
               20                  25                  30
   Lys Ser Tyr Val Ile Thr Thr Ser Arg Cys Pro Gln Lys Ala Val Ile
           35                  40                  45
   Phe Arg Thr Lys Leu Gly Lys Glu Ile Cys Ala Asp Pro Lys Glu Lys
       50                  55                  60
   Trp Val Gln Asn Tyr Met Lys His Leu Gly Arg Lys Ala His Thr Leu
   65                  70                  75                  80
   Lys Thr
<210> 228
   <211> 40
   <212> RNA
   <213> Artificial
<220>
   <223> Synthetic
<220>
   <221> misc _feature
   <223> L-RNA
<220>
   <221> misc _feature
   <223> Linker at 5' end, whereby two PEG moieties are attached to the linker
<400> 228
   gcacgucccu caccggugca agugaagccg uggcucugcg 40